# EUROPEAN PATENT SPECIFICATION

(11) **EP 3 980 085 B1**
(45) Date of publication and mention of the grant of the patent: **11.03.2026**
(21) Application number: 20817687.5
(22) Date of filing: 04.06.2020
(51) Int. Cl.: A61L 27/26, A61L 27/22, A61L 27/52, A61L 27/58, A61K 38/44, A61P 25/00, A61K 31/728, A61K 31/375, A61K 31/335, A61K 31/4045, A61P 43/00

(54) **COMPOSITIONS AND METHODS OF USING SAME FOR TISSUE REGENERATION**
ZUSAMMENSETZUNGEN UND VERFAHREN ZU IHRER VERWENDUNG FÜR DIE GEWEBEREGENERATION
COMPOSITIONS ET PROCÉDÉS D'UTILISATION DE CELLES-CI POUR LA RÉGÉNÉRATION TISSULAIRE

(30) Priority: 07.06.2019 US 201962858347 P; 07.06.2019 US 201962858346 P; 07.06.2019 US 201962858344 P
(43) Date of publication of application: 13.04.2022
(73) Proprietor: Ramot at Tel-Aviv University Ltd., 6139201 Tel-Aviv (IL); The Medical Research, Infrastructure, And Health Services Fund Of The Tel Aviv Medical Center, 6423906 Tel-Aviv (IL)
(72) Inventor: ROCHKIND, Shimon, 6949903 Tel-Aviv (IL)
(74) Representative: RGTH
(86) International application number: PCT/IL2020/050631
(87) International publication number: WO 2020/245832

(56) References cited:
- WO-A1-2009/022339
- WO-A1-2018/100511
- WO-A2-2006/057003

## Description

### RELATED APPLICATION

This application claims priority from US Patent Application No. 62/858,344, 62/858,346 and 62/858,347 filed on June 7, 2019.

### FIELD AND BACKGROUND OF THE INVENTION

The present invention relates to compositions and uses of said compossitions for tissue regeneration.

Central nervous system (CNS) injuries such as spinal cord injury (SCI) have no successful treatment to date. SCI is associated with an immediate loss of sensory and motor locomotion with permanent deficit in reflex functions below the level of injury. Furthermore, CNS injuries are usually characterized by total failure to regenerate and heal. Immediate events which occur following SCI include ischemia, an immune response activating microglia cells and infiltration of injured cells from the neighboring vascular tissues, due to apoptotic and necrotic damages to capillary blood vessels. The injury is simultaneously accompanied by nonspecific reactive changes of glial cells e.g. astrocytes and macrophages in response to the damage including secretion of connective tissue matrix substances such as proteoglycans (PGs), collagens and myelin-derived residues. These substances lead to formation of a scar tissue resulting in inhibition of axons sprouting and restricting neuronal regeneration. Indeed, scientific reports describe treatment with anti-gliotic agents such as chondroitinase ABC to induce improvement in the synaptic plasticity and regeneration parameters (e.g. Bradbury EJ et al., Nature. 11;416(6881):636-40, 2002; and Barritt AW et al., J Neurosci. 18;26(42):10856-67, 2006).

Copolymer 1 (also known as Cop-1, glatiramer acetate, GA or COPAXONE^{®}), is an FDA (as well as other regulatory agencies) approved drug for the treatment of MS. The underlying multifactorial anti-inflammatory, neuroprotective effect of Copolymer 1 is in the induction of reactive T cells that release immunomodulatory cytokines and neurotrophic factors at the injury site. These Copolymer 1-induced cytokines and growth factors may have a direct effect on axon function. Indeed, Copolymer 1 treatment was shown to have positive effects on both myelinated and non-myelinated axons resulting in improved axon function in a chronic mouse model of MS (Spencer Moore et al., 2014, Journal of Neuroscience Research, 92:1621-1636). Copolymer 1 was also shown to have a neuroprotective role by reducing synaptic loss during the course of the disease (Scorisa JM et al., 2011, Int J Biol Sci, 7(8):1188-1202).

Hydrated gels (hydrogels) are viscous, semisolid entities at physiological temperatures and pH which can be used for tissue engineering, regenerative medicine and as biomaterials. For example, hydrogels were prepared from polysaccharides (Coviello et al, 2007) such as hyaluronic acid (e.g., using hybrid combinations of hyaluronic acid with calcium phosphate, chitosan, gelatin or alginate) or chitosan (e.g., chitosan with laminin peptides; Suzuki et al., 2003; Itoh et al., 2005; Matzuda et al., 2005, Ho et al., 2005) as well as from synthetic materials such as Poly (2-hydroxyethyl methacrylate). Hyaluronic acid-based hydrogels provide a growth supportive milieu for cells and tissues such as for nerve regeneration (Suzuki et al., 2003; Itoh et al., 2005), while guiding migration and regeneration of nutritional-trophic and anti-oxidative agents.

Laminins are basement membrane glycoproteins which function as adhesive molecules, mediating and interacting with cytoskeleton's bound integrins, cadherins, cell adhesive molecules (CAMs) and extracellular matrix (ECM) constituents and support cell migration, attachment, proliferation, differentiation and survival. Hyaluronic acid-based hydrogels which were modified with laminin were shown to promote neurite extension (Hou et al., 2005). Certain laminin sequence repeats were found to have a biological activity on cell surface receptors. These include IKVAV (SEQ ID NO: 1) and YIGSR (SEQ ID NO: 2) which serve as guiding tracks for migration, regeneration and growth (Tashiro et al., 1989; Powell and Kleinmann, 1997; Niece et al., 2003; Hallmann et al., 2005).

An antioxidant can protect cells or macromolecules (e.g., the polysaccharide) from oxidative stress (oxidative damage caused by free radicals). Thus, the antioxidant can both extend the survival of the macromolecules by preventing their oxidative depolymerization and diminish the oxidative stress conditions created in cultures and in implanted transplantation construct in-vivo.

International Patent Application Publication No. WO2009/022339 discloses the use of a hyaluronic acid-based hydrogel containing the antioxidant sodium dismutase and a laminin peptide for neural tissue regeneration and repair.

WO 2018/100511 A discloses a hydrogel composition comprising a hyaluronic acid, a laminin polypeptide, an antioxidant such as Vitamin E and an anti-gliotic agent (e.g. Copaxone) for the treatment of nerve injury. The anti-gliotic agent is provided at a concentration range of about 5-300 µg/ml in said hydrogel.

Vitamin E is a lipophilic vitamin with antioxidant properties acting as a radical scavenger, delivering an H atom to free radicals and also has various health beneficial effects such as, for example, cholesterol lowering, tumor suppression and inhibition of blood platelet aggregation.

### SUMMARY OF THE INVENTION

The scope of the invention is defined by the claims. Any references in the description to methods of treatment refer to the pharmaceutical compositions of the present invention for use in a method for treatment of the human or animal body by therapy. According to an aspect of the present invention there is provided a composition comprising a hyaluronic acid, a laminin polypeptide, vitamin E and Copolymer 1 at a concentration range of 10 - 150 µg / ml.

According to some embodiments of the invention, the Copolymer 1 concentration range is 10 - 50 µg / ml.

According to some embodiments of the invention, the Copolymer 1 concentration range is 10 - 30 µg / ml.

According to some embodiments of the invention, the Copolymer 1 is at a concentration of 10 µg / ml.

According to some embodiments of the invention, the Vitamin E is at a concentration range of 0.6 - 30 mM.

According to some embodiments of the invention, the Vitamin E is at a concentration range of 1 - 30 mM.

According to some embodiments of the invention, the Vitamin E is at a concentration range of 3 - 30 mM.

According to some embodiments of the invention, the Vitamin E is at a concentration range of 3 - 10 mM.

According to some embodiments of the invention, the Vitamin E is at a concentration of 3 mM.

According to some embodiments of the invention, the hyaluronic acid, the vitamin E and the laminin polypeptide are cross linked.

According to some embodiments of the invention, the laminin polypeptide is set forth by SEQ ID NO: 1.

According to an aspect of some embodiments of the present invention there is provided a matrix comprising the composition.

According to an aspect of some embodiments of the present invention there is provided a hydrogel comprising the composition.

According to some embodiments of the invention, the composition, the matrix or the hydrogel, being formulated for local administration.

According to some embodiments of the invention, the vitamin E is provided at a concentration range of 5 - 40 µg / ml in the hydrogel.

According to some embodiments of the invention, the vitamin E is provided at a concentration range of 1 - 10 mM in the hydrogel.

According to some embodiments of the invention, the hyaluronic acid, the laminin polypeptide and the vitamin E are provided at a total concentration of about 0.01 - 0.6 %.

According to some embodiments of the invention, the hyaluronic acid, the laminin polypeptide and the vitamin E are provided at a total concentration of about 0.2 - 0.4 %.

According to some embodiments of the invention, the agent is provided at a concentration range of 1 - 50 µg / ml in the hydrogel.

According to some embodiments of the invention, the composition, the matrix or the hydrogel, further comprising cells.

According to an aspect of the present invention there is provided a method of inducing ex-vivo formation of a tissue, comprising:
(a) providing the composition, the matrix or the hydrogel and;
   (ii) seeding the composition, the matrix or the hydrogel with cells in a medium suitable for proliferation, differentiation and/or migration of the cells,
   thereby inducing the formation of the tissue.

According to some embodiments of the invention, the cells are stem cells.

According to some embodiments of the invention, the stem cells differentiate into neuronal cells when seeded in the composition, the matrix or the hydrogel.

According to an aspect of some embodiments of the present invention there is provided a method of inducing formation or regeneration of a tissue in a subject in need thereof, the method comprising implanting the composition, the matrix or the hydrogel in the subject, thereby inducing the formation or regeneration of the tissue in the subject.

According to some embodiments of the invention, the tissue is a neural tissue.

According to an aspect of some embodiments of the present invention there is provided a method of inducing formation or regeneration of a neuronal tissue in a subject in need thereof, the method comprising implanting the composition, the matrix or the hydrogel in the subject, thereby inducing the formation or regeneration of the neuronal tissue in the subject.

According to an aspect of some embodiments of the present invention there is provided a method of treating a pathology characterized by diseased, damaged or loss of tissue in a subject in need thereof, the method comprising implanting the composition, the matrix or the hydrogel at or near the diseased, damaged or loss of tissue of the subject, thereby treating the subject.

According to an aspect of some embodiments of the present invention there is provided the composition, the matrix or the hydrogel for use in treating a pathology characterized by diseased, damaged or loss of tissue in a subject in need thereof.

According to some embodiments of the invention, the pathology comprises nerve injury.

According to an aspect of some embodiments of the present invention there is provided a method of treating nerve injury in a subject in need thereof, the method comprising implanting the composition, the matrix or the hydrogel at or near the nerve injury of the subject, thereby treating the nerve injury in the subject.

According to an aspect of some embodiments of the present invention there is provided the composition, the matrix or the hydrogel, for use in treating nerve injury in a subject in need thereof.

According to some embodiments of the invention, the nerve injury is part of the peripheral nervous system (PNS).

According to an aspect of some embodiments of the present invention there is provided a method of preventing or treating neurogenic shock following nerve injury in a subject in need thereof, the method comprising implanting the composition, the matrix or the hydrogel at or near the nerve injury of the subject, thereby preventing or treating the neurogenic shock in the subject.

According to some embodiments of the invention, the implanting is effected within 48 hours following the nerve injury.

According to an aspect of some embodiments of the present invention there is provided the composition, the matrix or the hydrogel for use in preventing or treating neurogenic shock following nerve injury in a subject in need thereof.

According to some embodiments of the invention, the preventing or treating is effected within 48 hours following the nerve injury.

According to some embodiments of the invention, the nerve injury is part of the central nervous system (CNS).

According to some embodiments of the invention, the nerve injury comprises spinal cord injury (SCI).

According to some embodiments of the invention, the nerve injury comprises traumatic brain injuries (TBI) or traumatic optic neuropathy (TON).

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWING(S)

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a schematic representation of the PC12 cells based in-vitro assay for evaluation of Neurite sprouting and outgrowth. A: Seed PC12 cells in complete growth media (CGM); B: Stimulate with NGF 2.5S in incomplete media (IM); C: Change to IM + treatments w/o NGF; D: Analysis of neurite length.
FIG. 2 is a schematic representation of the embryonic spinal cord cells based in-vitro assay for evaluation of Neurite sprouting and outgrowth. A: Embryonic spinal cord dissection and dissociation; B: Seed cells in complete neurobasal media (CNM); C: Change to incomplete neurobasal media (INM) + treatments; D: Analysis of neurite length.
FIG. 3 is a schematic representation of the DRG neurons based in-vitro assay for evaluation of Neurite sprouting and outgrowth. A: Seed primary DRG neuron cells in growth media; B: Add treatments to each well; C and D: Analysis of neurite length.
FIG. 4 is a graph demonstrating the effect of GRG comprising several different antiOxidants on neurites growth in the PC12-based assay. * p < 0.05 vs. G1, ** p < 0.01 vs. G1, *** p < 0.001 vs. G1, # p < 0.05 vs. G2, and #### p < 0.0001 vs. G2 using One-way ANOVA followed by Dunnett's post hoc test.
FIG. 5 is a schematic representation of the in-vivo rat peripheral nerve injury model assay.
FIG. 6 is a graph demonstrating no effect of any of the treatments on rat body weight in the in-vivo rat peripheral nerve injury model assay.
FIG. 7 is a graph demonstrating the effect of the GRG comprising the indicated concentrations of Tocopherol on the clinical signs in the in-vivo rat peripheral nerve injury model assay as compared to empty NeuraGen^{®} nerve guide control.
FIG. 8 illustrates the signal and measurement of the Compound Muscle Action Potentials (CMAPs) in the in-vivo rat peripheral nerve injury model assay. On the left, there is a graphic description showing the amplitude and the "peak to peak" parameters. On the top right: normal signal. On the bottom right a signal following axonal degeneration.
FIGs. 9A-B demonstrate the effect of GRG comprising the indicated concentrations of Tocopherol on CMAPs. Figure 9A is a bar graph demonstrating the amplitude normalized to the contralateral leg at each time-point. * p < 0.05 vs. empty NeuraGen^{®} nerve guide and ** p < 0.01 vs. empty NeuraGen^{®} nerve guide, using T-test. Figure 9B is a bar graph demonstrating peak to peak (ms) values obtained from the left (operated) leg. * p < 0.05 vs. empty NeuraGen^{®} nerve guide and ** p < 0.01 vs. empty NeuraGen^{®} nerve guide, using T-test.
FIG. 10 demonstrate the effect of GRG comprising the indicated concentrations of Tocopherol on Catwalk number of steps recorded from the operated leg.
FIG. 11 is a schematic representation of the in-vitro assay for evaluation of neurite outgrowth. A: Purification of spine primary cells and culture in complete culture media; B: Replacement to incomplete (serum free) culture media and addition of tested compounds; C-E: Analysis of neurite length, total number of cells with neurites, neurite length per cell, number of neurites per cell and total number of bifurcations.
FIG. 12 demonstrates the effect of GRG comprising 3 Mm Tocopherol on Motor Evoked Potentials (MEP) in the in-vivo rat acute spinal cord injury model. The graph shows MEP latency in the right (R) and left (L) legs of each rat tested at each time-point.
FIG. 13 demonstrates the effect of GRG comprising 3 Mm Tocopherol on BBB locomotor rating scale in the in-vivo rat acute spinal cord injury model. The graph shows BBB score in the right (R) and left (L) legs of each rat tested at each time-point.
FIG. 14 demonstrates the effect of GRG comprising 3 Mm Tocopherol on the histopathological score in the in-vivo rat acute spinal cord injury model determined at the end of the experiment. Data is presented as mean ± SEM; * p < 0.05 vs. sham control untreated group, using Student's T-test.
FIG. 15 shows representative H&E stained sections of the injury area demonstrating the effect of the GRG comprising 0.3 mM, 1 mM, 3 mM or 10 mM Tocopherol (Groups 3 - 6, respectively) on formation of a new nerve bundle in the in-vivo rat peripheral nerve injury model assay as compared to empty NeuraGen^{®} nerve guide control (group 2) and autologous transplantation control (group 1).
FIG. 16 is a graph demonstrating the effect of the GRG comprising the indicated concentrations of Tocopherol on the mean number of axons at distal section (only sections with number of axons > 10) in the in-vivo rat peripheral nerve injury model assay as compared to empty NeuraGen^{®} nerve guide control and autologous transplantation control. * p < 0.05 using T-test vs. empty NeuraGen^{®} Nerve Guide.
FIG. 17 is a graph demonstrating the effect of the GRG comprising the indicated concentrations of Tocopherol on the mean normalized number of axons (distal / proximal) in the in-vivo rat peripheral nerve injury model assay as compared to empty NeuraGen^{®} nerve guide control and autologous transplantation control. ** p < 0.01 using F-test vs. empty NeuraGen^{®} Nerve Guide.
FIG. 18 is a graph demonstrating the effect of GRG comprising Copaxone on the percentage of SEPs recorded at week 24 following in the in-vivo rat acute spinal cord injury model.
FIGs. 19A-B demonstrate the effect of GRG comprising Copaxone on regeneration in the in-vivo rat acute spinal cord injury model, as assessed by MBP staining. Figure 18A is a graph demonstrating the mean ± SE of MBP in cranial (CRAN), middle (MID) and caudal (CAUD) sections. Figure 18B is a graph demonstrating the mean ± SE of MBP in the middle (MID) only. * p < 0.01 using two-tailed Student's T-test vs. untreated.

### DESCRIPTION OF SPECIFIC EMBODIMENTS OF THE INVENTION

The present invention, in some embodiments thereof, relates to compositions and methods of using same for tissue regeneration.

While reducing specific embodiments of the invention to practice, the present inventors have designed a novel composition comprising hyaluronic acid, a laminin peptide (SEQ ID NO: 1) and at least one antioxidant (Vitamin E, melatonin, Coenzyme Q10, Acetyl-DL-Carnitine and N-Acetyl-D-Glucosamine) which is capable of supporting regeneration of e.g. neuronal tissue. When formulated as a gel the formulation is referred to herein as GRG for guiding regenerative gel. In addition, the present inventors have designed a combined therapy for the treatment of nerve injury and prevention or treatment of neurogenic shock following nerve injury. The combined treatment is based on a co-treatment with hyaluronic acid, an anti-oxidant, a laminin peptide (SEQ ID NO: 1) and at least one agent [ibuprofen, indomethacin, methylprednisolone, N-acetyl-cysteine, serotonin, a calcium channel blocker, a TNFα inhibitor, an IL-1 inhibitor, an agent capable of upregulating activity of peroxisome proliferator-activated receptor (PPAR), an agent capable of upregulating activity of S1P receptor and/or Copolymer 1 (Copaxone^{®})], which is capable of supporting regeneration of neuronal tissue.

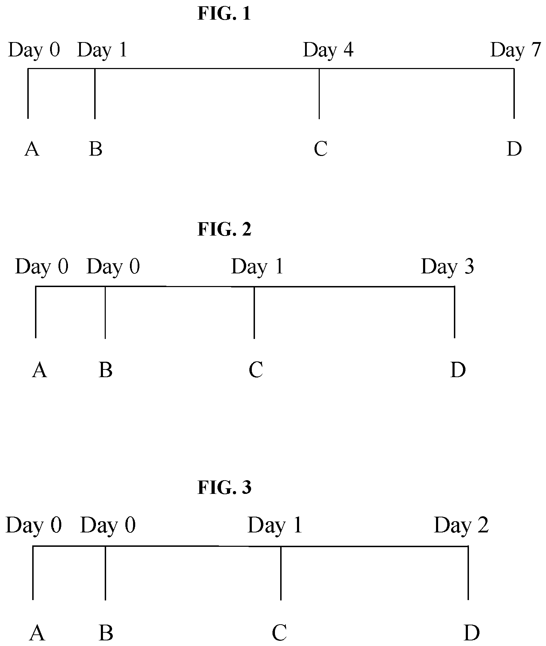

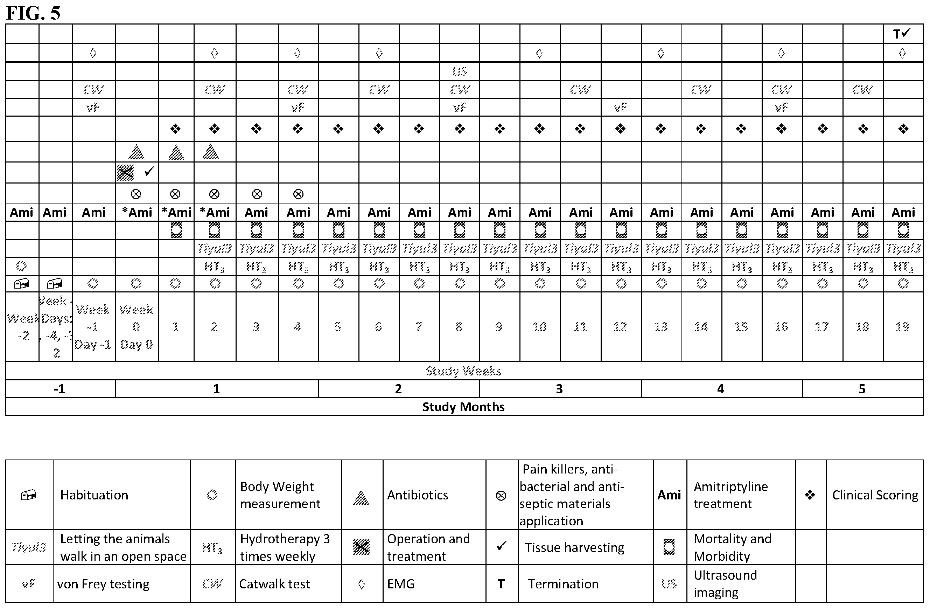

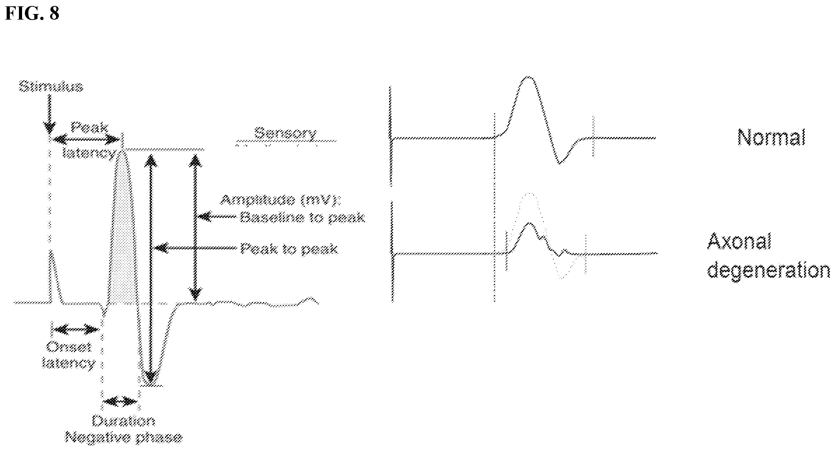

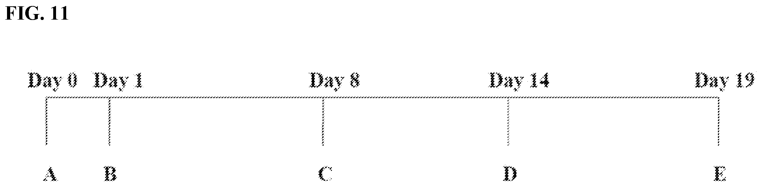

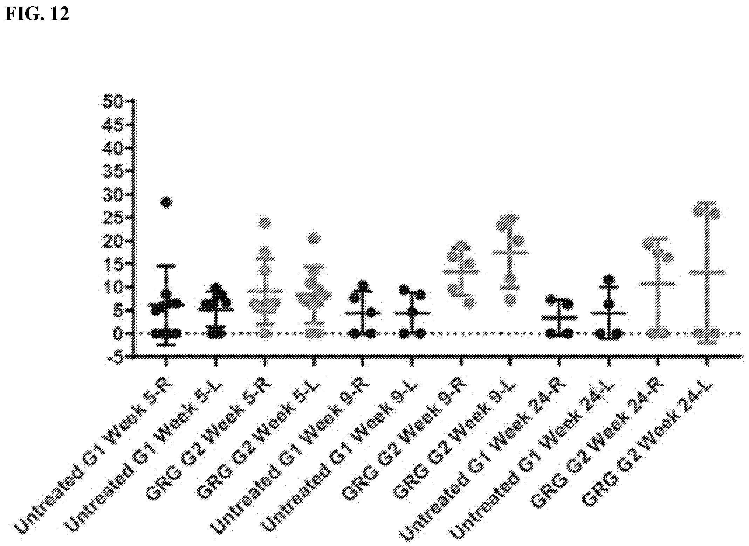

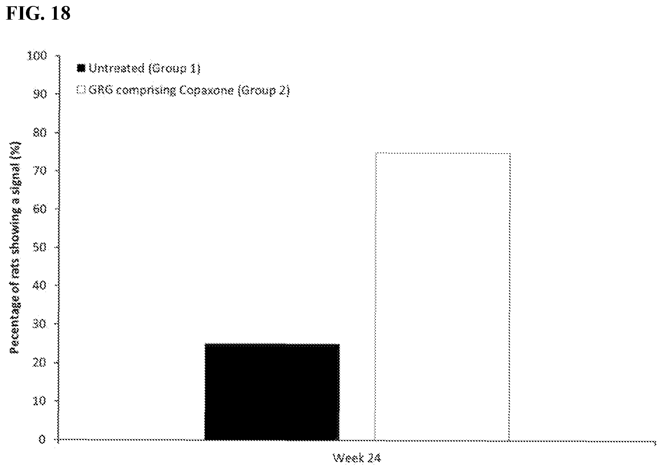

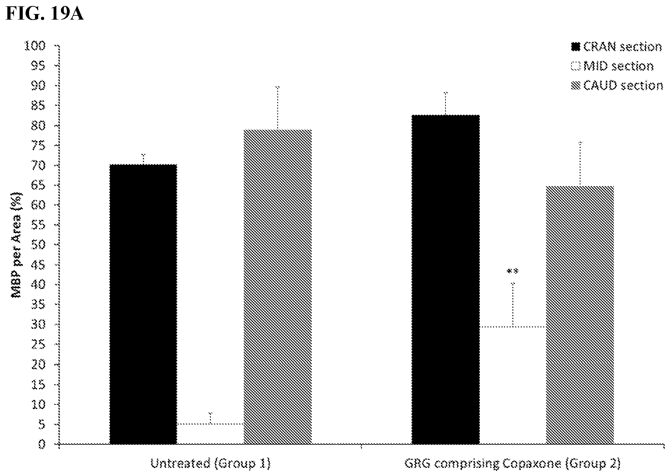

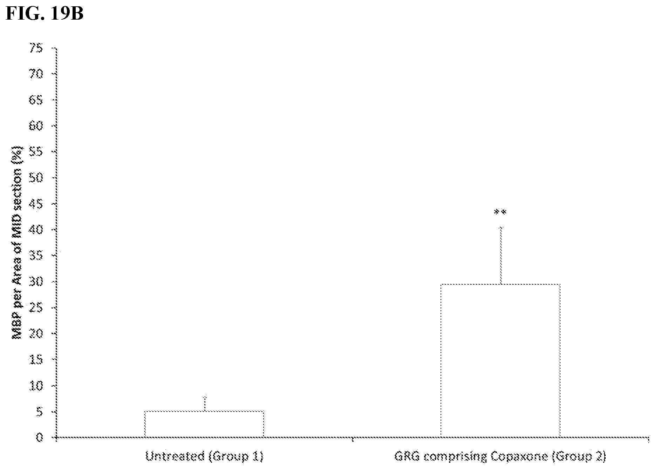

As is illustrated hereinunder and in the Examples section, which follows, the present inventors demonstrate that GRG comprising different antioxidants (e.g. Vitamin E, N-Acetyl-D-glucosamine, Melatonin, Coenzyme Q, Acetyl-L-carnitine) support neurite sprouting and outgrowth in a level comparable to a GRG comprising SOD1 as the antioxidant, in four different in-vitro models (PC12 cells, embryonic spinal cord cells, primary DRG neurons and adult spine primary cells) (Examples 1 and 3, Figures 1-4 and 11). Following, the present inventors demonstrate that a formulation of GRG comprising Vitamin E as the antioxidant at a concentration of 3 mM and 10 mM improved the clinical score, Compound Muscle Action Potentials (CMAPs) and histology in a rat peripheral nerve injury model (Example 2, Figures 5-10 and 15-17). Moreover, a formulation of GRG comprising Vitamin E as the antioxidant at a concentration of 3 mM improved the electrophysiology, BBB locomotor rating and histopathological scores in a rat acute spinal cord injury model (Example 4, Figures 12-14). In addition, a formulation of GRG comprising Vitamin E and Etanercept (Enbrel^{®}) or Copolymer 1 (Copaxone^{®}) significantly increased neurite sprouting and outgrowth compared to a GRG only formulation in an in-vitro adult spine primary cells model (Example 3, Figure 11). Furthermore, a formulation of GRG comprising Copolymer 1 improved the electrophysiology and histological evaluation in a rat acute spinal cord injury model (Example 5, Figures 18 and 19A-B).

According to an alternative or an additional aspect of the present invention, there is provided a composition comprising a hyaluronic acid, a laminin polypeptide, an antioxidant and Copolymer 1 at a concentration range of 10 - 150 µg / ml.

As used herein, the term "hyaluronic acid (HA)", also known as hyaluronan, hyaluronate, refers to an unsulphated glycosaminoglycan composed of repeating disaccharide units of N-acetylglucosamine (GlcNAc) and glucuronic acid (GlcUA) linked together by alternating beta- 1, 4 and beta-1, 3 glycosidic bonds. According to specific embodiments the hyaluronic acid is Na-HA. According to specific embodiments, the hyaluronic acid has a molecular weight from about 10⁴ Daltons to about 3 x 10⁶ Daltons. The molecular weight of HA can be evaluated by e.g. viscosity measurement with a digital viscosimeter Brookfield brand Cone / Plate DVII + Per (Brookfield Engineering Laboratories Inc. Middleboro, MA 02346-1031 USA). The molecular weight of HA can be calculated as well by the discrepancy between the figure obtained in Dische's assays versus the data obtained by Park-Johnson (Park J.T. Johnson M.J. A submicrodetermination of glucose J. Biol. Chem. 181, 149-151, 1949) determination for reducing sugars.

The hyaluronic acid described herein includes naturally occurring HA synthetic HA or a combination of same. According to specific embodiments, the hyaluronic acid can be extracted and isolated from an organism such as rooster combs or umbilical cords or from bacterial cultures such as those of hemolytic group A or C Streptococci, or can be synthetically produced using methods which are well known in the art.

According to specific embodiments of the invention, the hyaluronic acid is pure enough from chemical or biological constituents so that it is biologically inert having a low rate of reactivity with other substances under ordinary conditions.

According to specific embodiments of the invention, the hyaluronic acid is pure enough so that it is biocompatible, e.g., when in contact with cells, tissues or body fluid of an organism does not induce adverse effects such as immunological reactions and/or rejections, cellular death, and the like.

According to specific embodiments, the hyaluronic acid is at least 80 %, at least 90 %, at least 95 %, at least 98 % or at least 99 % pure.

According to specific embodiments, the hyaluronic acid is analytical (i.e. 99.5 % - 100 %) or pharmaceutical grade (98 % - 100 %) hyaluronic acid.

According to specific embodiments, the hyaluronic acid is a hyaluronic acid such as commercially obtained from Lifecore Biomedical LLC Cat No. HAHA15M-1.

The hyaluronic acid described herein is capable of forming highly hydrated gels in aqueous solutions.

Total content of HA in the composition can be determined by methods known in the art such as, but not limited to the content of uronic acids (lucuronic acid) by the routine test of Dische (Dische Z. A new specific color reaction of hexuronic Acids. J. Biol. Chem, 167, 189-197, 1947) employing the carbazol reagent.

As used herein the term "laminin" refers to the family of extracellular matrix glycoproteins, which form the major noncollagenous constituent of basement membrane. Laminins have been implicated in a wide variety of biological processes including cell adhesion, differentiation, migration, signaling, neurite outgrowth and metastasis. Laminins are composed of 3 non identical chains: laminin alpha, beta and gamma, each encoded by a distinct gene.

As used herein the phrase "laminin polypeptide" refers to an amino acid sequence which comprises at least 4 consecutive amino acids of a laminin polypeptide and which exhibits a biological activity (e.g., support cell survival, growth, proliferation, differentiation and/or migration).

According to some embodiments of the invention the laminin polypeptide can include an amino acid sequence of a laminin alpha-chain such as LAMA1 (e.g., GenBank Accession No. NP_005550.2), LAMA2 (e.g., GenBank Accession Nos. NP_000417.2 and NP_001073291.1), LAMA3 (e.g., GenBank Accession Nos. NP_937762.1 and NP_000218.2), LAMA4 (e.g., GenBank Accession Nos. NP_001098677.1, NP_001098676.1, NP_002281.2, NP_001098679.1, and NP_001098678.1), and LAMA5 (e.g., GenBank Accession No. NP_005551.3); a laminin beta-chain such as LAMB1 (e.g., GenBank Accession No. NP_002282.1), LAMB2 (e.g., GenBank Accession No. NP_002283.3), LAMB3 (e.g., GenBank Accession Nos. NP_000219.2 and NP_001017402.1) and LAMB4 (e.g., GenBank Accession No. NP_031382.2); and/or a laminin gamma-chain such as LAMC1 (e.g., GenBank Accession No. NP_002284.3), LAMC2 (e.g., GenBank Accession Nos. NP_005553.2 and NP_061486.2) and LAMC3 (e.g., GenBank Accession No. NP_006050.3).

According to specific embodiments of the invention, the laminin polypeptide includes a repeated amino acid sequence (e.g., a 4 or 5 amino acid repeated sequence) of a laminin sequence.

Non-limiting examples of laminin polypeptides which can be included in the composition of the invention include the peptides set forth in SEQ ID NOs: 1, 2 or 3. [KSIKVAVRSYIGSRCV (SEQ ID NO: 1), IKVAV (SEQ ID NO: 2), YIGSR (SEQ ID NO: 3)].

According to specific embodiments, the laminin polypeptide is set forth by SEQ ID NO: 1 (KSIKVAVRSYIGSRCV).

According to specific embodiments the laminin polypeptide is 5-50, 5-25, 5-20, 16-50 or 16-25 amino acids long.

According to specific embodiments, the laminin polypeptide is at least 16 amino acids long but shorter than 50 amino acids long.

The terms "polypeptide" or "peptide" which are interchangeably used herein, encompass native peptides (either degradation products, synthetically synthesized peptides or recombinant peptides) and peptidomimetics (typically, synthetically synthesized peptides), as well as peptoids and semipeptoids which are peptide analogs, which may have, for example, modifications rendering the peptides more stable while in a body or more capable of penetrating into cells. Such modifications include, but are not limited to N terminus modification, C terminus modification, peptide bond modification, including, but not limited to, CH2-NH, CH2-S, CH2-S=O, O=C-NH, CH2-O, CH2-CH2, S=C-NH, CH=CH or CF=CH, backbone modifications, and residue modification. Methods for preparing peptidomimetic compounds are well known in the art and are specified, for example, in Quantitative Drug Design, C.A. Ramsden Gd., Chapter 17.2, F. Choplin Pergamon Press (1992). Further details in this respect are provided hereinunder.

Peptide bonds (-CO-NH-) within the peptide may be substituted, for example, by N-methylated bonds (-N(CH3)-CO-), ester bonds (-C(R)H-C-O-O-C(R)-N-), ketomethylen bonds (-CO-CH2-), α-aza bonds (-NH-N(R)-CO-), wherein R is any alkyl, e.g., methyl, carba bonds (-CH2-NH-), hydroxyethylene bonds (-CH(OH)-CH2-), thioamide bonds (-CS-NH-), olefinic double bonds (-CH=CH-), retro amide bonds (-NH-CO-), peptide derivatives (-N(R)-CH2-CO-), wherein R is the "normal" side chain, naturally presented on the carbon atom.

These modifications can occur at any of the bonds along the peptide chain and even at several (2-3) at the same time.

Natural aromatic amino acids, Trp, Tyr and Phe, may be substituted for synthetic non-natural acid such as TIC, naphthylelanine (Nol), ring-methylated derivatives of Phe, halogenated derivatives of Phe or o-methyl-Tyr.

In addition to the above, the peptides of some embodiments of the present invention may also include one or more modified amino acids or one or more non-amino acid monomers (e.g. fatty acids, complex carbohydrates etc.).

The term "amino acid" or "amino acids" is understood to include the 20 naturally occurring amino acids; those amino acids often modified post-translationally in-vivo, including, for example, hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids.

Tables 1 and 2 below list naturally occurring amino acids (Table 1) and non-conventional or modified amino acids (e.g., synthetic, Table 2) which can be used with the present invention.

**Table 1**

| ***Amino Acid*** | ***Three-Letter Abbreviation*** | ***One-letter Symbol*** |
|---|---|---|
| alanine | Ala | A |
| Arginine | Arg | R |
| Asparagine | Asn | N |
| Aspartic acid | Asp | D |
| Cysteine | Cys | C |
| Glutamine | Gln | Q |
| Glutamic Acid | Glu | E |
| glycine | Gly | G |
| Histidine | His | H |
| isoleucine | Iie | I |
| leucine | Leu | L |
| Lysine | Lys | K |
| Methionine | Met | M |
| phenylalanine | Phe | F |
| Proline | Pro | P |
| Serine | Ser | S |
| Threonine | Thr | T |
| tryptophan | Trp | W |
| tyrosine | Tyr | Y |
| Valine | Val | V |
| Any amino acid as above | Xaa | X |

**Table 2**

| ***Non-conventional amino acid*** | ***Code*** | ***Non-conventional amino acid*** | ***Code*** |
|---|---|---|---|
| α-aminobutyric acid | Abu | L-N-methylalanine | Nmala |
| α-amino-α-methylbutyrate | Mgabu | L-N-methylarginine | Nmarg |
| aminocyclopropane- | Cpro | L-N-methylasparagine | Nmasn |
| carboxylate | | L-N-methylaspartic acid | Nmasp |
| aminoisobutyric acid | Aib | L-N-methylcysteine | Nmcys |
| aminonorbornyl- | Norb | L-N-methylglutamine | Nmgin |
| carboxylate | | L-N-methylglutamic acid | Nmglu |
| cyclohexylalanine | Chexa | L-N-methylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylomithine | Nmom |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methvlthreonine | Nmthr |
| D-methionine | Dmet | L-N-methyltryptophan | Nmtrp |
| D-ornithine | Dorn | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl-γ-aminobutyrate | Mgabu |
| D-α-methylalanine | Dmala | α-methylcyclohexylalanine | Mchexa |
| D-α-methylarginine | Dmarg | α-methylcyclopentylalanine | Mcpen |
| D-α-methylasparagine | Dmasn | α-methyl-α-napthylalanine | Manap |
| D-α-methylaspartate | Dmasp | α- methylpenicillamine | Mpen |
| D-α-methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3-aminopropyl)glycine | Norm |
| D-α-methylisoleucine | Dmile | N- amino-α-methylbutyrate | Nmaabu |
| D-α-methylleucine | Dmleu | α-napthylalanine | Anap |
| D-α-methyllysine | Dmlys | N-benzylglycine | Nphe |
| D-α-methylmethionine | Dmmet | N-(2-carbamylethyl)glycinc | Ngln |
| D-α-methylornithine | Dmom | N-(carbamylmethyl)glycine | Nasn |
| D-α-methylphenylalanine | Dmphe | N-(2-carboxyethyl)glycine | Nglu |
| D-α-methylproline | Dmpro | N-(carboxymethyl)glycine | Nasp |
| D-α-methylserine | Dmser | N-cyclobutylglycine | Ncbut |
| D-α-methylthreonine | Dmthr | N-cycloheptylglycine | Nchep |
| D-α-methyltryptophan | Dmtrp | N-cyclohexylglycine | Nchex |
| D-α-methyltyrosine | Dmty | N-cyclodecylglycine | Ncdec |
| D-α-methylvaline | Dmval | N-cyclododeclglycine | Ncdod |
| D-α-methylalnine | Dnmala | N-cyclooctylglycine | Ncoct |
| D-α-methylarginine | Dnmarg | N-cyclopropylglycine | Ncpro |
| D-α-methylasparagine | Dnmasn | N-cycloundecylglycine | Ncund |
| D-α-methylasparatate | Dnmasp | N-(2,2-diphenylethyl)glycine | Nbhm |
| D-α-methylcysteine | Dnmcys | N-(3,3-diphenylpropyl)glycine | Nbhe |
| D-N-methylleucine | Dnmleu | N-(3-indolylyethyl) glycine | Nhtrp |
| D-N-methyllysine | Dnmlys | N-methyl-γ-aminobutyrate | Nmgabu |
| N-methylcyclohexylalanine | Nmchexa | D-N-methylmethionine | Dnmmet |
| D-N-methylomithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nva |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(*p*-hydroxyphenyl)glycine | Nhtyr |
| L-*t*-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-*t*-butylglycine | Mtbug |
| L-α-methylcysteine | Mcys | L-methylethylglycine | Metg |
| L-α-methylglutamine | Mgln | L-α-methylglutamate | Mglu |
| L-α-methylhistidine | Mhis | L-α-methylhomo phenylalanine | Mhphe |
| L-α-methylisoleucine | Mile | N-(2-methylthioethyl)glycine | Nmet |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl)glycine | Nser |
| D-N-methylisoleucine | Dnmile | N-(imidazolylethyl)glycine | Nhis |
| D-N-methylleucine | Dnmleu | N-(3-indolylyethyl)glycine | Nhtrp |
| D-N-methyllysine | Dnmlys | N-methyl-γ-aminobutyrate | Nmgabu |
| N-methylcyclohexylalanine | Nmchexa | D-N-methylmethionine | Dnmmet |
| D-N-methylomithine | Dnmom | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(*p*-hydroxyphenyl)glycine | Nhtyr |
| L-*t*-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-*t*-butylglycine | Mtbug |
| L-α-methylcysteine | Mcys | L-methylethylglycine | Metg |
| L-α-methylglutamine | Mgln | L-α-methylglutamate | Mglu |
| L-α-methylhistidine | Mhis | L-α-methylhomophenylalanine | Mhphe |
| L-α-methylisoleucine | Mile | N-(2-methylthioethyl)glycine | Nmet |
| L-α-methylleucine | Mleu | L-α-methyllysine | Mlys |
| L-α-methylmethionine | Mmet | L-α-methylnorleucine | Mnle |
| L-α-methylnorvaline | Mnva | L-α-methylornithine | Morn |
| L-α-methylphenylalanine | Mphe | L-α-methylproline | Mpro |
| L-α-methylserine | mser | L-α-methylthreonine | Mthr |
| L-α-methylvaline | Mtrp | L-α-methyltyrosine | Mtyr |
| L-α-methylleucine | Mval Nnbhm | L-N-methylhomophenylalanine | Nmhphe |
| N-(N-(2,2-diphenylethyl) | | N-(N-(3,3-diphenylpropyl) | |
| carbamylmethyl-glycine | Nnbhm | carbamylmethyl(1)glycine | Nnbhe |
| 1-carboxy-1-(2,2-diphenyl ethylamino)cyclopropane | Nmbc | | |

The peptides of some embodiments of the present invention are preferably utilized in a linear form, although it will be appreciated that in cases where cyclicization does not severely interfere with peptide characteristics, cyclic forms of the peptide can also be utilized.

Since the present peptides can be utilized in therapeutics or diagnostics which require the peptides to be in soluble form, the peptides of some embodiments of the present invention preferably include one or more non-natural or natural polar amino acids, including but not limited to serine and threonine which are capable of increasing peptide solubility due to their hydroxyl-containing side chain.

The peptides of some embodiments of the present invention may be synthesized by any techniques that are known to those skilled in the art of peptide synthesis. For solid phase peptide synthesis, a summary of the many techniques may be found in J. M. Stewart and J. D. Young, Solid Phase Peptide Synthesis, W. H. Freeman Co. (San Francisco), 1963 and J. Meienhofer, Hormonal Proteins and Peptides, vol. 2, p. 46, Academic Press (New York), 1973. For classical solution synthesis see G. Schroder and K. Lupke, The Peptides, vol. 1, Academic Press (New York), 1965.

In general, these methods comprise the sequential addition of one or more amino acids or suitably protected amino acids to a growing peptide chain. Normally, either the amino or carboxyl group of the first amino acid is protected by a suitable protecting group. The protected or derivatized amino acid can then either be attached to an inert solid support or utilized in solution by adding the next amino acid in the sequence having the complimentary (amino or carboxyl) group suitably protected, under conditions suitable for forming the amide linkage. The protecting group is then removed from this newly added amino acid residue and the next amino acid (suitably protected) is then added, and so forth. After all the desired amino acids have been linked in the proper sequence, any remaining protecting groups (and any solid support) are removed sequentially or concurrently, to afford the final peptide compound. By simple modification of this general procedure, it is possible to add more than one amino acid at a time to a growing chain, for example, by coupling (under conditions which do not racemize chiral centers) a protected tripeptide with a properly protected dipeptide to form, after deprotection, a pentapeptide and so forth. Further description of peptide synthesis is disclosed in U.S. Pat. No. 6,472,505.

A preferred method of preparing the peptide compounds of some embodiments of the present invention involves solid phase peptide synthesis.

Large scale peptide synthesis is described by Andersson Biopolymers 2000; 55 (3):227-50.

In cases where large amounts or long polypeptides (e.g., longer than 20 amino acids) are desired, the polypeptides of some embodiments of the present invention can be generated using recombinant techniques such as described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680, Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463.

The composition further comprises an antioxidant which can protect cells or macromolecules (e.g., the polysaccharide) from oxidative stress (oxidative damage caused by free radicals). Thus, the antioxidant can extend the survival of the macromolecules by preventing their oxidative depolymerization.

The antioxidant is vitamin E.

According to other specific embodiments, the composition comprises several distinct (e.g. at least 2, at least 3, at least 4, at least 5) antioxidants.

According to other specific embodiments, the composition comprises at least two distinct antioxidants.

According to specific embodiments, the composition comprises at least Vitamin E and melatonin, at least Vitamin E and Coenzyme Q10, at least Vitamin E and Acetyl-DL-Carnitine or at least Vitamin E and N-Acetyl-D-Glucosamine.

According to specific embodiments, the composition comprises at least Vitamin E and melatonin.

According to specific embodiments, the composition comprises at least Vitamin E and N-Acetyl-D-Glucosamine.

As used herein, the term "vitamin E", also referred to herein as "Tocopherol", includes alpha, beta, gamma, and delta-tocopherols and their derivatives, conjugates, metabolites and salts. The vitamin E may also be a combination of alpha, beta, gamma, and delta-tocopherols.

According to specific embodiments, the vitamin E is alpha Tocopherol.

The vitamin E described herein includes naturally occurring vitamin E synthetic vitamin or a combination of same.

The alpha-form occurs naturally as the d-isomer known as d-alpha-tocopherol (d-2,5,7,8-tetramethyl-2-(4',8',12'-trimethyltridecyl)-6-chromanol). Other non-limiting forms of vitamin E which can be used with specific embodiments of the invention include: d-alpha.-tocopheryl acetate, d-alpha-tocopheryl succinate, d-alpha -tocopheryl nicotinate and d-alpha.-tocopheryl linoleate. Also the corresponding dl forms may be used which include: dl-.alpha.-tocopherol, dl-.alpha.-tocopheryl acetate, dl-alpha-tocopheryl succinate, dl-alpha-tocopheryl nicotinate and dl-alpha.-tocopheryl linoleate and their derivatives, conjugates, metabolites and salts.

Methods of extracting, purifying or synthesizing vitamin E are known in the art. For example, naturally sourced d-alpha-tocopherol can be extracted and purified from seed oils; gamma-tocopherol can be extracted, purified, and methylated to create d-alpha-tocopherol. dl-alpha-tocopherol can be synthesized e.g. from a mixture of toluene and 2,3,5-trimethylhydroquinone that reacts with isophytol to all-rac-alpha-tocopherol, using iron in the presence of hydrogen chloride gas as catalyst.

The antioxidant of some embodiments of the invention can be produced by recombinant techniques, e.g. as described in Hartman JR., et al., 1986 (Proc. Natl. Acad. Sci. USA, Vol: 83, pp 7142-7146). For example, a polynucleotide encoding superoxide dismutase 1 (GenBank Accession No. NM_000454; SEQ ID NO: 5) can be ligated into a nucleic acid construct suitable for expression in a host cell (e.g., bacterial cell, yeast cell, mammalian cell). Such a nucleic acid construct includes a promoter sequence for directing transcription of the polynucleotide sequence in the cell in a constitutive or inducible manner, and may also include sequences which render this vector suitable for replication and integration in prokaryotes, eukaryotes, or preferably both (e.g., shuttle vectors); transcription and translation initiation sequence, enhancers, transcription and translation terminator, and a polyadenylation signal which may increase the efficiency of mRNA translation; a signal sequence for secretion; sequences engineered to enhance stability, production, purification, yield or toxicity of the expressed polypeptide.

The antioxidant can be recovered and purified using a variety of standard protein purification techniques, such as, but not limited to, affinity chromatography, ion exchange chromatography, filtration, electrophoresis, hydrophobic interaction chromatography, gel filtration chromatography, reverse phase chromatography, concanavalin A chromatography, chromate-focusing and differential solubilization.

According to specific embodiments of the invention, the antioxidant is provided such that it is pure enough from chemical or biological constituents to allow for the effective use of the compound or the recombinant polypeptide as an antioxidant. Activity of the anti-oxidant may be determined by methods well known in the art and include measurement at 560 nm as the rate of suppression of reduction of nitrotetrazolium blue when superoxide anion radical was generated during oxidation of xanthine by xanthine oxidase.

According to specific embodiments, the anti-oxidant is at least 80 %, at least 90 %, at least 95 %, at least 98 % or at least 99 % pure.

According to specific embodiments, the anti-oxi is analytical or pharmaceutical grade anti-oxidant.

According to specific embodiments, the compositions described herein may further comprise other agents capable of supporting regeneration of neuronal tissue such as, but not limited to anti-gliotic agents and/or neuronal supporting agents.

According to specific embodiments, the composition described herein may further comprise an anti-gliotic agent.

As used herein, the term "gliosis" refers to a nonspecific change of glial cells e.g. astrocytes and macrophages, in response to damage to the central nervous system (CNS). Typically, gliosis involves proliferation of glial cell, hypertrophy of glial cells and secretion of connective tissue matrix substances such as proteoglycans (PGs), collagens and myelin-derived residues. Gliosis, in its extreme form, leads to the formation of a scar tissue in the CNS comprising dense fibrous network of glial cell in areas of damage resulting in inhibition of axons sprouting and restricting neuronal regeneration.

Methods of determining gliosis are known in the art and are further described in the Examples section which follows and include in-vitro methods determining neuronal cells survival and astrocytes survival and quality, biosynthesis and accumulation of inhibitory intracellular, pericellular and extracellular (ECM) components such as GAGs; and in-vivo methods determining neuronal regeneration in response to CNS injury e.g. SCI.

As used herein, the term "anti-gliotic agent" refers to an agent capable of decreasing the extent of gliosis. Typically, an anti-gliotic agent decreases the extent of gliosis by degrading the scar barrier and/or inhibiting its further formation. According to specific embodiments the decrease is at least 1.05 fold, at least 1.1 fold, at least 1.2 fold, at least 1.3 fold, at least 1.4 fold, 1.5 fold, at least 2 fold, at least 3 fold, at least 5 fold, at least 10 fold, or at least 20 fold as compared to same in the absence of the anti-gliotic agent.

According to other specific embodiments the decrease is by at least 5 %, by at least a 10 %, at least 20 %, at least 30 %, at least 40 % or at least 50 % as compared to same in the absence of the anti-gliotic agent.

Non limiting examples of anti-gliotic agents which can be used with specific embodiments of the present invention include Chondroitinase ABC (E.C. No 4.2.2.4), β-D-xyloside (E.C. No 217.897.1), Collagenase Type I (E.C. No 232-582-9), Mitomycin-C (CAS No 50-07-7), MMP-3-Matrix Metalloproteinase (E.C. No 3.4.24), anti Nogo A, anti-TGFβ 1, 2 & 3, angiotensin Converting Enzyme (ACEa, E.C No 3.4.15.1), anti NG-2-domain, Decorin (e.g. human Decorin such as Uniprot accession No. P07585, PAPN-beta aminopropionyl, Mannose-6-phosphate (CAS No 3672-15-9), Oxidized recombinant human galectin-1, Copaxone (glatiamer acetate) and Tri peptide (ser-gly-gly).

According to specific embodiments, the compositions described herein may further comprise a neuronal supporting agent.

Non limiting examples of neuronal supporting agents which can be used with specific embodiments of the present invention include Copolymer 1 (e.g. glatiramer acetate or its trade name Copaxone^{®}), ibuprofen, indomethacin, methylprednisolone, N-acetyl-cysteine, serotonin, a calcium channel blocker (e.g. Verapamil) a TNFα inhibitor (e.g. Etanercept, Infliximab), an IL-1 inhibitor (e.g. canacinumab, Anakinra), an agent capable of upregulating activity of peroxisome proliferator-activated receptor (PPAR, e.g. Rosiglitazone) and an agent capable of upregulating activity of S1P receptor (e.g. fingolimod).

According to specific embodiments, the "neuronal supporting agent" is capable of increasing sprouting, growth, survival and/or function of a neuronal cell. Methods of determining neuronal sprouting, growth, survival and/or function are known in the art and are further described in the Examples section which follows and include in-vitro methods determining neuronal cells growth and survival; and in-vivo methods determining neuronal regeneration in response to CNS injury e.g. SCI.

According to specific embodiments the increase is at least 1.05 fold, at least 1.1 fold, at least 1.2 fold, at least 1.3 fold, at least 1.4 fold, 1.5 fold, at least 2 fold, at least 3 fold, at least 5 fold, at least 10 fold, or at least 20 fold as compared to same in the absence of the neuronal supporting agent.

According to other specific embodiments the increase is by at least 2 %, at least 5 %, at least a 10 %, at least 20 %, at least 30 %, at least 40 % or at least 50 % as compared to same in the absence of the neuronal supporting agent.

According to specific embodiments, the composition comprises at least one of the neuronal supporting agents disclosed herein.

According to specific embodiments, the composition comprises one of the neuronal supporting agents disclosed herein.

According to other specific embodiments, the composition comprises several distinct (e.g. at least 2, at least 3, at least 4, at least 5) of the neuronal supporting agents disclosed herein.

According to other specific embodiments, the composition comprises at least two distinct neuronal supporting agents disclosed herein.

According to specific embodiments, the agent is ibuprofen (CAS NO: 15687-27-1). Ibuprofen can be commercially obtained from e.g. Millipore.

According to specific embodiments, the agent is indomethacin (CAS NO: 53-86-1). Indomethacin can be commercially obtained from e.g. Teva.

According to specific embodiments, the agent is methylprednisolone (CAS NO: 83-43-2). Methylprednisolone can be commercially obtained from e.g. Sigma.

According to specific embodiments, the agent is N-acetyl-cysteine (CAS NO: 616-91-1). N-acetyl-cysteine can be commercially obtained from e.g. Sigma.

According to specific embodiments, the agent is serotonin, also known as 5-hydroxytryptamine (5-HT) (CAS NO: 50-67-9). Serotonin can be commercially obtained from e.g. Sigma.

According to specific embodiments, the agent is a calcium channel blocker.

As used herein, the term "calcium channel blocker" refers to an agent which inhibits the transmembrane flux of calcium (Ca2+) ions. The calcium channel blocker may be a polypeptide, a nucleic acid (including DNA or RNA), an antibody, a small molecule and any combinations thereof.

According to specific embodiments, the calcium channel blocker is a small molecule. Numerous calcium-channel blockers are known in the art, such as, but not limited to amlodipine, bepridil, nitrendipine, diltiazem, felodipine, flunarizine, isradipine, mibefradil, nicardipine, nifedipine, nimodipine, nisoldipine, nivaldipine, and verapamil.

According to specific embodiments, the calcium channel blocker is Verapamil (CAS NO: 52-53-9). Verapamil can be commercially obtained from e.g. Alomone.

According to specific embodiments, the agent is a TNFα inhibitor.

As used herein the term "TNFa inhibitor" refers to an agent which down-regulates activity and/or expression of TNFα.

According to a specific embodiment the TNFα inhibitor specifically inhibits TNFα and not an activator or effector thereof.

Thus, according to specific embodiments, the TNFα inhibitor binds TFNα.

"TNFα" refers to the polynucleotide or polypeptide expression product of the TNFA gene (Gene ID: 7124). According to specific embodiments, the TNFα refers to the human TNFα, such as provided in the following Accession Numbers: NM_000594 and NP_000585.

As used herein, "down-regulating activity and/or expression" or "down-regulates activity and/or expression refers to a decrease of at least 5 % in biological function and/or expression in the presence of the agent in comparison to same in the absence of the agent, as determined by e.g. PCR, ELISA, Western blot analysis, immunopercipitation, flow cytometry, immuno-staining, kinase assays.

Down-regulating activity and/or expression may be effected at the genomic [e.g. targeted homologous recombination, site specific recombinases (e.g. Cre recombinase and Flp recombinase), PB transposases and genome editing by engineered nucleases (e.g. meganucleases, Zinc finger nucleases (ZFNs), transcription-activator like effector nucleases (TALENs) and CRISPR/Cas system)] and/or the transcript level using a variety of molecules which interfere with transcription and/or translation (e.g., RNA silencing agents e.g. dsRNAs, siRNAs, miRNAs, shRNAs, antisense) or on the protein level (e.g., aptamers, small molecules and inhibitory peptides, antagonists, enzymes that cleave the polypeptide, antibodies and the like).

According to specific embodiments, measures should be taken to use molecules that penetrate the cell membrane or modified to enter through the cell membrane.

According to specific embodiments, the inhibitor is a small molecule or a peptide which binds and/or interferes with activity of the target.

According to specific embodiments, the inhibitor is a molecule which binds to and/or cleaves the target. Such molecules can be a small molecule, an inhibitory peptide.

Another down-regulating agent which can be used along with some embodiments of the invention is an aptamer. As used herein, the term "aptamer" refers to double stranded or single stranded RNA molecule that binds to specific molecular target, such as a protein. Various methods are known in the art which can be used to design protein specific aptamers. The skilled artisan can employ SELEX (Systematic Evolution of Ligands by Exponential Enrichment) for efficient selection as described in Stoltenburg R, Reinemann C, and Strehlitz B (Biomolecular engineering (2007) 24(4):381-403).

According to specific embodiments the inhibitor is an antibody.

The term "antibody" as used in this invention includes intact molecules as well as functional fragments thereof (such as Fab, F(ab')2, Fv, scFv, dsFv, or single domain molecules such as VH and VL) that are capable of binding to an epitope of an antigen.

Suitable antibody fragments for practicing some embodiments of the invention include a complementarity-determining region (CDR) of an immunoglobulin light chain (referred to herein as "light chain"), a complementarity-determining region of an immunoglobulin heavy chain (referred to herein as "heavy chain"), a variable region of a light chain, a variable region of a heavy chain, a light chain, a heavy chain, an Fd fragment, and antibody fragments comprising essentially whole variable regions of both light and heavy chains such as an Fv, a single chain Fv (scFv), a disulfide-stabilized Fv (dsFv), an Fab, an Fab', and an F(ab')2.

According to specific embodiments, the antibody is an intracellular antibody.

According to specific embodiments, the antibody is a monoclonal antibody.

According to other specific embodiments, the antibody is a polyclonal antibody.

Methods of producing polyclonal and monoclonal antibodies as well as fragments thereof are well known in the art (See for example, Harlow and Lane, Antibodies: A Laboratory Manual, Cold Spring Harbor Laboratory, New York, 1988.

According to specific embodiments, the antibody is a human or a humanized antibody.

Methods for producing human antibodies and humanizing non-human antibodies are well known in the art.

Humanized forms of non-human (e.g. murine) antibodies are chimeric molecules of immunoglobulins, immunoglobulin chains or fragments thereof (such as Fv, Fab, Fab', F(ab').sub.2 or other antigen-binding subsequences of antibodies) which contain minimal sequence derived from non-human immunoglobulin. Humanized antibodies include human immunoglobulins (recipient antibody) in which residues form a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

According to specific embodiments, the TNFα inhibitor is Infliximab. Infliximab can be commercially obtained e.g. as Remicade^{®} from e.g. Janssen Biotech.

Other inhibitors that can be used according to some embodiments of the present invention including nucleotides, expression vectors, small molecules, peptides, fusion proteins and fragments which bind the target or its receptor, non-functional target polypeptides, soluble target polypeptides or fragments thereof that bind to the target receptor and prevent binding of the endogenous target, or non-functional receptor, soluble receptor or fragments thereof that bind to the target and prevent binding to the endogenous receptor.

Thus, non-limiting examples of TNFα inhibitor which can be used with specific embodiments of the invention include nucleotides, expression vectors, small molecules, peptides, fusion proteins and fragments targeting TNFα or TNFα receptor, non-functional TNFα, soluble TNFα or fragments thereof that bind to TNFα receptor and prevent binding of the endogenous TNFα, or non-functional TNFα receptor, soluble TNFα receptor or fragments thereof that bind to TNFα and prevent binding to the endogenous receptor.

According to specific embodiments, the TNFα inhibitor is a fusion protein.

According to specific embodiments, the TNFα inhibitor is Etanercept (Enbrel^{®}), CAS NO: 185243-69-0. Etanercept can be commercially obtained from e.g. Amgen, Pfizer and Takeda Pharmaceuticals.

According to specific embodiments, the agent is an IL-1 inhibitor.

As used herein the term "IL-1 inhibitor" refers to an agent which downregulates activity and/or expression of IL-1. Non-limiting examples of agents that can function as down-regulating agents are described in details hereinabove and below.

As used herein the term "IL1" refers to a superfamily of 11 cytokines and encompasses IL-1α, IL-1β, IL-1Ra, IL-18, IL-36Ra, IL-36α, IL-37, IL-36β, IL-36γ, IL-38 and IL-33.

According to specific embodiments, the IL-1 is IL-1α.

"IL-1α" refers to the polynucleotide or polypeptide expression product of the IL1A gene (Gene ID: 3552). According to specific embodiments, the TNFα refers to the human TNFα, such as provided in the following Accession Numbers: NM_000575 and NP_000566.

According to specific embodiments, the IL-1 is IL-1β.

"IL-1β" refers to the polynucleotide or polypeptide expression product of the IL1B gene (Gene ID: 3553). According to specific embodiments, the TNFα refers to the human TNFα, such as provided in the following Accession Numbers: NM_000576 and NP_000567.

According to a specific embodiment the agent specifically inhibits IL-1 and not an activator or effector thereof.

Thus, according to specific embodiments, the IL-1 inhibitor binds IL-1.

Non-limiting examples of IL-1 inhibitors which can be used with specific embodiments of the invention include nucleotides, expression vectors, small molecules, peptides, fusion proteins and fragments targeting IL-1 or IL-1 receptor, non-functional IL-1, soluble IL-1 or fragments thereof that bind to IL-1 receptor and prevent binding of the endogenous IL-1, or non-functional IL-1 receptor, soluble IL-1 receptor or fragments thereof that bind to IL-1 and prevent binding to the endogenous receptor.

According to specific embodiments, the IL-1 inhibitor is canacinumab. Canacinumab can be commercially obtained as e.g. ILARIS^{®} from e.g. Novartis.

According to specific embodiments, the IL-1 inhibitor is anakinra. Anakinra can be commercially obtained as Kineret^{®} from e.g. Amgen.

According to specific embodiments, the neuronal supporting agent is an agent capable of upregulating activity of peroxisome proliferator-activated receptor (PPAR).

According to a specific embodiment the agent capable of upregulating activity of PPAR specifically inhibits PPAR and not an activator or effector thereof.

Thus, according to specific embodiments, the agent capable of upregulating activity of PPAR binds PPAR.

As used herein, "Peroxisome proliferator-activated receptor (PPAR)" refers to a group of transcription factors and encompasses PPARα, PPARβ, PPARγ and PPARδ.

According to specific embodiments, PPAR is PPARγ.

"PPARγ" refers to the polynucleotide or polypeptide expression product of the *PPARG* gene (Gene ID: 5468). According to specific embodiments, the PPARγ refers to the human PPARγ, such as provided in the following Accession Numbers: NM_005037, NM_015869, NM_138711, NM_138712, NM_001330615, NP_001317544, NP_005028, NP_056953, NP_619725, NP_619726.

According to a specific embodiment the agent specifically up-regulates PPAR and not an activator or effector thereof.

Thus, according to specific embodiments, the agent binds PPAR.

As used herein, "up-regulating activity and/or expression" or "up-regulates activity and/or expression refers to an increase of at least 5 % in biological function and/or expression in the presence of the agent in comparison to same in the absence of the agent, as determined by e.g. PCR, ELISA, Western blot analysis, immunoprecipitation, flow cytometry, immuno-staining, kinase assays.

Up-regulating activity and/or expression can be effected at the protein level (e.g., antibodies, small molecules, peptides and the like) but may also be effected at the genomic level (e.g., activation of transcription via promoters, enhancers, regulatory elements) and/or the transcript level using a variety of molecules which promote transcription and/or translation (e.g., correct splicing, polyadenylation, activation of translation) of a target expression product described herein.

According to specific embodiments, measures should be taken to use molecules that penetrate the cell membrane or modified to enter through the cell membrane.

According to specific embodiments the up-regulating is effected at the nucleic acid level. Thus, the up-regulating agent can also be a molecule which is capable of increasing the transcription and/or translation of an endogenous DNA or mRNA encoding the target protein.

Another up-regulating agent may be an exogenous polynucleotide (DNA or RNA) sequence designed and constructed to express at least a functional portion of the target protein. The coding sequences information for the targets described herein is available from several databases including the GenBank database available through www(dot)ncbi(dot)nlm(dot)nih(dot)gov/, and is further described hereinabove.

Methods of expressing an exogenous protein in mammalian cells are well known in the art.

According to specific embodiments the up-regulating is effected at the polypeptide level. Thus, according to specific embodiments, the up-regulating agent is the naturally occurring activator or a functional derivative or variant thereof which retain the ability to specifically bind to the target protein.

It will be appreciated that a functional analogue of at least a catalytic or binding portion of a target protein can be also used as an up-regulating agent. Thus, according to specific embodiments, the agent is an exogenous polypeptide including at least a functional portion (e.g. catalytic or interaction) of the target protein.

According to specific embodiments, the up-regulating agent is an antibody.

Another up-regulating agent would be a molecule which promotes and/or increases the function (e.g. catalytic or interaction) of the target protein by binding to the target or an intermediate thereof. Such molecules can be, but are not limited to, small molecules, peptides and aptamers, wherein each possibility is a separate embodiment of the invention.

According to specific embodiments, the up-regulating agent is a peptide.

According to specific embodiments, up-regulating the agent is a small molecule.

According to specific embodiments, the agent capable of up-regulating activity of PPAR is Rosiglitazone. Roziglitazone can be commercially obtained from e.g. Sigma.

According to specific embodiments, the neuronal supporting agent is an agent capable of upregulating activity of S1P receptor.

As used herein the term "sphingosine-1-phosphate receptor (S1P receptor or S1PR)" refers to a class of G protein-coupled receptor that is a target of the lipid signaling molecule Sphingosine-1-phosphate (S1P) and encompasses S1PR1, S1PR2, S1PR3, S1PR4 and S1PR5.

According to specific embodiments, S1PR is S1PR1.

"S1PR1", also known as endothelial differentiation gene 1 (EDG1) refers to the polynucleotide or polypeptide expression product of the S1PR1 gene (Gene ID: 1901). According to specific embodiments, the S1PR1 refers to the human S1PR1, such as provided in the following Accession Numbers: NM_001400, NM_001320730, NP_001307659 and NP_001391. According to specific embodiments, S1PR is S1PR2.

"S1PR2" refers to the polynucleotide or polypeptide expression product of the S1PR2 gene (Gene ID: 9294). According to specific embodiments, the S1PR2 refers to the human S1PR2, such as provided in the following Accession Numbers: NM_004230 and NP_004221. According to specific embodiments, S1PR is S1PR3.

"S1PR3" refers to the polynucleotide or polypeptide expression product of the S1PR3 gene (Gene ID: 1903). According to specific embodiments, the S1PR3 refers to the human S1PR3, such as provided in the following Accession Numbers: NM_005226 and NP_005217.

According to specific embodiments, S1PR is S1PR4.

"S1PR4" refers to the polynucleotide or polypeptide expression product of the S1PR4 gene (Gene ID: 8698). According to specific embodiments, the S1PR4 refers to the human S1PR4 such as provided in the following Accession Numbers: NM_003775 and NP_003766. According to specific embodiments, SIPR is S1PR5.

"S1PRS" refers to the polynucleotide or polypeptide expression product of the S1PR5 gene (Gene ID: 53637). According to specific embodiments, the S1PR5 refers to the human S1PR5 such as provided in the following Accession Numbers: NM_030760, NM_001166215, NP_001159687 and NP_110387.

According to a specific embodiment the agent specifically up-regulates S1PR and not an activator or effector thereof.

Thus, according to specific embodiments, the agent binds S1P receptor.

Non-limiting examples of agents capable of up-regulating activity of S1P receptor include fingolimod (FTY720), siponimod, ozanimod, AUY954, AAL(R), sphingosine-1-phosphate, sphingosine analogues, 2-substituted 2-amino-propane-1,3-diol or 2-amino-propanol derivatives of sphingosine, (S)-phosphoric acid mono-[2-amino-3-(-4-octyl-phenylamino)-propyl]ester (VPC 24191; Avanti^{®} Polar Lipids, Inc., Alabaster, Ala.), (R)-phosphoric acid mono-[2-amino-2-(6-octyl-1H-benzoimiazol-2-yl)-ethyl]ester (VPC 23153, Avanti^{®} Polar Lipids, Inc.), CID 2321431 (SID 3714904), CID 5309153 (SID 7967985), CID 665518 (SID 864271), CID 2842253 (SID 7977380), KM10340 (3-(6-tert-butyl-1,1-dimethyl-2,3-dihydro-1H-inden-4-yl)-5-(trifluoromethyl)-1H-pyrazole), TC-SP 14 (CAS# 1257093-40-5), SEW 2871 (CAS# 256414-75-2), RP 001 hydrochloride (CAS# 1306761-53-4), CYM 5442 hydrochloride (CAS# 109404.2-01-9), CS 2100 (CAS# 913827-99-3), CYM 50260, CYM 50308, CYM 5520, CYM 5541, RP 001 hydrochloride, TC-G 1006, A 971432, TC-SP 14 and those agonists described in U.S. Patent Application Publication Nos. 2011 / 012-4605 and 2017 / 0020826; U.S. Pat. Nos. 7,842,685; 7,064,217; 7,208,502; 7,241,790; and 7,638,637; Shurer et al. (2008) ACS Chem. Biol., 3:486-498); Sammani et al. (2011) Am. J. Respir. Cell Mol. Biol., 45:1022-7; and "MLSCN Probe Summary: S1P3 Agonist" by The Scripps Research Institute Molecular Screening Center (available at mli(dot)nih(dot)gov/mli/?dl_id=721).

According to specific embodiments, the agent capable of up-regulating activity of S1P receptor is fingolimod (FTY720). Fingolimod can be commercially obtained from e.g. Sigma

As used herein in the application, the terms "Copolymer 1", "Cop 1", "glatiramer acetate" and "GA", are used interchangeably.

For the purpose of the present invention, "Copolymer 1" is intended to include any peptide or polypeptide, including a random copolymer that cross-reacts functionally with myelin basic protein (MBP) and is able to compete with MBP on the MHC class II in the antigen presentation.

According to some embodiments, the agent comprises at least one copolymer selected from the group consisting of random copolymers comprising one amino acid selected from each of at least three of the following groups: (a) lysine and arginine; (b) glutamic acid and aspartic acid; (c) alanine and glycine; and (d) tyrosine and tryptophan.

According to some embodiments, the copolymers can be composed of L- or D-amino acids or mixtures thereof. As is known by those of skill in the art, L-amino acids occur in most natural proteins. However, D-amino acids are commercially available and can be substituted for some or all of the amino acids used to make the copolymers used in the present invention. According to some embodiments, the present invention contemplates copolymers containing both D- and L-amino acids, as well as copolymers consisting essentially of either L- or D-amino acids.

In one embodiment of the invention, the copolymer contains four different amino acids, each from a different one of the groups (a) to (d).

According to some embodiments, the composition comprises Copolymer 1, a mixture of random polypeptides consisting essentially of the amino acids L-glutamic acid (E), L-alanine (A), L-tyrosine (Y) and L-lysine (K) in an approximate ratio of 1.5:4.8:1:3.6, having a net overall positive electrical charge and of a molecular weight from about 2 KDa to about 40 KDa.

According to some embodiments, the Cop 1 has average molecular weight of about 2 KDa to about 20 KDa, about 4 KDa,7 KDa to about 13 K Da, still about 4 KDa to about 8.6 KDa, of about 5 KDa to 9 KDa, or of about 6.25 KDa to 8.4 KDa. In another embodiment, the Cop 1 has average molecular weight of about 13 KDa to about 20 KDa, about 13,5 KDa to about 18 KDa, with an average of about 15 KDa to about 16 KD, e.g., of 16kDa. Other average molecular weights for Cop 1, lower than 40 KDa, are also encompassed by the present invention. Copolymer 1 of said molecular weight ranges can be prepared by methods known in the art, for example by the processes described in U.S. Patent No. 5,800.808. The Copolymer 1 may be a polypeptide comprising from about 15 to about 100, e.g., from about 40 to about 80, amino acids in length.

In one embodiment of the invention, the agent is Copolymer 1 in the form of its acetate salt known under the generic name glatiramer acetate or its trade name Copaxone^{®} (a trademark of Teva Pharmaceutical Industries Ltd., Petach Tikva, Israel).

The activity of Copolymer 1 for the composition disclosed herein is expected to remain if one or more of the following substitutions is made: aspartic acid for glutamic acid, glycine for alanine, arginine for lysine, and tryptophan for tyrosine.

In another embodiment of the invention, the Copolymer 1-related peptide or polypeptide is a copolymer of three different amino acids each from a different one of three groups of the groups (a) to (d). These copolymers are herein referred to as terpolymers.

In one embodiment, the Copolymer 1-related peptide or polypeptide is a terpolymer containing tyrosine, alanine, and lysine, hereinafter designated YAK, in which the average molar fraction of the amino acids can vary: tyrosine can be present in a mole fraction of about 0.05-0.250; alanine in a mole fraction of about 0.3 - 0.6; and lysine in a mole fraction of about 0.1-0.5. According to some embodiments, the molar ratios of tyrosine, alanine and lysine are about 0.10:0.54:0.35, respectively. It is possible to substitute arginine for lysine, glycine for alanine, and/or tryptophan for tyrosine.

In another embodiment, the Copolymer 1-related peptide or polypeptide is a terpolymer containing tyrosine, glutamic acid, and lysine, hereinafter designated YEK, in which the average molar fraction of the amino acids can vary: glutamic acid can be present in a mole fraction of about 0.005 - 0.300, tyrosine can be present in a mole fraction of about 0.005 - 0.250, and lysine can be present in a mole fraction of about 0.3 - 0.7. According to some embodiments, the molar ratios of glutamic acid, tyrosine, and lysine are about 0.26:0.16:0.58, respectively. It is possible to substitute aspartic acid for glutamic acid, arginine for lysine, and/or tryptophan for tyrosine.

According to some embodiments, the Copolymer 1-related peptide or polypeptide is a terpolymer containing lysine, glutamic acid, and alanine, hereinafter designated KEA, in which the average molar fraction of the amino acids can vary: glutamic acid can be present in a mole fraction of about 0.005 - 0.300, alanine in a mole fraction of about 0.005 - 0.600, and lysine can be present in a mole fraction of about 0.2 - 0.7. According to some embodiments, the molar ratios of glutamic acid, alanine and lysine are about 0.15:0.48:0.36, respectively. It is possible to substitute aspartic acid for glutamic acid, glycine for alanine, and/or arginine for lysine.

According to some embodiments, the Copolymer 1-related peptide or polypeptide is a terpolymer containing tyrosine, glutamic acid, and alanine, hereinafter designated YEA, in which the average molar fraction of the amino acids can vary: tyrosine can be present in a mole fraction of about 0.005 - 0.250, glutamic acid in a mole fraction of about 0.005-0.300, and alanine in a mole fraction of about 0.005-0.800. According to some embodiments, the molar ratios of glutamic acid, alanine, and tyrosine are about 0.21: 0.65:0.14, respectively. It is possible to substitute tryptophan for tyrosine, aspartic acid for glutamic acid, and/or glycine for alanine.

The average molecular weight of the terpolymers YAK, YEK, KEA and YEA can vary between about 2 KDa to 40 KDa, e.g., between about 3 KDa to 35 KDa, e.g., between about 5 KDa to 25 KDa.

Copolymer 1 and related peptides and polypeptides may be prepared by methods known in the art, for example, under condensation conditions using the desired molar ratio of amino acids in solution, or by solid phase synthetic procedures. Condensation conditions include the proper temperature, pH, and solvent conditions for condensing the carboxyl group of one amino acid with the amino group of another amino acid to form a peptide bond. Condensing agents, for example dicyclohexylcarbodiimide, can be used to facilitate the formation of the peptide bond. Blocking groups can be used to protect functional groups, such as the side chain moieties and some of the amino or carboxyl groups against undesired side reactions.

For example, the copolymers can be prepared by the process disclosed in U.S. Patent 3,849,550, wherein the N-carboxyanhydrides of tyrosine, alanine, γ-benzyl glutamate and N ε-trifluoroacetyl-lysine are polymerized at ambient temperatures (20 °C - 26 °C) in anhydrous dioxane with diethylamine as an initiator. The γ-carboxyl group of the glutamic acid can be deblocked by hydrogen bromide in glacial acetic acid. The trifluoroacetyl groups are removed from lysine by 1M piperidine. One of skill in the art readily understands that the process can be adjusted to make peptides and polypeptides containing the desired amino acids, that is, three of the four amino acids in Copolymer 1, by selectively eliminating the reactions that relate to any one of glutamic acid, alanine, tyrosine, or lysine.

The molecular weight of the copolymers can be adjusted during polypeptide synthesis or after the copolymers have been made. To adjust the molecular weight during polypeptide synthesis, the synthetic conditions or the amounts of amino acids are adjusted so that synthesis stops when the polypeptide reaches the approximate length that is desired. After synthesis, polypeptides with the desired molecular weight can be obtained by any available size selection procedure, such as chromatography of the polypeptides on a molecular weight sizing column or gel, and collection of the molecular weight ranges desired. The copolymers can also be partially hydrolyzed to remove high molecular weight species, for example, by acid or enzymatic hydrolysis, and then purified to remove the acid or enzymes.

In one embodiment, the copolymers with a desired molecular weight may be prepared by a process, which includes reacting a protected polypeptide with hydrobromic acid to form a trifluoroacetyl-polypeptide having the desired molecular weight profile. The reaction is performed for a time and at a temperature that is predetermined by one or more test reactions. During the test reaction, the time and temperature are varied and the molecular weight range of a given batch of test polypeptides is determined. The test conditions that provide the optimal molecular weight range for that batch of polypeptides are used for the batch. Thus, a trifluoroacetyl-polypeptide having the desired molecular weight profile can be produced by a process, which includes reacting the protected polypeptide with hydrobromic acid for a time and at a temperature predetermined by test reaction. The trifluoroacetyl-polypeptide with the desired molecular weight profile is then further treated with an aqueous piperidine solution to form a low toxicity polypeptide having the desired molecular weight.

According to some embodiments, a test sample of protected polypeptide from a given batch is reacted with hydrobromic acid for about 10 - 50 hours at a temperature of about 20 - 28 °C. The best conditions for that batch are determined by running several test reactions. For example, in one embodiment, the protected polypeptide is reacted with hydrobromic acid for about 17 hours at a temperature of about 26 °C.

As binding motifs of Cop 1 to MS-associated HLA-DR molecules are known (Fridkis-Hareli et al, 1999), polypeptides derived from Cop 1 having a defined sequence can readily be prepared and tested for binding to the peptide binding groove of the HLA-DR molecules as described in the Fridkis-Hareli et al (1999) publication. Examples of such peptides are those disclosed in WO 00 / 05249 and WO 00 / 05,250 and include the peptides of SEQ ID Nos: 6-37 hereinbelow.

| **SEQ ID NO.** | **Peptide Sequence** |
|---|---|
| 6 | AAAYAAAAAAKAAAA |
| 7 | AEKYAAAAAAKAAAA |
| 8 | AKEYAAAAAAKAAAA |
| 9 | AKKYAAAAAAKAAAA |
| 10 | AEAYAA,KAAAKAAAA |
| 11 | KEAYAAAAAAKAAAA |
| 12 | AEEYAAAAAAKAAAA |
| 13 | AAEYAAAAAAKAAAA |
| 14 | EKAYAAAAAAKAAAA |
| 15 | AAKYEAAAAAKAAAA |
| 16 | AAKYAEAAAAKAAAA |
| 17 | EAAYAAAAAAKAAAA |
| 18 | EKKYAAAAAAKAAAA |
| 19 | EAKYAAAAAAKAAAA |
| 20 | AEKYAAAAAAAAAAA |
| 21 | AKEYAAAAAAAAAAA |
| 22 | AKKYEAAAAAAAAAA |
| 23 | AKKYAEAAAAAAAAA |
| 24 | AEAYKAAAAAAAAAA |
| 25 | KEAYAAAAAAAAAAA |
| 26 | AEEYKAAAAAAAAAA |
| 27 | AAEYKAAAAAAAAAA |
| 28 | EKAYAAAAAAAAAAA |
| 29 | AAKYEAAAAAAAAAA |
| 30 | AAKYAEAAAAAAAAA |
| 31 | EKKYAAAAAAAAAAA |
| 32 | EAKYAAAAAAAAAAA |
| 33 | AEYAKAAAAAAAAAA |
| 34 | AEKAYAAAAAAAAAA |
| 35 | EKYAAAAAAAAAAAA |
| 36 | AYKAEAAAAAAAAAA |
| 37 | AKYAEAAAAAAAAAA |

Such peptides and other similar peptides derived from Cop 1 would be expected to have similar activity as Cop 1. Such peptides, and other similar peptides, are also considered to be within the definition of Cop 1-related peptides or polypeptides and their use is considered to be part of the present invention.

The definition of "Copolymer 1-related peptide or polypeptide" according to the invention is meant to encompass other synthetic amino acid copolymers such as the random four-amino acid copolymers described by Fridkis-Hareli et al., 2002 (as candidates for treatment of multiple sclerosis), namely copolymers (14-, 35- and 50-mers) containing the amino acids phenylalanine, glutamic acid, alanine and lysine (poly FEAK), or tyrosine, phenylalanine, alanine and lysine (poly YFAK), and any other similar copolymer to be discovered that can be considered a universal antigen similar to Copolymer 1.According to specific embodiments, the neuronal supporting agent and/or the anti-gliotic agent is chondroitinase ABC, anti Nogo A, Copolymer 1, serotonin, a TNFα inhibitor and/or an IL-1 inhibitor.

According to specific embodiments, the antioxidants and/or the agents (e.g. small molecules) described herein can be utilized in their free base form or as a pharmaceutically acceptable salt.

As used herein, the phrase "pharmaceutically acceptable salt" refers to a charged species of the parent compound and its counter-ion, which is typically used to modify the solubility characteristics of the parent compound and/or to reduce any significant irritation to an organism by the parent compound, while not abrogating the biological activity and properties of the administered compound. A pharmaceutically acceptable salt of a compound as described herein can alternatively be formed during the synthesis of the compound, e.g., in the course of isolating the compound from a reaction mixture or re-crystallizing the compound.

Non-limiting examples of suitable salts comprise alkali metal salts (for example sodium and/or potassium salts), alkaline earth metal salts (for example magnesium and/or calcium salts), aluminium salts, ammonium salts, salts of suitable organic bases (for example salts of alkylamines and/or N-methyl-D-glutamine), salts of amino acids (for example salts of arginine and/or lysine).

In the context of some of the present embodiments, a pharmaceutically acceptable salt of the compounds described herein may optionally be an acid addition salt comprising at least one basic (e.g., amine) group of the compound which is in a positively charged form (e.g., wherein the basic group is protonated), in combination with at least one counter-ion, derived from the selected base, that forms a pharmaceutically acceptable salt.

The acid addition salts of the compounds described herein may therefore be complexes formed between one or more basic groups of the compound and one or more equivalents of an acid.

Depending on the stoichiometric proportions between the charged group(s) in the compound and the counter-ion in the salt, the acid additions salts can be either mono-addition salts or poly-addition salts.

The phrase "mono-addition salt", as used herein, refers to a salt in which the stoichiometric ratio between the counter-ion and charged form of the compound is 1:1, such that the addition salt includes one molar equivalent of the counter-ion per one molar equivalent of the compound.

The phrase "poly-addition salt", as used herein, refers to a salt in which the stoichiometric ratio between the counter-ion and the charged form of the compound is greater than 1:1 and is, for example, 2:1, 3:1, 4:1 and so on, such that the addition salt includes two or more molar equivalents of the counter-ion per one molar equivalent of the compound.

An example, without limitation, of a pharmaceutically acceptable salt would be an ammonium cation or guanidinium cation and an acid addition salt thereof.

The acid addition salts may include a variety of organic and inorganic acids, such as, but not limited to, hydrochloric acid which affords a hydrochloric acid addition salt, hydrobromic acid which affords a hydrobromic acid addition salt, acetic acid which affords an acetic acid addition salt, ascorbic acid which affords an ascorbic acid addition salt, benzenesulfonic acid which affords a besylate addition salt, camphorsulfonic acid which affords a camphorsulfonic acid addition salt, citric acid which affords a citric acid addition salt, maleic acid which affords a maleic acid addition salt, malic acid which affords a malic acid addition salt, methanesulfonic acid which affords a methanesulfonic acid (mesylate) addition salt, naphthalenesulfonic acid which affords a naphthalenesulfonic acid addition salt, oxalic acid which affords an oxalic acid addition salt, phosphoric acid which affords a phosphoric acid addition salt, toluenesulfonic acid which affords a p-toluenesulfonic acid addition salt, succinic acid which affords a succinic acid addition salt, sulfuric acid which affords a sulfuric acid addition salt, tartaric acid which affords a tartaric acid addition salt and trifluoroacetic acid which affords a trifluoroacetic acid addition salt. Each of these acid addition salts can be either a mono-addition salt or a poly-addition salt, as these terms are defined herein.

The present embodiments further encompass any enantiomers, diastereomers, solvates, and/or hydrates of the compounds described herein.

As used herein, the term "enantiomer" refers to a stereoisomer of a compound that is superposable with respect to its counterpart only by a complete inversion / reflection (mirror image) of each other. Enantiomers are said to have "handedness" since they refer to each other like the right and left hand. Enantiomers have identical chemical and physical properties except when present in an environment which by itself has handedness, such as all living systems. In the context of the present embodiments, a compound may exhibit one or more chiral centers, each of which exhibiting an R- or an S-configuration and any combination, and compounds according to some embodiments of the present invention, can have any their chiral centers exhibit an R- or an S-configuration.

The term "diastereomers", as used herein, refers to stereoisomers that are not enantiomers to one another. Diastereomerism occurs when two or more stereoisomers of a compound have different configurations at one or more, but not all of the equivalent (related) stereocenters and are not mirror images of each other. When two diastereoisomers differ from each other at only one stereocenter they are epimers. Each stereo-center (chiral center) gives rise to two different configurations and thus to two different stereoisomers. In the context of the present invention, embodiments of the present invention encompass compounds with multiple chiral centers that occur in any combination of stereo-configuration, namely any diastereomer.

The term "solvate" refers to a complex of variable stoichiometry (e.g., di-, tri-, tetra-, penta-, hexa-, and so on), which is formed by a solute (the compound of the present invention) and a solvent, whereby the solvent does not interfere with the biological activity of the solute. Suitable solvents include, for example, ethanol, acetic acid and the like.

The term "hydrate" refers to a solvate, as defined hereinabove, where the solvent is water.

According to specific embodiments, the components of the composition are cross-linked.

According to specific embodiments, the hyaluronic acid, laminin polypeptide and the antioxidant or the at least two distinct antioxidants are cross linked.

According to specific embodiments, the hyaluronic acid, laminin polypeptide, the antioxidant or the at least two distinct antioxidants and the neuronal supporting agent and/or an anti-gliotic agent are cross linked.

According to specific embodiments, the hyaluronic acid, the vitamin E and the laminin peptide of the composition are cross-linked.

According to specific embodiments, the hyaluronic acid, the vitamin E, the laminin peptide and the neuronal supporting agent and/or an anti-gliotic agent of the composition are cross-linked.

Cross-linking (*i.e*., binding via covalent or ionic bonds) of the components comprised in the composition can be performed using any cross-linking or coupling agent known in the art. Basically the principles of cross linking is combining free primary amino groups with carboxyl groups, or oxidizing in between close hydroxyl groups, forming reactive aldehydes, to interact either among themselves or with amines of additional conjugate may be formed via tiol residues.

According to specific embodiments, cross-linking does not affect the biological activities of the bonded elements.

Non-limiting examples of suitable cross-linking agents include dimethyl suberimidate (an imidoester cross linker); Bis(Sulfosuccinimidyl) suberate (BS3; an NHS-ester cross linker); formaldehyde; 1-Ethyl-3-[3-dimethylaminopropyl]carbodiimide hydrochloride (EDC; the carbodiimide cross linker); N-hydroxyuccinimide (NHS) [Mao J.S, et al., Biomaterials. 24,1621-1629, 2003; Choi Y.S., et al., J. Biomed. Mater. Res. 48,631-639, 1999; Richert L., et al., Biomacromolecules, 5, 284-294, 2004)]; Divinyl sulfone (DVS); and genipin [Sung H.W., et al., J Biomed. Mater. Res. A, 64A:427-438, 2003; Chen SC., et al., J. Control Release. 96, 285-300, 2004; Mwale F., et al., Tissue Eng., 11, 130-40, 2005; Chen H., et al., Biomacromolecules, 7, 2091-2098, 2006]. For ex-vivo or in-vivo cross-linking photo-reactive amino acid analogs (e.g., diazirine analogs to leucine and methionine) can be added to the composition and following exposure to ultraviolet light, the diazirines are activated and bind to interacting side chains (e.g., carboxyl or amino groups).

According to specific embodiments of the invention, cross-linking is performed using a non-toxic and/or biocompatible agent. Examples include, but are not limited to 3-dimenthy-aminoprophyl)-N-ethyl carbodiimide (EDC-N; Sigma-Aldrich- Fluka, St Louis, Missouri 63178, Catalogue No. 03459), divinyl sulfone (DVS; Sigma, Catalogue No. V-370-0) and genipin (Sigma Catalogue No. G-4796).

Thus, according to specific embodiments, there is provided a composition as disclosed herein wherein the hyaluronic acid, the antioxidant and the laminin polypeptide are cross linked.

According to other specific embodiments, there is provided a composition as disclosed herein wherein the hyaluronic acid, the antioxidant, the laminin polypeptide and the neuronal supporting agent and/or the anti-gliotic agent (e.g. Copolymer 1) are cross linked.

According to specific embodiments, the composition described herein has combined improved activity on neural cells survival, neuronal regeneration and/or prevention of glial scar tissue growth. As used herein the phrase "combined improved activity" refers to at least additive but preferably synergistically improved activity.

It should be noted that since the components comprised in the composition of some embodiments of the invention can be prepared using synthetic or recombinant techniques they are obtainable sterile preparations of analytical or pharmaceutical grade.

As mentioned above, the present inventors have generated a novel hydrogel from a hyaluronic acid, a laminin polypeptide (SEQ ID NO: 1) and an antioxidant (e.g. Vitamin E). Further, the present inventors have generated a novel hydrogel from a hyaluronic acid, a laminin polypeptide (SEQ ID NO: 1), an antioxidant (SOD, Vitamin E) and a neuronal supporting agent (e.g. Copolymer 1).

Thus, according to specific embodiments, the composition described herein is formulated as a hydrogel.

According to specific embodiments of the invention, there is provided a hydrogel comprising the composition described herein.

According to another aspect of the present invention, there is provided a method of generating a hydrogel, the method comprising suspending the composition described herein in water so as to obtain a suspension which comprises at least 40 % water, thereby generating the hydrogel.

According to specific embodiments, the method of generating a hydrogel further comprises adding an anti-gliotic agent and/or a neuronal supporting agent to the suspension.

According to an additional or an alternative aspect of the present invention, there is provided a method of generating a hydrogel, the method comprising:
(i) suspending a composition comprising a hyaluronic acid, a laminin polypeptide and an antioxidant in water so as to obtain a suspension which comprises at least 40 % water; and
(ii) adding to said suspension an agent selected from the group consisting of ibuprofen, indomethacin, methylprednisolone, N-acetyl-cysteine, serotonin, a calcium channel blocker, a TNFα inhibitor, an IL-1 inhibitor, an agent capable of upregulating activity of peroxisome proliferator-activated receptor (PPAR) and an agent capable of upregulating activity of S1P receptor,
thereby generating the hydrogel.

According to an additional or an alternative aspect of the present invention, there is provided a method of generating a hydrogel, the method comprising:
(i) suspending a composition comprising a hyaluronic acid, a laminin polypeptide and an antioxidant in water so as to obtain a suspension which comprises at least 40 % water; and
(ii) adding to said suspension at least two agents selected from the group consisting of ibuprofen, indomethacin, methylprednisolone, N-acetyl-cysteine, serotonin, a calcium channel blocker, a TNFα inhibitor, an IL-1 inhibitor, an agent capable of upregulating activity of peroxisome proliferator-activated receptor (PPAR), an agent capable of upregulating activity of S1P receptor and Copolymer 1.
thereby generating the hydrogel.

According to an additional or an alternative aspect of the present invention, there is provided a method of generating a hydrogel, the method comprising:
(i) suspending a composition comprising a hyaluronic acid, a laminin polypeptide and an antioxidant in water so as to obtain a suspension which comprises at least 40 % water; and
(ii) adding to said suspension Copolymer 1 at a concentration below 5 µg / ml in the hydrogel,
thereby generating the hydrogel.

According to an alternative or an additional aspect of the present invention, there is provided a method of generating a hydrogel, the method comprising:
(i) suspending a composition comprising a hyaluronic acid, a laminin polypeptide and an antioxidant in water so as to obtain a suspension which comprises at least 40 % water; and
(ii) adding to said suspension Copolymer 1 at a concentration range of 10-150 µg / ml in the hydrogel,
thereby generating the hydrogel.

According to an alternative or an additional aspect of the present invention, there is provided a method of generating a hydrogel, the method comprising:
(i) suspending a composition comprising a hyaluronic acid, a laminin polypeptide and an antioxidant in water so as to obtain a suspension which comprises at least 40 % water; and
(ii) adding to said suspension glatiramer acetate at a concentration below 5 µg / ml in the hydrogel,
thereby generating the hydrogel.

According to an alternative or an additional aspect of the present invention, there is provided a method of generating a hydrogel, the method comprising:
(i) suspending a composition comprising a hyaluronic acid, a laminin polypeptide and an antioxidant in water so as to obtain a suspension which comprises at least 40 % water; and
(ii) adding to said suspension glatiramer acetate at a concentration range of 10 150 µg / ml in the hydrogel,
thereby generating the hydrogel.

According to specific embodiments, the suspending is effected according to the teachings of International Patent Application Publication No. WO2009/022339.

As used herein, the term "hydrogel" refers to a material comprising the composition of some embodiment of the invention and water, in which the water constitutes more than 40 %.

According to specific embodiments of the invention, the hydrogel comprises at least about 50 %, at least about 60 % water, at least about 70 % water, at least about 80 % water, at least about 90 % water, at least about 95 % water, at least about 96 % water, at least about 97 % water, at least about 98 % water, at least about 99 % water.

According to specific embodiments, the hydrogel is viscous (e.g. approximately 0cP during no movement and 110-130cP during movement).

According to specific embodiments, the hydrogel is transparent.

According to specific embodiments, the hyaluronic acid is provided at a concentration range of about 0.2 - 1 %, about 0.4 - 1 %, about 0.7 - 1 %, about 0.3 - 2 %, e.g., about 0.4 - 1.8 %, e.g., about 0.5 - 1.6, e.g., about 0.5 - 1.5 %, e.g., about 0.6 - 1.4 %, e.g., about 0.7 - 1.4 %, about 0.8 - 1.2 %, e.g., about 1.2 % in the composition e.g. hydrogel.

According to a specific embodiment, the hyaluronic acid is provided at a concentration range of about 0.5 - 1.5 % in the composition e.g. hydrogel.

According to a specific embodiment, the hyaluronic acid is provided at a concentration range of about 0.2 - 1 % in the composition e.g. hydrogel.

According to a specific embodiment, the hyaluronic acid is provided at a concentration of about 0.2 % in the composition e.g. hydrogel.

According to a specific embodiment, the hyaluronic acid is provided at a concentration of about 0.4 % in the composition e.g. hydrogel.

According to a specific embodiment, the hyaluronic acid is provided at a concentration of about 0.7 % in the composition e.g. hydrogel.

According to a specific embodiment, the hyaluronic acid is provided at a concentration of 0.2 % in the composition e.g. hydrogel.

According to a specific embodiment, the hyaluronic acid is provided at a concentration of 0.4 % in the composition e.g. hydrogel.

According to a specific embodiment, the hyaluronic acid is provided at a concentration of 0.7 % in the composition e.g. hydrogel.

According to some embodiments, the laminin polypeptide (e.g., SEQ ID NO: 1) is provided at a concentration range of about 10 - 200 µg / ml, e.g., about 10 - 100 µg / ml, e.g., about 20 - 100 µg / ml, e.g., about 50 µg / ml in the composition e.g. hydrogel.

According to a specific embodiment, the laminin polypeptide is provided at a concentration range of about 20 - 100 µg / ml in the composition e.g. hydrogel.

According to a specific embodiment, the laminin polypeptide is provided at a concentration range of about 1 - 100 µg / ml in the composition e.g. hydrogel.

According to a specific embodiment, the laminin polypeptide is provided at a concentration range of about 10 - 100 µg / ml in the composition e.g. hydrogel.

According to a specific embodiment, the laminin polypeptide is provided at a concentration of about 10 µg / ml in the composition e.g. hydrogel.

The antioxidant is vitamin E.

According to some embodiments of the invention, the antioxidant is provided at a concentration range of 8 µM to 8 mM in the hydrogel. For example, the antioxidant can be provided at a concentration range of 0.5 µg / ml to 200 µg / ml, e.g., from 1 µg / ml to 100 µg / ml, e.g., from 2 µg / ml to 80 µg / ml, e.g., from 4 µg / ml to 40 µg / ml, e.g., from 5 µg / ml to 0 µg / ml, e.g., from 10 µg / ml to 50 µg / ml, e.g., from 15 µg / ml to 40 µg / ml, e.g., from 20 µg / ml to 30 µg / ml, e.g., 25 µg / ml.

According to specific embodiments, the antioxidant is provided at a concentration range of 0.3 - 300 µg / ml, 3 - 300 µg / ml or 3 - 300 µg / ml in the composition e.g. hydrogel.

According to specific embodiments, the antioxidant is provided at a concentration range of 5 - 40 µg / ml in the composition e.g. hydrogel.

According to some embodiments of the invention, the antioxidant is provided at a concentration range of 0.3 mM (e.g. for Vitamin E 129 µg / ml) to 30 mM (e.g. for Vitamin E 12.9 mg / ml) in the hydrogel. For example, the antioxidant can be provided at a concentration range of 129 µg / ml, - 12.9 mg / ml, e.g., 200 µg / ml - 10 mg / ml, e.g., 250 µg / ml - 7.5 mg / ml, e.g., 300 µg / ml µg / ml - 5 mg / ml, 1 mg/ml - 5 mg / ml, e.g., 1 mg / ml - 4.5 mg / ml, e.g., 1 mg / ml - 2.5 mg / ml, e.g. about 1.29 mg / ml.

According to specific embodiments, the antioxidant is provided at a concentration range of 0.3 - 30 Mm, 0.6 - 30 mM, 1 - 30 mM, 1.5 - 30 mM, 2 - 30 mM, 2.5 - 30 mM, 3 - 30 mM, 5 - 30 mM, 10 - 30 mM, or 20 - 30 mM.

According to specific embodiments, the antioxidant is provided at a concentration range of 0.3 - 10 Mm, 0.3 - 5 mM, 0.3 - 3 mM, 0.6 - 10 mM, 0.6 - 5 mM, 0.6 - 3 mM, 1 - 10 mM, 1 - 5 mM or 1 - 3 mM.

According to specific embodiments, the antioxidant is provided at a concentration range of 0.001 - 100 mM. 0.001 - 50 mM, 0.01 - 100 mM, 1 - 100 mM, 1 - 50 mM in the composition e.g. hydrogel.

According to specific embodiments, the antioxidant is provided at a concentration range of 0.3 - 30 mM in the composition e.g. hydrogel.

According to specific embodiments, the antioxidant is provided at a concentration range of 0.6 - 30 mM in the composition e.g. hydrogel.

According to specific embodiments, the antioxidant is provided at a concentration range of 1 - 30 mM in the composition e.g. hydrogel.

According to specific embodiments, the antioxidant is provided at a concentration range of 3 - 30 mM in the composition e.g. hydrogel.

According to specific embodiments, the antioxidant is provided at a concentration range of 1 - 10 mM in the composition e.g. hydrogel.

According to specific embodiments, the antioxidant is provided at a concentration range of 3 - 10 mM in the composition e.g. hydrogel.

According to specific embodiments, the antioxidant is provided at a concentration range of 2.5 - 5 mM in the composition e.g. hydrogel.

According to specific embodiments, the antioxidant is provided at a concentration of 3 mM in the composition e.g. hydrogel.

According to specific embodiments, the antioxidant is provided at a concentration range of 0.3 - 3000 µg / ml in the composition e.g. hydrogel.

According to specific embodiments, the antioxidant s provided at a concentration range of 0.3 - 300 µg / ml, 3 - 300 µg / ml or 3 - 300 µg / ml in the composition e.g. hydrogel.

According to specific embodiments, the antioxidant s provided at a concentration range of 0.3 - 3 nM.

According to specific embodiments, the antioxidant is provided at a concentration range of 0.3 - 3 µM.

According to specific embodiments, the antioxidant is provided at a concentration range of 30 - 300 µg / ml.

According to specific embodiments, the ratio between the hyaluronic acid, the laminin polypeptide and the antioxidant in the composition e.g. hydrogel is between HA 0.01 mg: laminin polypeptide 50 µg: antioxidant 250 µg per ml to HA 1.2 mg: laminin polypeptide 50 µg: antioxidant 250 µg per ml.

According to a specific embodiment, the ratio between the hyaluronic acid, the laminin polypeptide and the antioxidant in the composition e.g. hydrogel is approximately HA 0.4 mg: laminin polypeptide 50µg: antioxidant 250µg per ml.

According to specific embodiments, the ratio between the hyaluronic acid, the laminin polypeptide and the antioxidant in the composition e.g. hydrogel is HA 0.1 - 1 % : laminin polypeptide 0.1 - 100 µg / ml : antioxidant (e.g. Vitamin E) 0.1 - 10 mM.

According to a specific embodiment, the ratio between the hyaluronic acid, the laminin polypeptide and the antioxidant in the composition e.g. hydrogel is approximately HA 0.2 %: laminin polypeptide 10 µg / ml : antioxidant (e.g. Vitamin E) 3 mM.

According to a specific embodiment, the ratio between the hyaluronic acid, the laminin polypeptide and the antioxidant in the composition e.g. hydrogel is approximately HA 0.4 %: laminin polypeptide 10 µg % ml : antioxidant (e.g. Vitamin E) 3 mM.

According to a specific embodiment, the ratio between the hyaluronic acid, the laminin polypeptide and the antioxidant in the composition e.g. hydrogel is approximately HA 0.7 %: laminin polypeptide 10 µg / ml : antioxidant (e.g. Vitamin E) 3 mM.

According to specific embodiments, the hyaluronic acid, the laminin polypeptide and the antioxidant are provided at a total concentration of about 0.01 - 0.6 %.

According to specific embodiments, the hyaluronic acid, the laminin polypeptide and the antioxidant are provided at a total concentration of about 0.02 - 0.5 %.

According to specific embodiments, the hyaluronic acid, the laminin polypeptide and the antioxidant are provided at a total concentration of about 0.2 - 0.4 %.

According to specific embodiments, the hyaluronic acid, the laminin polypeptide and the antioxidant are provided at a total concentration of about 0.4 %.

According to specific embodiments, the hyaluronic acid, the laminin polypeptide and the antioxidant are provided at a total concentration of about 0.2 %.

According to specific embodiments, the hyaluronic acid, the laminin polypeptide and the antioxidant are provided at a total concentration of about 0.02 %.

According to specific embodiments, the neuronal supporting agent and/or anti-gliotic agent is provided at a concentration range of 1 - 500 µg / ml, 0.1 - 100 µg / ml, 0.1 - 50 µg / ml or 1 - 50 µg / ml in the composition e.g. hydrogel.

According to specific embodiments, the neuronal supporting agent and/or anti-gliotic agent is provided at a concentration range of 5 - 300 µg / ml in the composition e.g. hydrogel.

According to specific embodiments, the neuronal supporting agent and/or anti-gliotic agent is provided at a concentration range of 10 - 300 µg / ml, 10 - 250 µg / ml, 10 - 200 µg / ml, 10 - 150 µg / ml, 10 - 100 µg / ml, 10 - 50 µg / ml, 10 - 40 µg / ml, 10 - 30 µg / ml or 10 - 20 µg / ml in the composition e.g. hydrogel.

According to specific embodiments, the neuronal supporting agent and/or anti-gliotic agent is provided at a concentration range of 1 - 50 µg / ml in the composition e.g. hydrogel.

According to specific embodiments, the neuronal supporting and/or anti-gliotic agent is provided at a concentration of 10 µg / ml in the composition e.g. hydrogel.

According to specific embodiments, the Copolymer 1 is provided at a concentration range of 10 - 300 µg / ml, 10 - 250 µg / ml, 10 - 200 µg / ml, 10 - 150 µg / ml, 10 - 100 µg / ml, 10 - 50 µg / ml, 10 - 40 µg / ml, 10 - 30 µg / ml or 10 - 20 µg / ml in the composition e.g. hydrogel.

According to specific embodiments, the Copolymer 1 is provided at a concentration range of 10 - 150 µg / ml in the composition e.g. hydrogel.

According to specific embodiments, the Copolymer 1 is provided at a concentration range of 10 - 50 µg / ml in the composition e.g. hydrogel.

According to specific embodiments, the Copolymer 1 is provided at a concentration range of 10 - 30 µg / ml in the composition e.g. hydrogel.

According to specific embodiments, the Copolymer 1 is provided at a concentration of 10 µg / ml in the composition e.g. hydrogel.

According to some embodiments of the invention, the composition is corsslinked. Methods and agents that can be used for cross-linking are well known in the art and were further described hereinabove.

According to specific embodiments of the invention, the hydrogel is lyophilized by methods well known in the art such that a dry matrix is obtained.

According to specific embodiments, the dry mix comprises less than 50 %, less than 30 %, less than 10 %, less than 5 %, less than 2 %, less than 1 %, or less than 0.5 % water. It should be noted that water-free matrices can be preserved for long periods of time without being subjected to enzymatic degradation or contamination (e.g., by microorganisms).

Thus, according to specific embodiments, the composition described herein is formulated as a matrix.

According to specific embodiments, there is provided a matrix comprising the composition described herein.

As used herein the phrase "matrix" refers to a two-dimensional or a three-dimensional scaffold (also referred to herein as supporting framework) comprising the composition of some embodiments of the invention. The scaffold may further provide mechanical stability and support.

The matrix can be kept in a dry or wet form, or can be frozen according to the intended use.

According to specific embodiments, the dry matrix can be further hydrated in an aqueous solution (e.g., water) until a hydrogel is formed.

According to specific embodiments, the dimensions of the matrix vary according with the lesion (e.g. nerve injury e.g. spinal cord injury) to be treated. For example, the size of the matrix can be smaller than or substantially the same size as the lesion to be treated. Alternatively, the size of the matrix can be larger than the lesion.

Scaffold material may comprise natural or synthetic organic polymers that can be gelled, or polymerized or solidified (e.g., by aggregation, coagulation, hydrophobic interactions, or cross-linking) into a two-dimensional or a three-dimensional structure.

The scaffold of some embodiments of the present invention may be made uniformly of a single polymer, co-polymer or blend thereof. However, it is also possible to form a scaffold according to the invention of a plurality of different polymers. There are no particular limitations to the number or arrangement of polymers used in forming the scaffold. Any combination which is biocompatible, may be formed into fibers, and degrades at a suitable rate, may be used.

Both the choice of polymer and the ratio of polymers in a co-polymer may be adjusted to optimize the stiffness of the scaffold. The molecular weight and cross-link density of the scaffold may also be regulated to control both the mechanical properties of the scaffold and the degradation rate (for degradable scaffolds). The mechanical properties may also be optimized to mimic those of the tissue at the implant site.

Polymers used in scaffold material compositions may be biocompatible, biodegradable and/or bioerodible and may act as adhesive substrates for cells. In exemplary embodiments, structural scaffold materials are easy to process into complex shapes and have a rigidity and mechanical strength suitable to maintain the desired shape under in-vivo conditions.

In certain embodiments, the structural scaffold materials may be non-resorbing or non-biodegradable polymers or materials. Such non-resorbing scaffold materials may be used to fabricate materials which are designed for long term or permanent implantation into a host organism.

The phrase "non-biodegradable polymer", as used herein, refers to a polymer or polymers which at least substantially (i.e. more than 50 %) do not degrade or erode in-vivo. The terms "non-biodegradable" and "non-resorbing" are equivalent and are used interchangeably herein. Examples of biocompatible non-biodegradable polymers which are useful as scaffold materials include, but are not limited to, polyethylenes, polyvinyl chlorides, polyamides such as nylons, polyesters, rayons, polypropylenes, polyacrylonitriles, acrylics, polyisoprenes, polybutadienes and polybutadiene-polyisoprene copolymers, neoprenes and nitrile rubbers, polyisobutylenes, olefinic rubbers such as ethylene-propylene rubbers, ethylene-propylene-diene monomer rubbers, and polyurethane elastomers, silicone rubbers, fluoroelastomers and fluorosilicone rubbers, homopolymers and copolymers of vinyl acetates such as ethylene vinyl acetate copolymer, homopolymers and copolymers of acrylates such as polymethylmethacrylate, polyethylmethacrylate, polymethacrylate, ethylene glycol dimethacrylate, ethylene dimethacrylate and hydroxymethyl methacrylate, polyvinylpyrrolidones, polyacrylonitrile butadienes, polycarbonates, polyamides, fluoropolymers such as polytetrafluoroethylene and polyvinyl fluoride, polystyrenes, homopolymers and copolymers of styrene acrylonitrile, cellulose acetates, homopolymers and copolymers of acrylonitrile butadiene styrene, polymethylpentenes, polysulfones, polyesters, polyimides, polyisobutylenes, polymethylstyrenes, and other similar compounds known to those skilled in the art.

In other embodiments, the structural scaffold materials may be a "bioerodible" or "biodegradable" polymer or material.

The phrase "biodegradable polymer" as used herein, refers to a polymer or polymers which degrade in-vivo, and wherein erosion of the polymer or polymers over time occurs concurrent with or subsequent to release of the composition. The terms "biodegradable" and "bioerodible" are equivalent and are used interchangeably herein.

Such bioerodible or biodegradable scaffold materials may be used to fabricate temporary structures. Examples of biocompatible biodegradable polymers which are useful as scaffold materials include, but are not limited to, polylactic acid, polyglycolic acid, polycaprolactone, and copolymers thereof, polyesters such as polyglycolides, polyanhydrides, polyacrylates, polyalkyl cyanoacrylates such as n-butyl cyanoacrylate and isopropyl cyanoacrylate, polyacrylamides, polyorthoesters, polyphosphazenes, polypeptides, polyurethanes, polystyrenes, polystyrene sulfonic acid, polystyrene carboxylic acid, polyalkylene oxides, alginates, agaroses, dextrins, dextrans, polyanhydrides, biopolymers such as collagens and elastin, alginates, chitosans, glycosaminoglycans, and mixtures of such polymers. In still other embodiments, a mixture of non-biodegradable and bioerodible and/or biodegradable scaffold materials may be used to form a biomimetic structure of which part is permanent and part is temporary.

According to specific embodiments, PLA, PGA and PLA / PGA copolymers are used for forming the scaffolds of some embodiments of the present invention.

In an exemplary embodiment, scaffolds materials may comprise naturally occurring substances, such as, fibrinogen, fibrin, thrombin, chitosan, collagen, alginate, poly(N-isopropylacrylamide), albumin, collagen, synthetic polyamino acids, prolamines, polysaccharides such as alginate, heparin, and other naturally occurring biodegradable polymers of sugar units.

According to specific embodiments, the scaffolds are porous. The porosity may be controlled by a variety of techniques known to those skilled in the art.

According to a specific embodiment of the present invention, the scaffolds are fabricated from synthetic biomaterials and are capable of conducting electricity and naturally eroding inside the body. In an exemplary embodiment, the scaffolds comprise a biocompatible polymer capable of conducting electricity e.g. a polypyrrole polymer. Polyaniline, polyacetyline, poly-p-phenylene, poly-p-phenylene-vinylene, polythiophene, and hemosin are examples of other biocompatible polymers that are capable of conducting electricity and may be used in conjunction with the present invention. Other erodible, conducting polymers are well known (for example, see Zelikin et al., Erodible Conducting Polymers for Potential Biomedical Applications, Angew. Chem. Int. Ed. Engl., 2002, 41(1):141-144). Any of the foregoing electrical conducting polymers can be applied or coated onto a malleable or moldable scaffold.

The scaffolds may be made by any of a variety of techniques known to those skilled in the art. Salt-leaching, porogens, solid-liquid phase separation (sometimes termed freezedrying), and phase inversion fabrication may all be used to produce porous scaffolds. Fiber pulling and weaving (see, e.g. Vacanti, et al., (1988) Journal of Pediatric Surgery, 23: 3-9) may be used to produce scaffolds having more aligned polymer threads. Those skilled in the art will recognize that standard polymer processing techniques may be exploited to create polymer scaffolds having a variety of porosities and microstructures.

Scaffold materials are readily available to one of ordinary skill in the art, usually in the form of a solution (suppliers are, for example, BDH, United Kingdom, and Pronova Biomedical Technology a.s. Norway). For a general overview of the selection and preparation of scaffolding materials, see the American National Standards Institute publication No. F2064-00 entitled Standard Guide for Characterization and Testing of Alginates as Starting Materials Intended for Use in Biomedical and Tissue Engineering Medical Products Applications".

According to specific embodiments the scaffold comprises a biodegradable membrane e.g. a dura film such as Lyodura, AESClJLAP.

According to specific embodiments, the composition, the matrix or the hydrogel of some embodiments of the present invention is comprised in an implantable tube. Such implantable tubes are well known in the art and disclosed for example in Jones et al. Int. J. Mol. Sci. 2016, 17, 1494 and include, but are not limited to NeuraGen^{®} nerve guide obtained from Integra, NeuroTube^{®} from Synovis Micro, NeuroFlex^{™} and NeuroMatrix^{™} from Collagen Matrix, NeuroMend ^{™} from Collagen Matrix, Neurolac^{®} from Polyganics BV, Groningen, Netherlands, SaluTunnel^{™} from Salumedica LLC, Avance^{®} from AxoGen, AxoGuard^{®} from AxoGen, Nerbridge from Toyobo, NeuraWrap^{™} from Integra LifeSciences Co., and acellular nerve allograft.

According to specific embodiments of the invention, the composition, the hydrogel or the matrix further comprises ex-vivo seeded cells such as stem cells or differentiated cells (e.g. neuronal progenitor cells).

According to specific embodiments, the cells are stem cells.

Non-limiting examples of stem cells which can be used by the invention include embryonic stem cells, induced pluripotent stem cells (iPS), neuronal progenitor cells, hematopoietic stem cells (e.g., bone marrow stem cells, cord blood cells, peripheral blood stem cells), adult stem cells and mesenchymal stem cells.

According to some embodiments of the invention, the stem cells are neuronal progenitor cells (such as those obtained from fetal neuronal tissue or brain)

According to specific embodiments, the stem cells differentiate into neuronal cells when seeded in the composition, the matrix or the hydrogel.

Non-limiting examples of differentiated cells which can be used by the invention include neural cells, retina cells, epidermal cells, hepatocytes, pancreatic cells, osseous cells, cartilaginous cells, elastic cells, fibrous cells, myocytes, myocardial cells, endothelial cells, smooth muscle cells, and hematopoietic cells (e.g., lymphocytes).

According to specific embodiments, the differentiated cells are neural cells.

As mentioned above and described in the Examples section which follows the composition developed by the present inventors (e.g. in the form of a hydrogel) can increase neuronal cells function and survival and support neuronal regeneration.

Thus, according to an aspect of some embodiments of the invention, there is provided a method of inducing ex-vivo formation of a tissue, comprising:
(a) providing the composition the matrix or the hydrogel and;
   (ii) seeding said composition, said matrix or said hydrogel with cells in a medium suitable for proliferation, differentiation and/or migration of said cells,
   thereby inducing the formation of the tissue.

The phrase "tissue" refers to a group of cells that perform a similar function. Examples include, but are not limited to, brain tissue, neuronal tissue, retina, skin tissue, hepatic tissue, pancreatic tissue, bone, cartilage, connective tissue, blood tissue, muscle tissue, cardiac tissue, vascular tissue, renal tissue, pulmonary tissue, gonadal tissue, hematopoietic tissue.

According to some embodiments of the invention, the phrase "tissue" also encompasses an "organ", *i.e.,* a fully differentiated structural and functional unit in an animal that is specialized for some particular function. Non-limiting examples of organs include head, brain, eye, bone (e.g., of leg and hand), heart, liver kidney, lung, pancreas, ovary, testis, and stomach.

According to some embodiments of the invention the tissue is a neural tissue.

The term "seeding" refers to plating, placing and/or dropping the cells of the within, under or on the matrix or the hydrogel of the invention.

The concentration of the seeded cells depends on the type of cells used and the concentration of the matrix or hydrogel's components.

The medium used according to this aspect of the invention can be any tissue culture medium suitable for inducing the proliferation, differentiation and/or migration of the cells (e.g., stem cells) of the invention into more specialized *(i.e.,* differentiated) cells. According to some embodiments of the invention the culture medium is supplemented with minerals, amino acids and/or nutrients (e.g., Eagle's culture medium supplemented with glutamine and glucose), or further with serum and/or growth factors.

Following seeding, the matrices or the hydrogels may be routinely examined using a microscope (e.g., an inverted microscope, an axioplan light microscope or an electronic microscope) for evaluation of cell growth, spreading and tissue formation.

According to some embodiments of the invention, the ex-vivo formed tissue can be further implanted in a subject. In such cases the cells seeded within the matrix or the hydrogel can be derived from the treated individual (autologous source) or from allogeneic sources such as embryonic stem cells which are not expected to induce an immunogenic reaction.

As used herein, the term "subject" refers to a mammalian subject (e.g., human being) of any gender and any age including neonatal, infant, juvenile, adolescent, adult and elderly adult.

According to some embodiments of the invention, the term encompasses individuals who suffer from a pathology (e.g. nerve injury, neurogenic shock) as described below.

According to specific embodiment, the subject is demonstrating symptom(s) characterizing the pathology.

Veterinary uses are also contemplated. Thus, according to specific embodiments, the components of the composition, the matrix and the hydrogel are selected avoiding xeno responses.

According to an aspect of the invention there is provided a method of inducing formation or regeneration of a tissue (e.g. neuronal tissue) in a subject in need thereof, the method comprising implanting the composition, the matrix or the hydrogel in the subject, thereby inducing the formation or regeneration of the tissue in the subject.

According to another aspect of the invention there is provided a method of treating a pathology characterized by diseased, damaged or loss of tissue in a subject in need thereof, the method comprising implanting the composition, the matrix or the hydrogel of present invention at or near the diseased, damaged or loss of tissue of the subject, thereby treating the subject.

According to another aspect of the invention there is provided the composition, the matrix or the hydrogel for use in treating a pathology characterized by diseased, damaged or loss of tissue in a subject in need thereof.

As used herein the phrase "pathology characterized by diseased, damaged or loss of tissue" refers to any disorder, disease or condition exhibiting a tissue damage *(i.e.,* non-functioning tissue, cancerous or pre-cancerous tissue, broken tissue, fractured tissue, fibrotic tissue, or ischemic tissue) or a tissue loss (e.g., following a trauma, an infectious disease, a genetic disease, and the like) which require tissue regeneration. Non-limiting examples of disorders or conditions requiring tissue regeneration include, but are not limited to, nerve injuries, liver cirrhosis such as in hepatitis C patients (liver), Type-1 diabetes (pancreas), cystic fibrosis (lung, liver, pancreas), bone cancer (bone), burn and wound repair (skin), age related macular degeneration (retina), myocardial infarction, myocardial repair, articular cartilage defects (chondrocytes), bladder degeneration, intestinal degeneration, and the like.

According to specific embodiments, the pathology comprises nerve injury.

Hence, according to an aspect of some embodiments of the invention, there is provided method of treating nerve injury in a subject in need thereof, the method comprising implanting the composition, the matrix or the hydrogel at or near the nerve injury of the subject, thereby treating the nerve injury in the subject.

According to an additional or an alternative aspect of some embodiments of the invention, there is provided the composition, the matrix or the hydrogel for use in treating nerve injury in a subject in need thereof.

As used herein the phrase "nerve injury" refers to any disorder, disease, or condition exhibiting damage *(i.e.,* non-functioning tissue, cancerous or pre-cancerous tissue, broken tissue, fractured tissue, fibrotic tissue, or ischemic tissue) or loss (e.g., following a trauma, an infectious disease, a genetic disease, and the like) of neuronal tissue which requires tissue regeneration. According to specific embodiments the nerve injury is caused by trauma and not by a disease.

According to specific embodiments the neuronal tissue and/or the nerve injury is part of the peripheral nervous system (PNS).

According to specific embodiments the neuronal tissue and/or the nerve injury is part of the central nervous system (CNS).

The term "central nervous system (CNS)", as used herein can refer to a subject's brain, spinal cord and/or optic nerve.

According to specific embodiments, the nerve injury comprises spinal cord injury.

As used herein, the phrase "spinal cord injury (SCI)" refers to an injury to the spinal cord that is caused by trauma and not by disease. Spinal cord injuries have many causes; according to specific embodiments, the SCI is caused by a major trauma from motor vehicle accidents, falls, sports injuries or violence. Depending on where the spinal cord and nerve roots are damaged, the symptoms can vary widely e.g. from pain to paralysis to incontinence. The SCIs can be incomplete or complete injury which means a total loss of function. According to specific embodiments, the SCI is complete SCI.

According to specific embodiments, the nerve injury comprises traumatic brain injury (TBI).

As used herein, the phrase "traumatic brain injury (TBI)" refers to brain injury caused by trauma and not by disease. TBIs have many causes; according to specific embodiment, the TBI is caused by falls, vehicle collisions, sports collisions or combats. The phrase includes both mild and severe TBI including closed-head injuries, concussions or contusions and penetrating head injuries.

According to specific embodiments, the nerve injury comprises traumatic optic neuropathy (TON).

As used herein, the phrase "traumatic optic neuropathy (TON)" refers to injury to the optic nerve caused by trauma and not by disease. According to specific embodiments, TON results in vision loss, which may be partial or complete. TONs have many causes; according to specific embodiments, the TON is caused by an anatomical disruption of the optic nerve fibers from penetrating orbital trauma, bone fragments within the optic canal or nerve sheath hematomas.

The phrase "treating" refers to inhibiting or arresting the development of a pathology (disease, disorder or condition) and/or causing the reduction, remission, or regression of a pathology. Those of skill in the art will understand that various methodologies and assays can be used to assess the development of a pathology, and similarly, various methodologies and assays may be used to assess the reduction, remission or regression of a pathology. According to specific embodiments, treating comprises increasing survival.

Thus, according to another aspect of some embodiments of the invention, there is provided a method of increasing survival following nerve injury in a subject in need thereof, the method comprising implanting the composition, the matrix or the hydrogel at or near the nerve injury of the subject, thereby increasing survival of the subject.

According to another aspect of some embodiments of the invention, there is provided the composition, the matrix or the hydrogel for use in increasing survival following nerve injury in a subject in need thereof.

According to specific embodiments the increase is at least 1.5 fold, at least 2 fold, at least 3 fold, at least 5 fold, at least 10 fold, or at least 20 fold as compared to same in the absence of implanting the composition, the matrix or the hydrogel which may be obtained from databases and the known literature.

According to other specific embodiments the increase is by at least 5 %, by at least a 10 %, at least 20 %, at least 30 %, at least 40 %, at least 50 %, at least 60 %, at least 70 %, at least 80 %, at least 90 %, at least 95 % or at least 100 % as compared to same in the absence of implanting the composition, the matrix or the hydrogel which may be obtained from databases and the known literature.

According to specific embodiments, the mortality associated with the pathology (e.g. nerve injury) is due to neurogenic shock.

Hence, according to another aspect of some embodiments of the invention, there is provided a method of preventing or treating neurogenic shock following nerve injury in a subject in need thereof, the method comprising implanting the composition, the matrix or the hydrogel at or near the nerve injury of the subject, thereby preventing or treating the neurogenic shock in the subject.

According to another aspect of some embodiments of the invention, there is provided the composition, the matrix or the hydrogel for use in preventing or treating neurogenic shock in a subject in need thereof.

As used herein, the term "preventing" refers to keeping a disease, disorder or condition from occurring in a subject who may be at risk for the disease, but does not yet display symptoms of the disease disorder or condition or has not yet been diagnosed as having the disease, disorder or condition. Those of skill in the art will understand that various methodologies and assays can be used to assess the development of a pathology.

As used herein, the phrase "neurogenic shock" refers to a shock resulting in low blood pressure, occasionally with a slowed heart rate that is attributed to the disruption of the autonomic pathways that occur following damage to the CNS (e.g. SCI). Methods of diagnosing neurogenic shock and evaluating its progression are known in the art and include, but not limited to, radiographic imaging, hemodynamic monitoring and/or clinical exam.

According to specific embodiments, the methods or compositions of some embodiments of the present invention prevent and/or treat at least one of the symptoms of neurogenic shock including, but not limited to, instantaneous hypotension due to sudden, massive vasodilation, warm, flushed skin due to vasodilation and inability to vasoconstrict, priapism, also due to vasodilation; inability to get tachycardic, bradycardia, diaphragmatic breathing due to loss of nervous control of the intercostal muscles (typically due to an injury below the 5th cervical vertebra), respiratory arrest immediately following the injury due to loss of nervous control of the diaphragm (typically due to an injury above the 3rd cervical vertebra), organ dysfunction or death.

According to specific embodiments, the methods or compositions of some embodiments of the present invention treat or prevent organ dysfunction or death resulting from neurogenic shock.

According to specific embodiments, the methods or compositions of some embodiments of the present invention treat a subject demonstrating at least one symptom of neurogenic shock, such as, but not limited to, the symptoms described hereinabove.

According to specific embodiments, the methods or compositions of some embodiments of the present invention treat a subject demonstrating at least one symptom of neurogenic shock selected from the group consisting of hypotension, bradycardia and diaphragmatic breathing.

Those of skills in the art are capable of determining when and how to implant the composition, the matrix or the hydrogel to thereby induce e.g. tissue formation within the subject. See for example, Artzi Z, et al., 2005, J. Clin. Periodontol. 32: 193-9; Butler CE and Prieto VG, 2004, Plast. Reconstr. Surg. 114: 464-73.

According to specific embodiments, the composition, the matrix or the hydrogel is implanted locally at the site of the injury (e.g. nerve injury).

For example, for treating nerve injuries, the composition, the matrix or the hydrogel is implanted directly into the lesion (e.g. into the epicenter of the injury) ornear the lesion (e.g. at distance of approximately 0.5 cm from the injured site.).

According to specific embodiments, the composition, the matrix or the hydrogel is injected into an implantable tube (e.g. NeuraGen^{®} nerve guide obtained from Integra, NeuroTube^{®} from Synovis Micro, NeuroFlex^{™} and NeuroMatrix^{™} from Collagen Matrix, NeuroMend^{™} from Collagen Matrix, Neurolac^{®} from Polyganics BV, Groningen, Netherlands, SaluTunnel^{™} from Salumedica LLC, Avance^{®} from AxoGen, AxoGuard^{®} from AxoGen, Nerbridge from Toyobo, NeuraWrap^{™} from Integra LifeSciences Co.) which is implanted directly at or near the lesion. Following implantation the implants can be fixed by surgical adhesives (such as disclosed in e.g. Bhagat et al. Biomacromolecules 2017, 18, 3009-3039 e.g. gelatin-resorcinol-formaldehyde / glutaraldehyde glue (GRFG), cyanoacrylate glue, polysaccharide adhesive, polypeptide adhesive, polymeric adhesive, polyethylene glycol (PEG)-based hydrogel adhesive, biomimetic tissue adhesive, gecko-inspired tissue adhesive, sandcastle worm-inspired tissue adhesive, barnacle mimetic adhesive, caddisfly-inspired tissue adhesive, and fibrin glue e.g. TISSEEL, BioGlue, CryoLife, Beriplast P, Hemaseel, Quixil, Bolheal, biocol, VIGuard F.S.); and finally the muscular and cutaneous planes are closed and sutured.

Thus, according to specific embodiments, the composition, the matrix or the hydrogel is formulated for local administration.

According to specific embodiments, the composition, the matrix or the hydrogel is implanted within 12 hours, within 24 hours, within 48 hours, within 72 hours or within 96 hours following the nerve injury, each possibility represents a separate embodiment of the present invention.

According to specific embodiments, preventing or treating with the composition, the matrix or the hydrogel is within 12 hours, within 24 hours, within 48 hours, within 72 hours or within 96 hours following the nerve injury, each possibility represents a separate embodiment of the present invention.

According to specific embodiments, the composition, the matrix or the hydrogel is implanted within 48 hours following the nerve injury.

According to specific embodiments, preventing or treating with the composition, the matrix or the hydrogel is within 48 hours following the nerve injury.

According to specific embodiments, the composition, the matrix or the hydrogel is implanted within 24 hours following the nerve injury.

According to specific embodiments, preventing or treating with the composition, the matrix or the hydrogel is within 24 hours following the nerve injury.

The compositions, the matrix and/or the hydrogel of some embodiments of the invention may, if desired, be presented in a pack or dispenser device, such as an FDA approved kit, or an article of manufacture (with packaging material), which may contain one or more unit dosage forms containing the active ingredient. The pack may, for example, comprise metal or plastic foil, such as a blister pack. The pack or dispenser device may be accompanied by instructions for administration, implantation and/or treating a subject. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. The compositions, matrix or hydrogel of some embodiments of the invention formulated in a compatible pharmaceutical carrier may also be prepared, placed in an appropriate container, and labeled for treatment of an indicated condition, as is further detailed above.

As used herein the term "about" refers to ± 10 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non-limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

In the following examples, only the compositions comprising a hyaluronic acid, a laminin polypeptide, vitamin E, and Copolymer 1 (Copaxone) at a concentration range of 10 - 150 µg / ml are according to the invention. All other examples are reference examples.

### EXAMPLE 1

### THE IN-VITRO EFFECT OF GRG COMPRISING DIFFERENT ANTIOXIDANTS ON NEURITE SPROUTING AND OUTGROWTH

### MATERIALS AND METHODS

***Materials*** - All vendors and storage conditions are shown in Table 3 hereinbelow. ATCC Culture Medium: For first culture of PC12 cells, RPMI-1640 medium modified to contain 2 mM L-glutamine, 10 mM HEPES, 1 mM sodium pyruvate, 4500 mg / L glucose, and 1500 mg / 1 sodium bicarbonate was used. Prior to use, ATCC culture medium was warmed to 37 °C.

Complete Growth Media (CGM): To formulate CGM, RPMI medium was supplemented with 10 % heat inactivated horse serum, 5 % FBS, 2 mM L-Glutamine, 100 U / ml penicillin and 100 µg / ml streptomycin. CGM was prepared aseptically and stored at 2 - 8 °C for the duration of the study. Prior to use, CGM was warmed to 37 °C.

Incomplete Media (IM): Incomplete media contained RPMI medium with 1 % horse serum, 2 mM L-Glutamine, 100 U / ml penicillin and 100 µg / ml streptomycin.

Complete Neurobasal Media (CNM): To formulate CNM, Neurobasal medium was supplemented with 2 % B27, 2 % horse serum, 1 % Glutamax, 100 U / ml penicillin, 100 µg / ml streptomycin, 25 µl Beta-Meracaptoethanol (for each 50 ml), 10 % GDNF, 10 % BDNF, and 5 % CBTF.

Incomplete Neurobasal Media (INM): Incomplete nuerobasal media contained Neurobasal medium with 1 % Glutamax, 100 U / ml penicillin and 100 µg / ml streptomycin.

Nerve Growth Factor (NGF) 2.5 S: Nerve growth factor (NGF) 2.5 S was prepared aseptically as a stock solution of 10 µg / ml, diluted in Sterile water, and stored at -80 °C. 2.5 µl of NGF stock was added to each well for a final volume of 0.5 ml and a final concentration of 50 ng / ml.

0.2 % Hyaluronic Acid (HA): 0.2 % of HA in incomplete media was prepared aseptically and stored at 4 °C.

Laminin: A stock solution of Laminin at a concentration of 25 mg / ml was prepared aseptically in 100 % DMSO, filtered with 0.22 µm filter, and divided into aliquots and stored at -20 °C. For a final concentration of 50 µg / ml, 4 µl from the stock solution was added to 2 ml incomplete media. For a final concentration 20 or 10 µg / ml, the stock solution was diluted in DMSO to obtain a solution at a concentration of 10 mg / ml or 5 mg / ml. Following, 4 µl from each solution was added to 2 ml incomplete media.

Superoxide Dismutase (SOD1): 100 µg of SOD1 were dissolved in 1 ml incomplete media or 0.2 % HA to obtain a stock solution at a concentration of 100 µg / ml. Following, in order to have a final concentration of 20 µg / ml, the stock solution was diluted in incomplete media or 0.2 % HA.

GRG Formulation: GRG formulation was prepared as 0.2 % HA solution with the addition of Laminin at a final concentration of 10 µg / ml, and SOD1 at a final concentration of 20 µg / ml.

L-Seleyometionine: L-Selenometionine was dissolved in incomplete media to obtain a stock solution of 50 mM. In order to obtain a final concentration of 1 mM, 40 µl of L-Selenometionine was added to 2 ml incomplete media.

Trans Retinoic Acid: Trans Retinoic Acid was dissolved in DMSO to obtain a stock solution of 0.5 mM. In order to obtain a final concentration of 1 µM (0.2 % DMSO), 4 µl of Trans Retinoic Acid was added to 2 ml incomplete media.

Ascorbic Acid: Ascorbic Acid was dissolved in incomplete media to obtain stock solutions of 50 mM, 5 mM and 0.5 mM. For a final concentration of 1 mM, 0.1 mM or 0.01 mM, 40 µl of each stock solution was added to 2 ml incomplete media.

D-L Tocopherol: D-L Tocopherol was dissolved in DMSO to obtain stock solutions of 1.5 M, 0.5 M, 150 mM, 15 mM and 0.15 mM. For a final concentration of 3 mM, 1 mM, 0.3 mM, 0.03 mM or 0.000 3mM, 4 µl of each stock solution was added to 2 ml incomplete media.

Melatonin: Melatonin was dissolved in DMSO to obtain stock solutions of 15 µM, 1.5 µM, 0.5 nM, 150 nM and 1.5 nM. For a final concentration of 30 nM, 3 nM, 1 nM, 0.3 nM or 0.003 nM, 4 µl of each stock solution was added to 2 ml incomplete media.

Coenzyme Q10: Coenzyme Q10 was dissolved in DMSO to obtain stock solutions of 15 mM, 5 mM and 1.5 mM. For a final concentration of 30 µM, 10 µM or 3 µM, 4 µl of each stock solution was added to 2 ml incomplete media.

Acetyl-DL-Carnitine: Acetyl-DL-Carnitine was dissolved in incomplete media to obtain stock solutions of 15 mg / ml, 5 mg / ml and 1.5 mg / ml. For a final concentration of 300 µg / ml, 100 µg / ml or 30 µg / ml, 40 µl of each stock solution was added to 2 ml incomplete media.

N-Acetyl-D-Glucosamine: N-Acetyl-D-Glucosamine was dissolved in incomplete media to obtain stock solutions of 15 mg / ml, 5 mg / ml, 1.5 mg / ml, 0.15 mg / ml and 0.015 mg / ml. For a final concentration of 300 µg / ml, 100 µg / ml, 30 µg / ml, 3 µg / ml or 0.3 µg / ml, 40 µl of each stock solution was added to 2 ml incomplete media.

**Table 3: list of material s**

| **Materials** | **Name** | **Cat. No.** | **Manufacturer** | **Storage Conditions** |
|---|---|---|---|---|
| GRG Formulation | Hyaluronic acid (HA) 1 | 25735 | Lifecore | -20 °C |
| | Hyaluronic acid (HA) 2 1.9*10^6 Da | PHI | HTL | 4 °C |
| | Hyaluronic acid (HA) 3 4*10^6 Da | PHI | HTL | 4°C |
| | Hyaluronic acid (HA) 4 2.16*10^6 Da | 00-51-50 | Contipro | 4°C |
| | Hyaluronic acid (HA) 5 1 % | CE 0434 | Habier | 4 °C |
| | Hyaluronic acid (HA) 6 0.46* 10^6 Da | 00-51-50 | Contipro | 4 °C |
| | Laminin Peptide | 4107326 | Bachem | -20 °C |
| | Superoxide Dismutase (SOD1) | PRO286b | PROSPEC | -20 °C |
| Vehicle | PBS | 02-020-1A | Biological Industries | 4 °C |
| | DMSO | D5879 | Sigma | RT |
| Test Items | D-L Tocopherol | 613420 | Millipore | 4 °C |
| | Trans-Retinoic Acid | 554720 | Millipore | 4 °C |
| | L-Ascorbic Acid | 1831 | Millipore | RT |
| | L-Selenomethionine | 561505 | Millipore | RT |
| | Melatonin | M5250 | Sigma | -20 °C |
| | Acetyl-DL-Carnitine | A1509 | Sigma | -20 °C |
| | Coenzyme Q10 99 % | C9538 | Sigma | -20 °C |
| | N-Acetyl-D-glucosamine | 1079 | Millipore | 4 °C |
| Cell-Line | PC12 | CRL-1721 | ATCC | Liquid Nitrogen |
| DRG tissue | E18 rat dorsal root ganglion primary neuron tissue | SDEDRG | BrainBits | 2 - 8 °C |
| Differentiation Factor | Nerve Growth Factor (NGF) 2.5S | N-100 | Alomone Labs | -20 °C |
| Complete Growth Media for PC12 | RPMI 1640 | 30-2001 | ATCC | 4 °C |
| | L-Glutamine | 03-020-1B | Biological Industries | -20 °C |
| | Horse Serum | 04-124-1A | Biological Industries | -20 °C |
| | Fetal Bovine Serum (FBS) | 12657-029 | Gibco | -20 °C |
| | Pen-Strep | 03-031-1B | Biological Industries | -20 °C |
| Complete Neurobasal Media for mix motor neurons | Nuerobasal | 21103049 | Gibco | 4 °C |
| | B27 Supplement x50 | 17504044 | Gibco | -20 °C |
| | Glutamax | 35050061 | Gibco | RT |
| | Beta-Mercaptoethanol | 31350010 | Gibco | 4 °C |
| | GDNF | G-240 | Alomone Labs | -20 °C |
| | CNTF | C-240 | Alomone Labs | -20 °C |
| | BDNF | B-250 | Alomone Labs | -20°C |
| Digestion solution | Collagenase / Dispase | 102696380 01 | Roche | -20 °C |
| | Hibernate A without Calcium and Magnesium | HACAMG 5 | BrainBits | 2 - 8 °C |
| Culture surface | 96-well Flat-bottom tissue culture-treated plate (3596) | 29442-056 | VWR/Coming | RT |
| | Poly-D-lysine | P-6407 | Sigma | 2 - 8 °C (reconst.) |
| 24-well plates | Collagen I, Coated Plate 24-well | A11428-02 | Gibco | RT |

***PC12 cells based in-vitro assay for evaluation of Neurite sprouting and outgrowth* -** A schematic representation of the in-vitro assay is shown in Figure 1. PC12 cells (ATCC NO. CRL-1721) were cultured in RPMI 1640 medium supplemented with 10 % heat inactivated horse serum + 5 % heat inactivated FBS + 2 mM L-Glutamine + 100 U / ml penicillin + 100 µg / ml streptomycin (complete growth medium: CGM). On study day 0, 8x10³ cells were seeded in Complete Growth Media (CGM) in a collagen type I coated well and incubated for 24 hours. The following day (Study day 1), 90 % of the CGM was removed and the cells were stimulated with 50 ng / ml NGF 2.5 S in Incomplete Media (I M). Following additional 3 days of culture, 90 % of the I M was replaced with IM + different treatments, in duplicates. Following additional 3 days of culture, images from several fields of each well were taken for the analysis of the mean neurite length using Image J software.

***Embryonic spinal cord cells based in-vitro assay for evaluation of Neurite sprouting and outgrowth** -* A schematic representation of the in-vitro assay is shown in Figure 2. On study day 0, mix motor neuron cells were harvested from the spinal cord ventral horn of rat embryos E12.5. 5x10³ cells were seeded in Complete Neurobasal Media (CNM; rich medium) in each of a Poly DL-Ornithine and Laminin coated well and incubated for 24 hours. The following day (Study day 1), 90 % of the CNM was removed and replaced with Incomplete Neurobasal Media (INM; poor medium) + different treatments (n=5 wells for each group). 48 hours later (Study day 3), 9 images from each well were taken (total of 45 images / group) for the analysis of the mean neurite length using the IncuCyte S3 Live-Cell Analysis System.

***Dorsal Rout ganglion (DRG) neurons based in-vitro assay for evaluation of Neurite sprouting and outgrowth** -* A schematic representation of the in-vitro assay is shown in Figure 3. On study 0, primary DRG neuron cells (5x 10⁴ cells) were seeded in growth media. Different treatments were added to each group, in triplicates. 24 and 48 hours later, images from several fields of each well were taken for the analysis of the mean neurite length using Image J software.

***Statistical evaluation*** - Data is presented as mean ± SEM. Each treatment group is compared to a relevant group using applicable statistical test. A p < 0.05 is considered to represent a significant difference.

### RESULTS

*PC12 cell-based assay:* PC12 cells, clonal cells from a transplantable rat pheochromocytoma, which differentiate upon nerve growth factor (NGF) stimulation, were used in an in-vitro assay to evaluate the effect of Guiding Regenerative Gel (GRG) formulation comprising 3 components: Hyaluronic acid (HA), Laminin peptide and several antioxidants substances on neurite outgrowth.

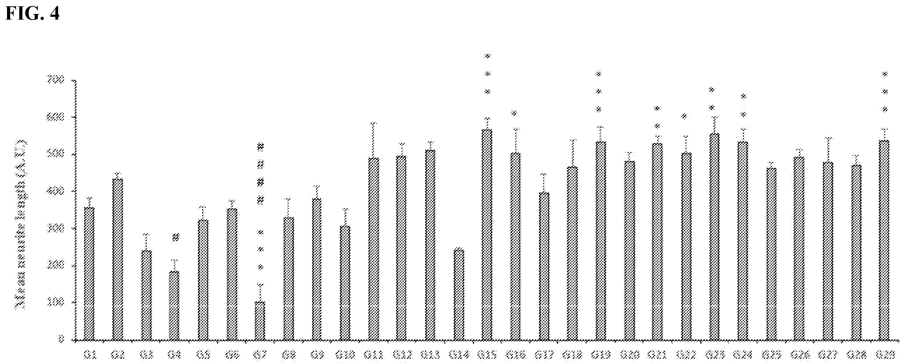

Treatment with GRG comprising N-Acetyl-D-glucosamine, Melatonin, Coenzyme Q, Acetyl-L-carnitine or D-L Tocopherol as an anti-oxidative agent increased neurite outgrowth compared to control NGF and DMSO treated cells, in a level comparable to a GRG comprising SOD 1 as an antioxidant (Table 4 hereinbelow and Figure 4). The effect on neurite outgrowth in this model was not dependent on the antioxidant dose tested (Table 4 hereinbelow and Figure 4).

In addition, GRG comprising a combination of two antioxidants had an additive effect on neurites growth in the PC12-based assay (data not shown).

**Table 4: Assessment of the effect of GRG comprising several different antioxidants on neurites growth in the PC 12-based assay**

| **Group #** | **Treatment** | **Mean neurite length (A.U.)** | **SEM** |
|---|---|---|---|
| G1 | NGF 50 ng / ml 3D + 0.2 % DMSO | 355.93 | 25.97 |
| G2 | HA 4 0.2 % + Laminin 10 µg / ml + SOD1 20 µg / ml | 431.99 | 17.42 |
| G8 | HA 4 0.2 % + Laminin 10 ug / ml + Acetyl-L-carnitine 30 ug / ml | 330.00 | 50.66 |
| G9 | HA 4 0.2 % + Laminin 10 ug / ml + Acetyl-L-carnitine 100 ug / ml | 379.14 | 34.44 |
| G10 | HA 4 0.2 % + Laminin 10 ug / ml + Acetyl-L-carnitine 300 ug / ml | 305.38 | 47.21 |
| G11 | HA 4 0.2 % + Laminin 10 ug / ml + Coenzyme-Q 0.3 uM | 489.33 | 94.83 |
| G12 | HA 4 0.2 % + Laminin 10 ug / ml + Coenzyme-Q 1 uM | 495.44 | 32.51 |
| G13 | HA 4 0.2 % + Laminin 10 ug / ml + Coenzyme-Q 3 uM | 509.14 | 26.37 |
| G14 | HA 4 0.2 % + Laminin 10 ug / ml + Coenzyme-Q 10 uM | 241.00 | 6.95 |
| G15 | HA 2 0.2 % + Laminin 10 ug / ml + N-acetyl glucosamine 0.03 ug / ml | 565.17 | 32.54 |
| G16 | HA 2 0.2 % + Laminin 10 ug / ml + N-acetyl glucosamine 3 ug / ml | 502.83 | 66.47 |
| G17 | HA 4 0.2 % + Laminin 10 ug / ml + N-acetyl glucosamine 30 ug / ml | 396.33 | 50.83 |
| G18 | HA 2 HA 4 0.2% + Laminin 10 ug/ml + N-acetyl glucosamine 100 ug/ml | 466.15 | 73.76 |
| G19 | HA 2 / HA 4 0.2% + Laminin 10 ug/ml + N-acetyl glucosamine 300 ug/ml | 533.86 | 40.43 |
| G20 | HA 2 0.2 % + Laminin 10 ug / ml + Melatonin 0.003 nM | 481.17 | 24.46 |
| G21 | HA 2 / HA 4 0.2 % + Laminin 10 ug / ml + Melatonin 0.3 nM | 528.07 | 23.41 |
| G22 | HA 2 / HA 4 0.2 % + Laminin 10 ug / ml + Melatonin 1 nM | 503.15 | 46.81 |
| G23 | HA 4 0.2 % + Laminin 10 ug / ml + Melatonin 3 nM | 554.50 | 45.29 |
| G24 | HA 2 0.2 % + Laminin 10 ug / ml + Melatonin 30 nM | 534.92 | 34.32 |
| G25 | HA 2 0.2 % + Laminin 10 ug / ml + Tocopherol 0.0003 mM | 461.75 | 16.31 |
| G26 | HA 2 0.2 % + Laminin 10 ug / ml + Tocopherol 0.03 mM | 490.58 | 22.25 |
| G27 | HA 4 0.2 % + Laminin 10 ug / ml + Tocopherol 0.3 mM | 477.67 | 67.00 |
| G28 | HA 2 / HA 4 0.2 % + Laminin 10 ug / ml + Tocopherol 1 mM | 471.38 | 25.19 |
| G29 | HA 2 / HA 4 0.2 % + Laminin 10 ug / ml + Tocopherol 3 mM | 536.68 | 32.41 |

*Embryonic Spinal Cord Cells-based assay:* Embryonic spinal cord cell, were used in an in-vitro assay to evaluate the effect of GRG formulation comprising HA, Laminin peptide and D-L Tocopherol on neurite outgrowth. Treatment with D-L Tocopherol at a dose of 3 mM resulted in normalized neurite outgrowth similar to GRG comprising HA, Laminin peptide and SOD1. Lower doses of D-L Tocopherol were less active than GRG comprising SOD1 as the antioxidant.

**Table 5: Assessment of the effect of GRG comprising Tocopherol compared to GRG comprising SOD1 on neurites growth in the PC12-based assay**

| **Group #** | **Treatment** | **Neurite Length per Cell-Body Cluster Area (mm / mm²)** | **SEM** |
|---|---|---|---|
| G1 | HA 2; 0.2 % + Laminin 10 ug/ml + SOD1 20 ug/ml | 849.73 | 42.48 |
| G2 | HA 2; 0.2 % + Laminin 10 ug/ml + SOD1 20 ug/ml + 0.2 % DMSO | 729.06 | 57.92 |
| G3 | HA 2; 0.2 % + Laminin 10 ug/ml + SOD1 20 ug/ml + 0.4 % DMSO | 641.62 | 86.29 |
| G4 | HA 2; 0.2 % + Laminin 10 ug/ml + Tocopherol 3 mM | 664.92 | 14.17 |
| G5 | HA 2; 0.2 % + Laminin 10 ug/ml + Tocopherol 0.03 mM | 250.72 | 29.54 |
| G6 | HA 2; 0.2 % + Laminin 10 ug/ml + Tocopherol 0.0003 mM | 432.21 | 46.25 |

*Primary DRG Neurons-based assay:* Incubation of DRG neurons with GRG comprising Melatonin as an antioxidant or GRG comprising D-L Tocopherol as an antioxidant increased neurites outgrowth compared to HA + Laminin treated cells in a level comparable to and even higher than GRG comprising SOD 1 as an antioxidant (Table 6 hereinbelow).

**Table 6: Assessment of the effect of GRG comprising several different antioxidants on neurites growth in the DRG neurons-based assay**

| **Group #** | **Treatment** | **Mean neurite length (A.U.)** | **SEM** |
|---|---|---|---|
| G1 | HA 2 0.2 % + Laminin 10 ug/ml | 27.67 | 1.76 |
| G2 | HA 2 0.2 % + Laminin 10 ug/ml + Tocopherol 3 mM | 85.33*** | 18.89 |
| G3 | HA 2 0.2 % + Laminin 10 ug/ml + SOD1 20 ug / ml | 31.67** | 6.57 |
| G4 | HA 2 0.2 % + Laminin 10 ug/ml + Melatonin 3 nM | 64.33** | 15.72 |

| | | | |
|---|---|---|---|
| ** p < 0.01 vs. G1 and *** p < 0.001 vs. G1 using One-way ANOVA followed by Tukey post-hoc test. | | | |

### EXAMPLE 2

### THE IN-VIVO EFFECT OF GRG COMPRISING TOCOPHEROL IN A RAT PERIPHERAL NERVE INJURY MODEL

### MATERIALS AND METHODS

***Materials*** - All vendors and storage conditions are shown in Table 7 hereinbelow.

**Table 7: list of materials**

| **Materials** | **Name** | **Cat. No.** | **Manufacturer** | **Supplier** | **Storage Conditions** |
|---|---|---|---|---|---|
| GRG composition | Tocopherol | 613420 | Millipore | Millipore | 4 °C |
| | Laminin peptide | 4107326 | Bachem | Bachem | -20 °C |
| | HA0.4 % | 025735 | Lifecore | Lifecore Biomedical | -20 °C |
| | DMSO | D5879 | Sigma | Sigma | RT |
| Antibiotic | Baytril | N / A | BAYER | Lidor chemicals | RT |
| | Synthomycine (Chloramphenicol 5 %) | N / A | Rekah, Israel | Vetmarket | RT |
| Antiseptic | Polydine | N / A | FLORIS ISRAEL | Vetmarket | RT |
| Anesthesia Items | Ketamine | N / A | Vetqvinol | Vetmarket | RT |
| | Xylazine | N / A | SURUPET | Vetmarket | RT |
| | Isoflurane | N / A | Vetmarket | Vetmarket | RT |
| Analgesia Items | Buprenorphine | N / A | Vetmarket | Vetmarket | RT |
| | Dipyron | N / A | Ratiopharm GmbH, Germany | Vetmarket | RT |
| | Amitriptyline | N / A | FAGRON | Tamar market | RT |
| | Marcaine 0.5 % | N / A | Astrazenca | Vetmarket | RT |
| | Voltaren cream | N / A | Novartis. Switzerland | Vetmarket | RT |
| Euthanasia Item | Pentobarbital Sodiun | N / A | CST | Vetmarket | RT |
| Histology Item | Formaldehyde 4 % | 6450365F1 | BioLab | BioLab | RT |

***Study procedures** -* A schematic representation of the study procedures and schedules is presented in Figure 5. Male Sprague Dawley rats were anesthetized using intraperitoneal (IP) injection of ketamine (35 mg / kg) and xylazine (8 mg / Kg). Following, the animals were placed on the surgery table and the area of the surgery was shaved, washed with ethanol and polydine solution and covered with a sterile sheet to ensure sterile conditions. All surgical procedures were performed on anesthetized rats, using a high magnification neurosurgical microscope. The rats were operated on the left hind leg, in which an incision of about 4 cm in length was made along the fusion line of the muscles. The fascia was sharply divided and the muscles were bluntly retracted to enable access to the sciatic nerve. Following, the sciatic nerve was exposed and separated from the proximal nerve, and transected proximally and distally removing 10 mm of length using a microsurgical razor. The experimental groups are shown in Table 8 hereinbelow.

Autologous nerve graft (ANG) reconstructive procedure (Group 1): The removed 10 mm nerve segment (autologous nerve graft - Gold Standard) was inverted and an end-to-end anastomosis was performed between the peripheral nerve segment and the proximal and distal parts of the left sciatic nerve, using 10-0 sutures. Cooptation of the nerve was carried out in order to preserve all of the fascicles within the epineural sac.

NeuraGen^{®} nerve guide (Reference empty tube treatment) reconstructive procedure (Group 2): The rats underwent nerve reconstruction with NeuraGen^{®} nerve guide in which the proximal and distal ends of the nerve were fixed into the 15 mm NeuraGen^{®} nerve guide (preimmersed in saline), creating a 10 mm gap between the two ends, and was microsurgically reconnected using 10-0 epineural sutures.

GRG treatment (Groups 3-6): Following removal of the 10 mm nerve segment, the proximal and distal ends of the nerve were fixed into the 15 mm NeuraGen^{®} nerve guide (preimmersed in saline) filled with GRG perior to immplantation (the GRG was contained in a syringe), creating a 10 mm gap between the two ends, and was microsurgically reconnected using 10-0 epineural sutures.

The muscles were sutured using 3-0 vicryl threads. The skin was closed using special metal staples. The rats were followed-up for a period of 5 months.

**Table 8: Rat peripheral nerve injury experimental groups**

| **Group #** | **# of animal operated** | **Number of animals at the end of the study** | **Treatment Groups** | **Tocopherol concentration** | **Volume of Injection** |
|---|---|---|---|---|---|
| 1 | 12 | 10 | ANG (Autologous nerve graft) | N / A | N / A |
| 2 | 12 | 11 | NeuraGen^{®} Nerve Guide (Integra Life Sciences) (empty tube) | N / A | N / A |
| 3 | 12 | 11 | GRG + Tocopherol in NeuraGen^{®} Nerve Guide (Integra Life Sciences) | 0.3 mM | 0.2 ml |
| 4 | 12 | 10 | GRG + Tocopherol in NeuraGen^{®} Nerve Guide (Integra Life Sciences) | 1 mM | 0.2 ml |
| 5 | 12 | 10 | GRG + Tocopherol in NeuraGen^{®} Nerve Guide (Integra Life Sciences) | 3 mM | 0.2 ml |
| 6 | 12 | 8 | GRG + Tocopherol in NeuraGen^{®} Nerve Guide (Integra Life Sciences) | 10 mM | 0.2 ml |

***Clinical signs and autotomy -*** The clinical score composed 2 categories: Redness (0-normal; 1- slightly redness at the paw; 2- spread redness) and Swelling (0- no swelling; 1-slightly swelling; 2- pronounced swelling) 3- autotomy. The final score of each rat was calculated as the SUM of the scores on each category.

***Body Weight Measurements*** - Animal body weight was measured on study day 0 prior to surgery and thereafter once weekly for the entire study period.

***Compound Muscle Action Potentials (CHAPs)*** - Compound Muscle Action Potentials (CMAPs) were recorded prior to and following the surgical procedure in both hind legs, using a Dantec^{™} KEYPONT^{®} PORTABLE. During the measurements the rats were anesthetized using IIsoflurane Inhalation. CMAPs were recorded by placing the stimulating needle electrodes at the sciatic notch and the recording needles at the tibialis anterior muscle. The ground electrode was placed on the thigh of the side of stimulation. The sciatic nerve was stimulated by a bipolar stimulating electrode with a pulse of 0.1 m / sec in duration. The stimulus intensity was increased gradually, until a maximal amplitude CMAP was obtained. Latency and amplitude of the CMAPs were measured.

***Gait recording and analysis (Catwalk assay)* -** Gait walk analysis was performed using CatWalkXT (Noldus Information Technology, The Netherlands) system. The system includes a 1.0 m enclosed walkway on which the rats traverse from side to side and recordings were made.

***Histological evaluation** -* Animals were sacrificed at week 19 post operation. The sciatic nerve was removed and immersed in formaldehyde (FA) 4 %. Prior to processing, each sample was cut at 3 locations: proximal, middle (tube region) and distal. Each section was embedded in paraffin and a 5 µm section was performed and stained with Hematoxylin and Eosin (H&E). The number of axons was counted in each section. A neuron was concedered if is showed a round shape with a blue / purple dot in the center.

***Statistical evaluation** -* Data is presented as mean ± SEM. Each treatment group was compared to its relative Vehicle group using Student's T-test or F-test (GraphPad). A p < 0.05 is considered to represent a significant difference.

***Animal care and use statement** -* The study was performed following approval of an application form submitted to the Committee for Ethical Conduct in the Care and Use of Laboratory Animals that stated that the study complied with the rules and regulations set forth.

### RESULTS

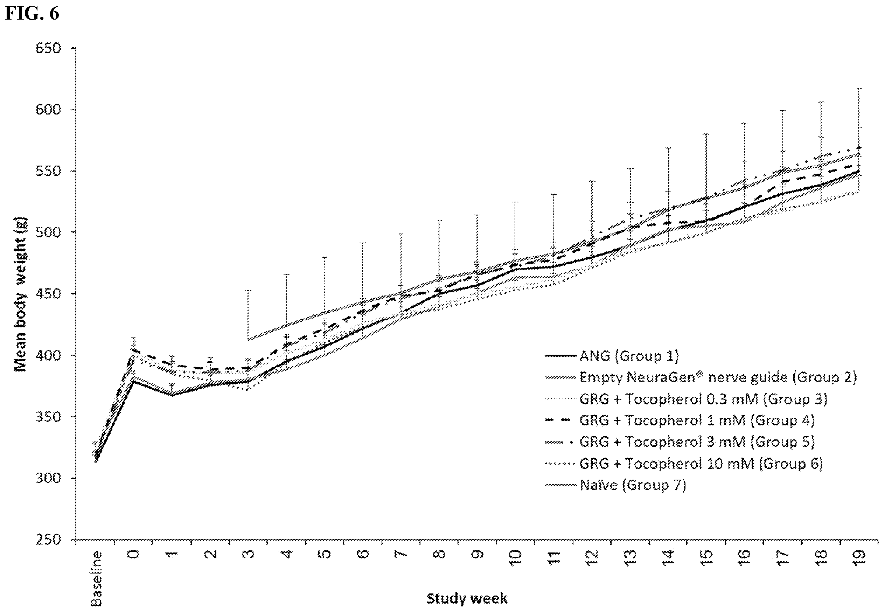

*Body Weight Measurements:* Animals were measured once weekly during the entire study period. Week 0 is the operation week. During the first 2 weeks post operation the animals slightly lost weight, a phenomenon that is expected following such a procedure. During the following weeks, the animals recovered and gained weight without any difference between the treated groups (Table 9 hereinbelow and Figure 6). On the third week naïve animals were added which served as a healthy animal control demonstrating that the weight gain in all treatment groups was similar to healthy animals from the same age strain and sex (Table 9 hereinbelow and Figure 6). Weight gain suggests that the overall health of the animals was good.

**Table 9: Mean body weight(s)**

| **Treatment Group #** | **Study Weeks** | | | | | | | | | | | |
|---|---|---|---|---|---|---|---|---|---|---|---|---|
| | **MEAN** | | | | | | **SEM** | | | | | |
| | **Baseline** | **0** | **1** | **2** | **3** | **4** | **Baseline** | **0** | **1** | **2** | **3** | **4** |
| ANG (Group 1) | 313.67 | 378.42 | 367.33 | 375.75 | 378.42 | 394.83 | 5.09 | 7.07 | 8.28 | 8.02 | 7.78 | 9.07 |
| Empty NeuraGen^{®} nerve guide (Group 2) | 322.67 | 382.67 | 369.00 | 377.67 | 380.58 | 388.17 | 5.67 | 4.76 | 7.79 | 8.63 | 8.27 | 8.54 |
| GRG + Tocopherol 0.3 mM (Group 3) | 323.75 | 403.08 | 386.83 | 386.00 | 385.83 | 401.33 | 6.10 | 11.95 | 12.76 | 12.10 | 11.50 | 12.83 |
| GRG + Tocopherol 1 mM (Group 4) | 317.50 | 404.25 | 392.25 | 388.50 | 389.75 | 408.92 | 4.83 | 6.96 | 6.52 | 6.20 | 6.69 | 8.27 |
| GRG + Tocopherol 3 mM (Group 5) | 317.17 | 396.08 | 386.58 | 386.08 | 387.67 | 407.75 | 4.09 | 9.09 | 7.95 | 8.19 | 7.83 | 7.70 |
| GRG + Tocopherol 10 mM (Group 6) | 314.83 | 399.67 | 384.42 | 379.42 | 371.58 | 393.83 | 4.96 | 8.64 | 8.73 | 8.43 | 8.98 | 7.70 |
| Naive (Group 7) | 319.00 | N/A | N / A | N / A | 412.67 | 424.33 | 8.33 | N / A | N / A | N / A | 40.19 | 41.33 |

| **Treatment Group #** | **Study Weeks** | | | | | | | | | | | |
|---|---|---|---|---|---|---|---|---|---|---|---|---|
| | **MEAN** | | | | | | **SEM** | | | | | |
| | **5** | **6** | **7** | **8** | **9** | **10** | **5** | **6** | **7** | **8** | **9** | **10** |
| ANG (Group 1) | 406.92 | 422.00 | 434.83 | 450.10 | 456.50 | 470.00 | 9.13 | 10.95 | 11.56 | 14.92 | 15.75 | 15.79 |
| Empty NeuraGen^{®} nerve guide (Group 2) | 400.17 | 414.09 | 429.45 | 439.82 | 450.55 | 463.18 | 8.36 | 9.26 | 10.25 | 9.72 | 10.77 | 11.54 |
| GRG + Tocopherol 0.3 mM (Group 3) | 412.67 | 426.27 | 434.45 | 441.09 | 449.73 | 455.36 | 13.00 | 14.36 | 14.37 | 13.39 | 13.87 | 13.59 |
| GRG + Tocopherol 1 mM (Group 4) | 421.42 | 436.50 | 448.08 | 452.67 | 465.67 | 473.50 | 8.57 | 9.40 | 8.50 | 7.59 | 8.16 | 9.23 |
| GRG + Tocopherol 3 mM (Group 5) | 418.50 | 434.50 | 446.42 | 453.67 | 465.92 | 472.25 | 8.68 | 9.00 | 10.04 | 9.86 | 9.73 | 9.74 |
| GRG + Tocopherol 10 mM (Group 6) | 410.25 | 423.36 | 433.36 | 437.45 | 445.55 | 452.82 | 8.34 | 9.67 | 10.13 | 9.93 | 8.76 | 8.43 |
| Naive (Group 7) | 434.67 | 443.00 | 450.67 | 462.00 | 468.00 | 477.00 | 44.67 | 48.51 | 47.70 | 47.23 | 46.18 | 47.65 |

| **Treatment Group #** | **Study Weeks** | | | | | | | | | | | |
|---|---|---|---|---|---|---|---|---|---|---|---|---|
| | **MEAN** | | | | | | **SEM** | | | | | |
| | **11** | **12** | **13** | **I 14** | **15** | **16** | **11** | **12** | **13** | **14** | **15** | **16** |
| ANG (Group 1) | 472.10 | 479.50 | 489.50 | 501.60 | 509.50 | 520.90 | 15.53 | 15.19 | 15.44 | 16.49 | 17.75 | 18.22 |
| Empty NeuraGen^{®} nerve guide (Group 2) | 463.82 | 472.91 | 489.73 | 502.45 | 505.36 | 508.00 | 11.98 | 11.82 | 11.07 | 11.32 | 12.45 | 13.15 |
| GRG + Tocopherol 0.3 mM (Group 3) | 461.73 | 473.55 | 485.73 | 491.27 | 500.27 | 511.18 | 13.90 | 15.29 | 16.28 | 15.86 | 17.75 | 19.46 |
| GRG + Tocopherol 1 mM (Group 4) | 477.33 | 490.83 | 504.08 | 507.50 | 508.67 | 520.92 | 9.60 | 10.69 | 10.75 | 10.93 | 14.91 | 16.52 |
| GRG + Tocopherol 3 mM (Group 5) | 480.75 | 496.50 | 511.42 | 519.50 | 528.17 | 542.42 | 10.77 | 12.20 | 13.10 | 13.75 | 14.32 | 15.56 |
| GRG + Tocopherol 10 mM (Group 6) | 457.36 | 471.30 | 483.90 | 491.67 | 499.11 | 512.00 | 8.79 | 9.94 | 10.43 | 10.63 | 10.69 | 11.66 |
| Naive (Group 7) | 482.67 | 492.33 | 503.67 | 518.67 | 528.00 | 536.33 | 48.27 | 49.14 | 48.35 | 49.97 | 51.94 | 51.94 |

| **Treatment Group #** | **Study weeks** | | | | | | | | | | | |
|---|---|---|---|---|---|---|---|---|---|---|---|---|
| | **MEAN** | | | **SEM** | | | | | | | | |
| | **17** | **18** | **19** | **17** | **18** | **19** | | | | | | |
| ANG (Group 1) | 531.50 | 538.80 | 549.80 | 183.91 | 18.84 | 19.68 | | | | | | |
| Empty NeuraGen^{®} nerve guide (Group 2) | 524.36 | 536.09 | 547.18 | 15.13 | 15.18 | 14.79 | | | | | | |
| GRG + Tocopherol 0.3 mM (Group 3) | 516.73 | 525.82 | 534.45 | 20.21 | 20.12 | 20.38 | | | | | | |
| GRG + Tocopherol 1 mM (Group 4) | 541.64 | 547.55 | 555.82 | 11.71 | 12.04 | 12.68 | | | | | | |
| GRG + Tocopherol 3 mM (Group 5) | 550.58 | 561.92 | 569.08 | 15.58 | 15.88 | 16.71 | | | | | | |
| GRG + Tocopherol 10 mM (Group 6) | 518.78 | 524.50 | 533.13 | 11.72 | 13.02 | 13.39 | | | | | | |
| Naive (Group 7) | 548.67 | 554.33 | 563.67 | 50.60 | 51.96 | 53.80 | | | | | | |

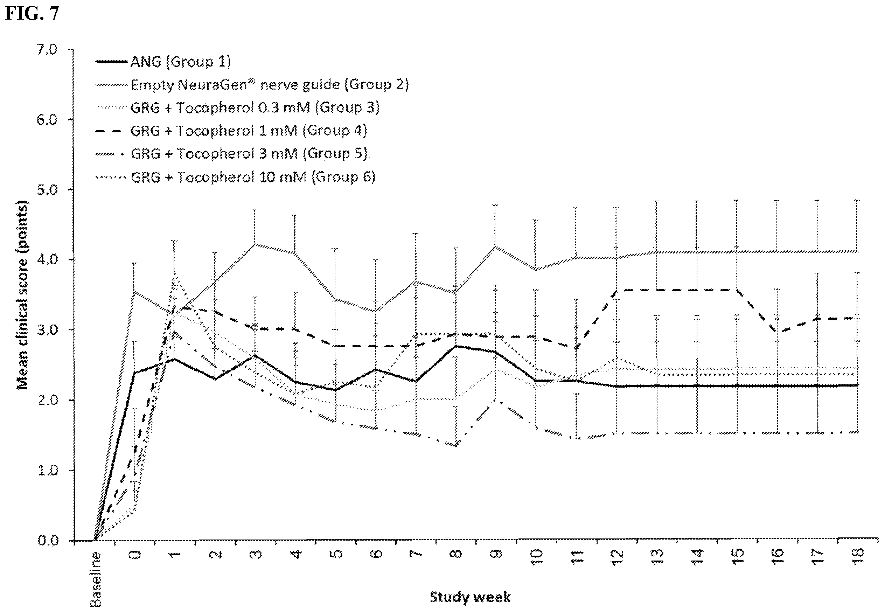

*Clinical signs and autotomy:* Following the surgery, a number of phenomenon were observed as expected: autotomy, swelling of the foot and redness. The empty NeuraGen^{®} nerve guide (i.e. empty tube) treated group exhibited the highest clinical score, with a maximum score of 4.21 ± 0.50 points out of 8 possible points (Table 10 hereinbelow and Figure 7). The remaining groups showed reduced clinical score, starting on week 4 and onwards. The most significant reduction in the clinical scores was observed in the animals treated with GRG comprising Tocopherol at a concentration of 3 mM (Group 5): 1.50 ± 0.69 points vs. 4.08 ± 0.73 points in the empty NeuraGen^{®} nerve guide (Group 2) (Table 10 hereinbelow and Figure 7).

Autotomy is expected in models of peripheral nerve transection. This phenomenon was already described in the 70's of the 20^{th} century when Wall et al., reported that injury to the sciatic nerve is characterised by autotomy, where they showed that the most serious autotomy was produced by the section of the nerve. Other manipulations, such as nerve ligation or crush also caused autotomy, but to a lesser degree (Wall PD, et al. Pain. 1979; 7(2):103-11). The empty NeuraGen^{®} nerve guide treated group experienced the highest percentage of autotomy one month following surgery (42 °%). The group that experienced the lowest level of autotomy was the group of animals that was treated with GRG comprising 3 mM Tocopherol (group 5, 8 %, Table 10 hereinbelow). In comparison, seventeen percent of the autologous operated animals showed autotomy. In this study the level of autotomy in the control (Empty NeuraGen^{®} nerve guide) was relatively low in comparison to the reported autotomy in the literature. In male Sprague-Dawley rats the level of autotomy can be as high as 65 % (Wall PD, et al. Pain. 1979; 7(2):103-11). The time for autotomy initiation is in line with other published studies.

**Table 10: Mean Group clinical signs (points)**

| **Study Weeks** | **Study Groups** | | | | | |
|---|---|---|---|---|---|---|
| | **ANG (Group 1)** | **Empty NeuraGen^{®} nerve guide (Group 2)** | **GRG + Tocopherol 0.3 mM (Group 3)** | **GRG + Tocopherol 1 mM (Group 4)** | **GRG + Tocopherol 3 mM (Group 5)** | **GRG + Tocopherol 10 mM (Group 6)** |
| | **MEAN** | | | | | |
| **0** | 0.00 | 0.00 | 0.00 | 0.00 | 0.00 | 0.00 |
| **1** | 2.38 | 3.54 | 0.50 | 1.25 | 0.92 | 0.42 |
| **2** | 2.58 | 3.21 | 3.25 | 3.33 | 2.96 | 3.79 |
| **3** | 2.29* | 3.67 | 2.96 | 3.25 | 2.46 | 2.75 |
| **4** | 2.63* | 4.21 | 2.58 | 3.00 | 2.17 | 2.38 |
| **5** | 2.25* | 4.08 | 2.08 | 3.00 | 1.92 | 2.08 |
| **6** | 2.13 | 3.42 | 1.92 | 2.75 | 1.67 | 2.25 |
| **7** | 2.42 | 3.25 | 1.83 | 2.75 | 1.58 | 2.17 |
| **8** | 2.25 | 3.67 | 2.00 | 2.75 | 1.50 | 2.92 |
| **9** | 2.75 | 3.50 | 2.00 | 2.92 | 1.33 | 2.92 |
| **10** | 2.67 | 4.17 | 2.42 | 2.88 | 2.00 | 2.92 |
| **11** | 2.25 | 3.83 | 2.17 | 2.88 | 1.58 | 2.42 |
| **12** | 2.25 | 4.00 | 2.33 | 2.71 | 1.42 | 2.25 |
| **13** | 2.17 | 4.00 | 2.42 | 3.54 | 1.50 | 2.58 |
| **14** | 2.17 | 4.08 | 2.42 | 3.54 | 1.50* | 2.33 |
| **15** | 2.17 | 4.08 | 2.42 | 3.54 | 1.50* | 2.33 |
| **16** | 2.17 | 4.08 | 2.42 | 3.54 | 1.50* | 2.33 |
| **17** | 2.17* | 4.08 | 2.42** | 2.92 | 1.50** | 2.33 |
| **18** | 2.17* | 4.08 | 2.42** | 3.13 | 1.50** | 2.33 |
| **19** | 2.17* | 4.08 | 2.42** | 3.13 | 1.50** | 2.33 |

| | **SEM** | | | | | |
|---|---|---|---|---|---|---|
| **0** | 0.00 | 0.00 | 0.00 | 0.00 | 0.00 | 0.00 |
| **1** | 0.45 | 0.40 | 0.34 | 0.62 | 0.42 | 0.29 |
| **2** | 0.31 | 0.41 | 0.32 | 0.37 | 0.48 | 0.47 |
| **3** | 0.43 | 0.42 | 0.46 | 0.45 | 0.48 | 0.51 |
| **4** | 0.44 | 0.50 | 0.47 | 0.46 | 0.52 | 0.61 |
| **5** | 0.44 | 0.54 | 0.53 | 0.52 | 0.56 | 0.72 |
| **6** | 0.62 | 0.72 | 0.57 | 0.64 | 0.54 | 0.74 |
| **7** | 0.65 | 0.73 | 0.59 | 0.64 | 0.54 | 0.73 |
| **8** | 0.63 | 0.68 | 0.60 | 0.68 | 0.54 | 0.69 |
| **9** | 0.62 | 0.65 | 0.60 | 0.62 | 0.56 | 0.68 |
| **10** | 0.56 | 0.58 | 0.68 | 0.66 | 0.58 | 0.69 |
| **11** | 0.60 | 0.71 | 0.66 | 0.66 | 0.58 | 0.75 |
| **12** | 0.60 | 0.72 | 0.68 | 0.70 | 0.65 | 0.78 |
| **13** | 0.63 | 0.72 | 0.70 | 0.61 | 0.69 | 0.82 |
| **14** | 0.63 | 0.73 | 0.70 | 0.61 | 0.69 | 0.85 |
| **15** | 0.63 | 0.73 | 0.70 | 0.61 | 0.69 | 0.85 |
| **16** | 0.63 | 0.73 | 0.70 | 0.61 | 0.69 | 0.85 |
| **17** | 0.63 | 0.73 | 0.70 | 0.62 | 0.69 | 0.85 |
| **18** | 0.63 | 0.73 | 0.70 | 0.64 | 0.69 | 0.85 |
| **19** | 0.63 | 0.73 | 0.70 | 0.64 | 0.69 | 0.85 |

| | | | | | | |
|---|---|---|---|---|---|---|
| * p < 0.05 vs. Empty NeuraGen^{®} nerve guide using T-test ** p < 0.01 vs. Empty NeuraGen^{®} nerve guide using T-test | | | | | | |

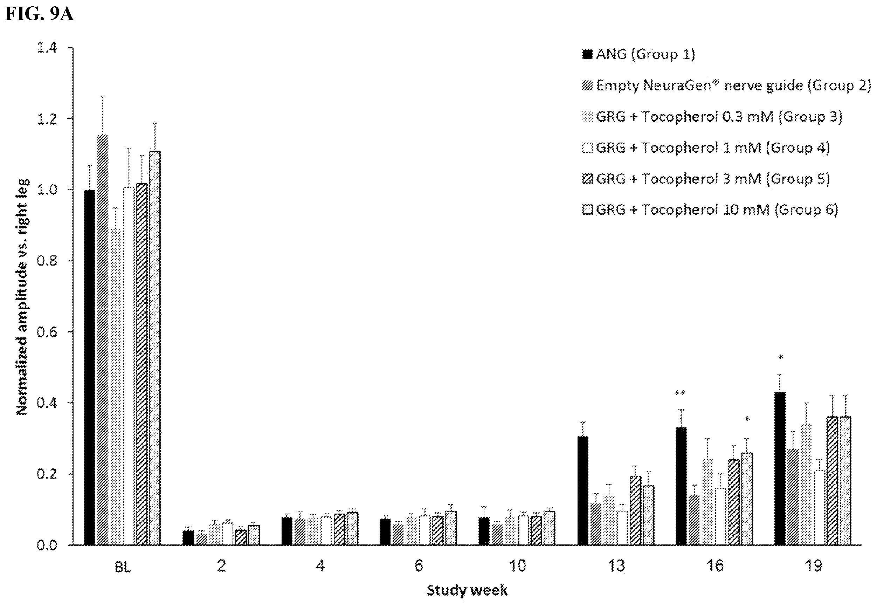

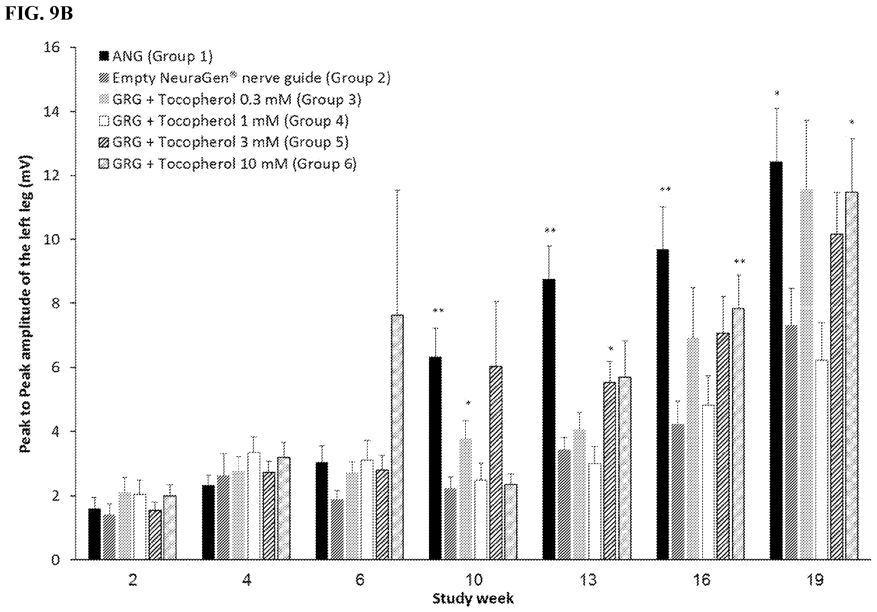

*Electrophysiology [Compound Muscle Action Potentials (CMAPs)]:* CMAPs consist of stimulating a nerve fiber followed by monitoring the response in the muscle. Injury to the nerve causes interruption of the electrical signal, which is recorded in the muscle. There are 3 major parameters in monitoring CMAPs: existence of electrical signaling; action potential amplitude and action potential duration which is referred to as "Peak to Peak (PtoP)"; i.e., the duration from the peak of stimulation to the peak of the action potential (Figures and 9A-B, Tables 11-14 hereinbelow). Following transection of the sciatic nerve the signal could be detected in most tested cases but the amplitude and the PtoP values were reduced (Rupp A, et al. Journal of Neuroscience Methods, 2007, 166: 266-277). The most accurate method of presenting the data obtained by monitoring the amplitude and the PtoP values is by normalizing the values recorded from the injured leg vs. the values recorded from the healthy (intact) leg. When applying this method of normalization, it can be seen (Figures 9A-B) that the action potential amplitude was significantly reduced following the surgery: Empty NeuraGen^{®} nerve guide (group 2) 0.03 ± 0.01 vs. 1.15 ± 0.11 prior to the operation (p < 0.001). PtoP values decreased from 43.91 ± 2.72 milliseconds prior to surgery to 1.40 ± 0.35 milliseconds (ms) (Empty NeuraGen^{®} nerve guide, Group 2. p < 0.001) post-surgery. Both the normalized amplitude and the PtoP values increased for the injured left leg during the study period. Towards the end of the study on weeks 16 and 19, the normalized amplitude was 0.33 ± 0.05 and 0.43 ± 0.05, respectively, for the autologous nerve graft group (1); significantly higher than the normalized amplitude of the empty NeuraGen^{®} nerve guide group (0.14 ± 0.03 and 0.27 ± 0.05; p < 0.01 and p < 0.05, respectively). Treatment with GRG comprising 3 mM Tocopherol resulted in an improvement trend of activity starting on week 13 (0.19 ± 0.03 vs. 0.12 ± 0.03). On week 16, a trend of improved activity was also detected following treatment with GRG comprising 10 mM Tocopherol (0.26 ± 0.04, p < 0.05 vs. empty NeuraGen^{®} nerve guide using student's T-test).

Comparing the PtoP signal duration of the ANG group with the empty NeuraGen^{®} nerve guide group suggests that starting from week 10 and throughout the study, a statistically significant improvement was recorded (week 10, 6.33 ± 0.90 ms vs. 2.23 ± 0.37 ms; p < 0.01). Treatment with GRG + Tocopherol at a concentration of 0.3mM resulted in trend of improvement on week 10, compared to the empty NeuraGen^{®} nerve guide group, which was found to be statistically significant (3.77 ± 0.57 ms vs. 2.23 ± 0.37; p < 0.05). This trend remained throughout the study, but without statistical significance. Following treatment with GRG + Tocopherol at a concentration of 3mM, a significant increase in the PtoP values was recorded on week 13 (5.54 ± 0.65ms, p < 0.05 vs. empty NeuraGen^{®} nerve guide group). Treatment with the higher dose of Tocopherol (10 mM) resulted in a significant improvement in the PtoP signal duration on weeks 16 and 19 (Week 19: 11.48 ± 1.67 ms vs. 7.32 ± 1.14 ms of the empty NeuraGen^{®} nerve guide group. At the end of the study (week 19), due to improvement of the CMAPs recorded from the empty NeuraGen^{®} nerve guide rats, the significance of the improved signal of either the ANG or the Tocopherol treated groups was reduced.

**Table 11: Amplitude (mV) of the Left leg (operated) and Right leg (intact).**

| **Treatment Group #** | **Study Weeks** | | | | | | | | | | | | | |
|---|---|---|---|---|---|---|---|---|---|---|---|---|---|---|
| | **-1** | | **2** | | **4** | | **6** | | | | **10** | | | |
| | **Right Leg** | **Left Leg** | **Right Leg** | **Left Leg** | **Right Leg** | **Left Leg** | **Right Leg** | | **Left Leg** | | **Right Leg** | | **Left Leg** | |
| | **MEAN** | | | | | | | | | | | | | |
| ANG (Group 1) | 22.96 | 22.24 | 29.11 | 1.06 | 21.88 | 1.68 | 22.15 | | 1.57 | | 24.45 | | 4.37 | |
| Empty NeuraGen^{®} nerve guide (Group 2) | 22.29 | 14.17 | 28.84 | 0.87 | 24.08 | 1.58 | 24.46 | | 1.37 | | 23.42 | | 1.40 | |
| GRG + Tocopherol 0.3 mM (Group 3) | 25.93 | 22.76 | 25.48 | 1.50 | 25.11 | 1.90 | 22.74 | | 1.78 | | 24.04 | | 2.21 | |
| GRG + Tocopherol 1 mM (Group 4) | 27.03 | 26.26 | 25.13 | 1.44 | 25.05 | 1.98 | 23.44 | | 1.75 | | 22.87 | | 1.63 | |
| GRG + Tocopherol 3 mM (Group 5) | 24.53 | 24.22 | 29.87 | 1.16 | 23.78 | 2.06 | 24.62 | | 1.91 | | 24.12 | | 1.70 | |
| GRG + Tocopherol 10 mM (Group 6) | 21.63 | 22.97 | 26.99 | 1.45 | 27.08 | 2.25 | 24.95 | | 2.31 | | 22.54 | | 1.62 | |

| **Treatment Group #** | **SEM** | | | | | | | | | | | | | |
|---|---|---|---|---|---|---|---|---|---|---|---|---|---|---|
| ANG (Group 1) | 1.37 | 1.37 | 1.65 | 0.25 | 0.99 | 0.26 | 0.95 | | 0.27 | | 1.17 | | 0.76 | |
| Empty NeuraGen^{®} nerve guide (Group 2) | 1.61 | 1.84 | 1.01 | 0.21 | 1.51 | 0.37 | 1.37 | | 0.21 | | 0.67 | | 0.22 | |
| GRG + Tocopherol 0.3 mM (Group 3) | 0.96 | 1.46 | 0.94 | 0.28 | 0.77 | 0.30 | 0.76 | | 0.22 | | 1.26 | | 0.36 | |
| GRG + Tocopherol 1 mM (Group 4) | 2.19 | 2.82 | 1.74 | 0.33 | 1.21 | 0.37 | 1.54 | | 0.43 | | 0.35 | | 0.32 | |
| GRG + Tocopherol 3 mM (Group 5) | 1.52 | 1.63 | 2.78 | 0.16 | 0.87 | 0.21 | 0.97 | | 0.27 | | 1.11 | | 0.24 | |
| GRG + Tocopherol 10 mM (Group 6) | 1.46 | 1.02 | 1.02 | 0.23 | 2.22 | 0.32 | 0.72 | | 0.46 | | 1.06 | | 0.21 | |

| **Treatment Group #** | **Study Weeks** | | | | | | | | | | | | | |
|---|---|---|---|---|---|---|---|---|---|---|---|---|---|---|
| | **13** | | | | **16** | | | | | **19** | | | | |
| | **Right Leg** | | **Left Leg** | | **Right Leg** | | **Left Leg** | | | **Right Leg** | | | **Left Leg** | |
| | **MEAN** | | | | | | | | | | | | | |
| ANG (Group 1) | 22.62 | | 6.73 | | 22.42 | | 7.31 | | | 21.15 | | | 8.96 | |
| Empty NeuraGen(R) nerve guide (Group 2) | 23.55 | | 2.69 | | 23.58 | | 3.39 | | | 21.40 | | | 5.75 | |
| GRG + Tocopherol 0.3 mM (Group 3) | 21.82 | | 3.05 | | 23.21 | | 5.34 | | | 23.37 | | | 7.77 | |
| GRG + Tocopherol 1 mM (Group 4) | 21.11 | | 1.90 | | 23.95 | | 3.43 | | | 24.40 | | | 5.09 | |
| GRG + Tocopherol 3 mM (Group 5) | 21.83 | | 4.15 | | 23.70 | | 5.64 | | | 23.50 | | | 8.08 | |
| GRG + Tocopherol 10 mM (Group 6) | 24.23 | | 3.87 | | 21.47 | | 5.43 | | | 24.68 | | | 8.63 | |

| | **SEM** | | | | | | | | | | | | | |
|---|---|---|---|---|---|---|---|---|---|---|---|---|---|---|
| ANG (Group 1) | 0.69 | | 0.94 | | 1.14 | | 1.18 | | | 1.08 | | | 1.07 | |
| Empty NeuraGen^{®} nerve guide (Group 2) | 0.79 | | 0.57 | | 1.05 | | 0.68 | | | 0.58 | | | 0.91 | |
| GRG + Tocopherol 0.3 mM (Group 3) | 0.74 | | 0.54 | | 0.61 | | 1.27 | | | 0.64 | | | 1.49 | |
| GRG + Tocopherol 1 mM (Group 4) | 0.90 | | 0.40 | | 1.07 | | 0.78 | | | 0.96 | | | 0.84 | |
| GRG + Tocopherol 3 mM (Group 5) | 0.71 | | 0.67 | | 0.95 | | 0.94 | | | 1.39 | | | 1.12 | |
| GRG + Tocopherol 10 mM (Group 6) | 1.22 | | 0.93 | | 0.90 | | 0.84 | | | 1.11 | | | 1.09 | |

**Table 12: Amplitude normalized to baseline**

| **Treatment Group #** | **Study Weeks** | | | | | | | | | |
|---|---|---|---|---|---|---|---|---|---|---|
| | **-1** | | **2** | | **4** | | **6** | | **10** | |
| | **Right Leg** | **Left Leg** | **Right Leg** | **Left Leg** | **Right Leg** | **Left Leg** | **Right Leg** | **Left Leg** | **Right Leg** | **Left Leg** |
| | **MEAN** | | | | | | | | | |
| ANG (Group 1) | 1.00 | 1.00 | 1.31 | 0.05 | 1.00 | 0.08 | 1.01 | 0.07 | 1.12 | 0.20 |
| Empty NeuraGen^{®} nerve guide (Group 2) | 1.00 | 1.00 | 1.36 | 0.04 | 1.14 | 0.07 | 1.12 | 0.06 | 1.07 | 0.07 |
| GRG + Tocopherol 0.3 mM (Group 3) | 1.00 | 1.00 | 1.00 | 0.07 | 0.99 | 0.09 | 0.86 | 0.08 | 0.91 | 0.10 |
| GRG + Tocopherol 1 mM (Group 4) | 1.00 | 1.00 | 0.99 | 0.06 | 0.97 | 0.08 | 0.91 | 0.07 | 0.91 | 0.07 |
| GRG + Tocopherol 3 mM (Group 5) | 1.00 | 1.00 | 1.32 | 0.05 | 1.01 | 0.09 | 1.05 | 0.08 | 1.03 | 0.07 |
| GRG + Tocopherol 10 mM (Group 6) | 1.00 | 1.00 | 1.33 | 0.07 | 1.30 | 0.10 | 1.17 | 0.10 | 1.03 | 0.07 |

| | **SEM** | | | | | | | | | |
|---|---|---|---|---|---|---|---|---|---|---|
| ANG (Group 1) | 0.00 | 0.00 | 0.11 | 0.01 | 0.08 | 0.01 | 0.09 | 0.01 | 0.12 | 0.04 |
| Empty NeuraGen^{®} nerve guide (Group 2) | 0.00 | 0.00 | 0.10 | 0.01 | 0.10 | 0.02 | 0.09 | 0.01 | 0.07 | 0.01 |
| GRG + Tocopherol 0.3 mM (Group 3) | 0.00 | 0.00 | 0.06 | 0.01 | 0.05 | 0.02 | 0.03 | 0.01 | 0.05 | 0.02 |
| GRG + Tocopherol 1 mM (Group 4) | 0.00 | 0.00 | 0.09 | 0.02 | 0.07 | 0.02 | 0.07 | 0.02 | 0.08 | 0.01 |
| GRG + Tocopherol 3 mM (Group 5) | 0.00 | 0.00 | 0.21 | 0.01 | 0.07 | 0.01 | 0.08 | 0.01 | 0.09 | 0.01 |
| GRG + Tocopherol 10 mM (Group 6) | 0.00 | 0.00 | 0.13 | 0.01 | 0.10 | 0.02 | 0.11 | 0.02 | 0.04 | 0.01 |

| | **Treatment** | | | **Study Weeks** | | | | | | |
|---|---|---|---|---|---|---|---|---|---|---|
| | | | | **13** | | **16** | | **19** | | |
| | | | | | | | | | | |

| **Group #** | **Righ t Leg** | **Left Leg** | **Right Leg** | | **Left Leg** | | **Right Leg** | | **Left Leg** | |
|---|---|---|---|---|---|---|---|---|---|---|
| | **MEAN** | | | | | | | | | |
| ANG (Group 1) | 1.04 | 0.31 | 1.03 | | 0.33 | | 0.98 | | 0.41 | |
| Empty NeuraGen^{®} nerve guide(Group 2) | 1.08 | 0.12 | 1.10 | | 0.15 | | 0.98 | | 0.27 | |
| GRG + Tocopherol 0.3 mM (Group 3) | 0.82 | 0.13 | 0.88 | | 0.23 | | 0.88 | | 0.34 | |
| GRG + Tocopherol 1 mM (Group 4) | 0.83 | 0.07 | 0.94 | | 0.13 | | 0.91 | | 0.20 | |
| GRG + Tocopherol 3 mM (Group 5) | 0.93 | 0.18 | 1.01 | | 0.24 | | 1.00 | | 0.34 | |
| GRG + Tocopherol 10 mM (Group 6) | 1.14 | 0.16 | 1.04 | | 0.24 | | 1.22 | | 0.38 | |

| | **SEM** | | | | | | | | | |
|---|---|---|---|---|---|---|---|---|---|---|
| AN G (Group 1) | 0.09 | 0.05 | 0.11 | | 0.06 | | 0.12 | | 0.05 | |
| Empty NeuraGen^{®} nerve guide (Group 2) | 0.07 | 0.02 | 0.09 | | 0.03 | | 0.05 | | 0.05 | |
| GRG + Tocopherol 0.3 mM (Group 3) | 0.03 | 0.02 | 0.04 | | 0.05 | | 0.03 | | 0.07 | |
| GRG + Tocopherol 1 mM (Group 4) | 0.06 | 0.01 | 0.07 | | 0.02 | | 0.07 | | 0.03 | |
| GRG + Tocopherol 3 mM (Group 5) | 0.07 | 0.03 | 0.08 | | 0.05 | | 0.08 | | 0.05 | |
| GRG + Tocopherol 10 mM (Group 6) | 0.06 | 0.03 | 0.08 | | 0.04 | | 0.12 | | 0.04 | |

**Table 13: Amplitude normalized to the contralateral leg in each testing time-point**

| **Treatment Group #** | **Study weeks** | | | | | | | |
|---|---|---|---|---|---|---|---|---|
| | **Baseline** | **2** | **4** | **6** | **10** | **13** | **16** | **19** |
| | **MEAN** | | | | | | | |
| ANG (Group 1) | 1.00 | 0.04 | 0.08 | 0.07 | 0.18 | 0.30 | 0.33** | 0.43* |
| Empty NeuraGen^{®} nerve guide (Group 2) | 1.15 | 0.03 | 0.07 | 0.06 | 0.06 | 0.12 | 0.14 | 0.27 |
| GRG + Tocopherol 0.3 mM (Group 3) | 0.89 | 0.06 | 0.08 | 0.08 | 0.10 | 0.14 | 0.24 | 0.34 |
| GRG + Tocopherol 1 mM (Group 4) | 1.01 | 0.06 | 0.08 | 0.08 | 0.07 | 0.10 | 0.16 | 0.21 |
| GRG + Tocopherol 3 mM (Group 5) | 1.02 | 0.04 | 0.09 | 0.08 | 0.07 | 0.19 | 0.24 | 0.36 |
| GRG + Tocopherol 10 mM (Group 6) | 1.11 | 0.05 | 0.09 | 0.09 | 0.07 | 0.17 | 0.26* | 0.36 |

| | **SEM** | | | | | | | |
|---|---|---|---|---|---|---|---|---|
| ANG (Group 1) | 0.07 | 0.01 | 0.01 | 0.01 | 0.03 | 0.04 | 0.05 | 0.05 |
| Empty NeuraGen^{®} nerve guide (Group 2) | 0.11 | 0.01 | 0.02 | 0.01 | 0.01 | 0.03 | 0.03 | 0.05 |
| GRG + Tocopherol 0.3 mM (Group 3) | 0.06 | 0.01 | 0.01 | 0.01 | 0.02 | 0.03 | 0.06 | 0.06 |
| GRG + Tocopherol 1 mM (Group 4) | 0.11 | 0.01 | 0.01 | 0.02 | 0.01 | 0.02 | 0.04 | 0.03 |
| GRG + Tocopherol 3 mM (Group 5) | 0.08 | 0.01 | 0.01 | 0.01 | 0.01 | 0.03 | 0.04 | 0.06 |
| GRG + Tocopherol 10 mM (Group 6) | 0.08 | 0.01 | 0.01 | 0.02 | 0.01 | 0.04 | 0.04 | 0.06 |

| | | | | | | | | |
|---|---|---|---|---|---|---|---|---|
| * p < 0.05 vs. empty NeuraGen^{®} nerve guide using T-test. ** p < 0.01 vs. empty NeuraGen^{®} nerve guide using T-test. | | | | | | | | |

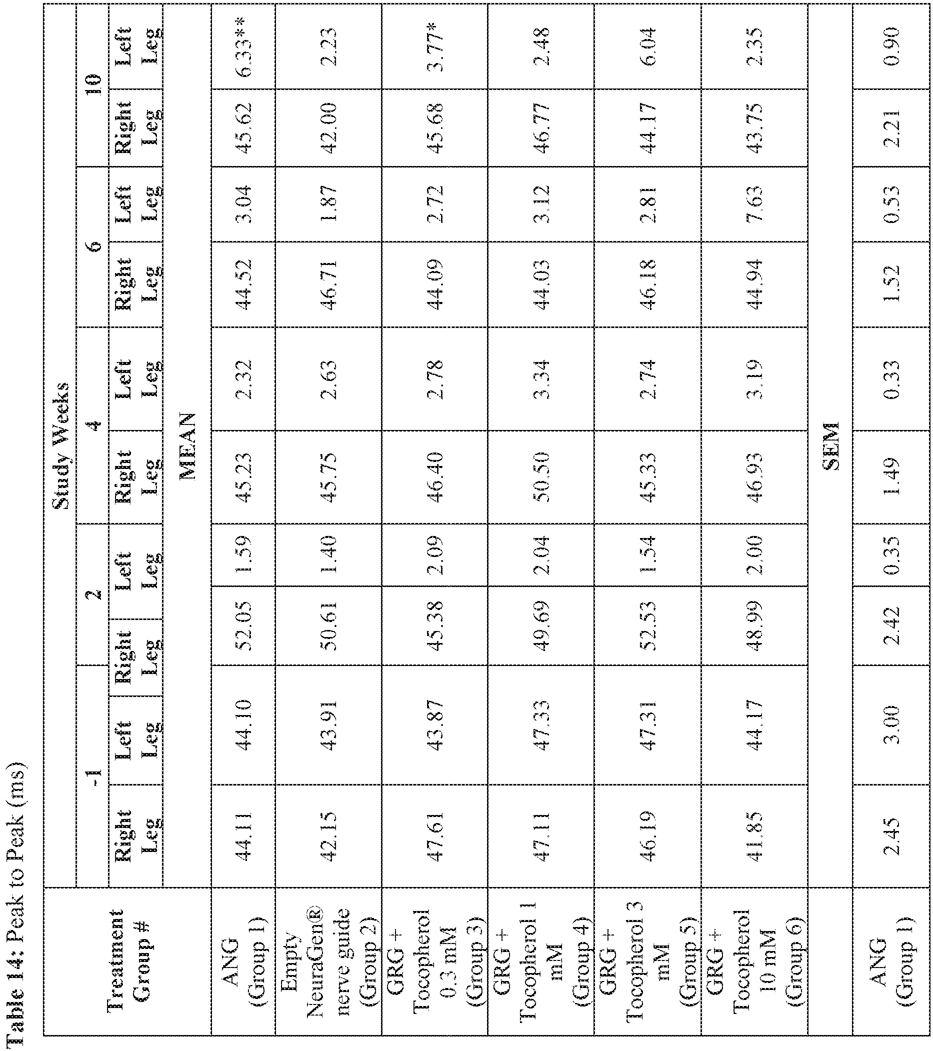

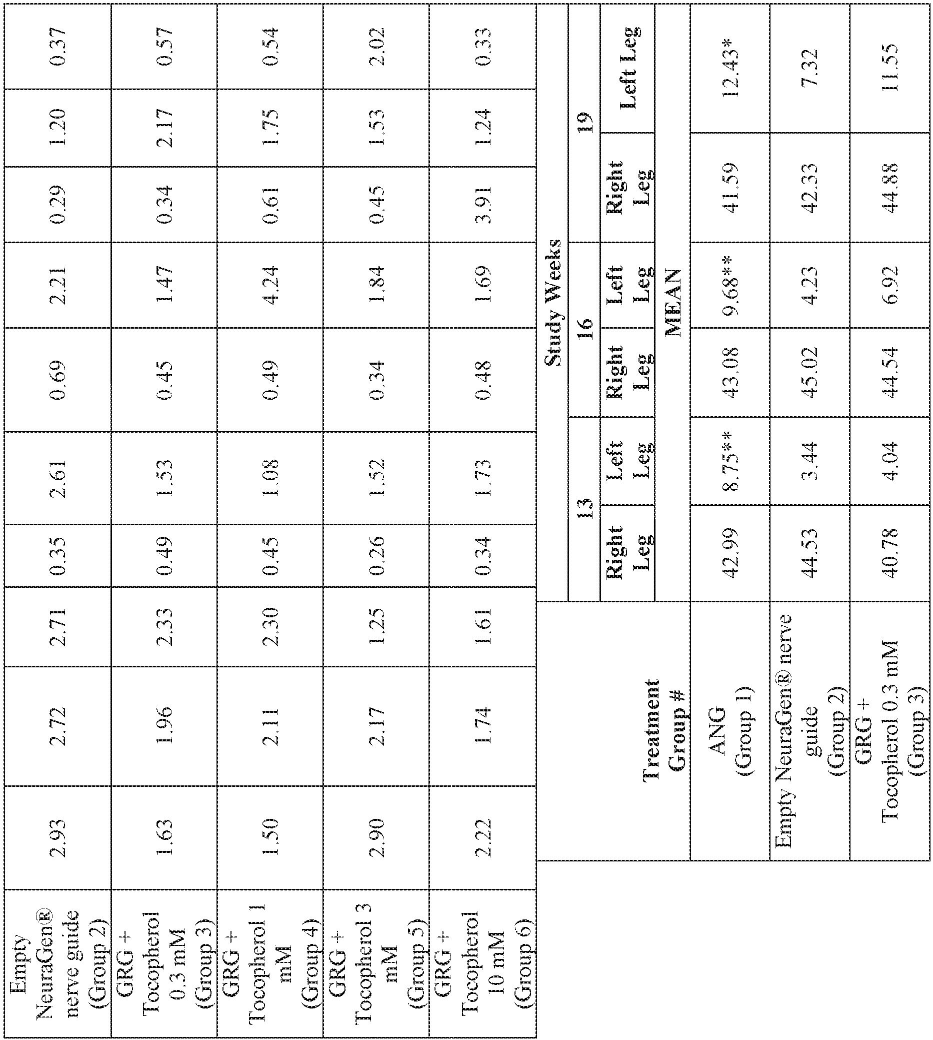

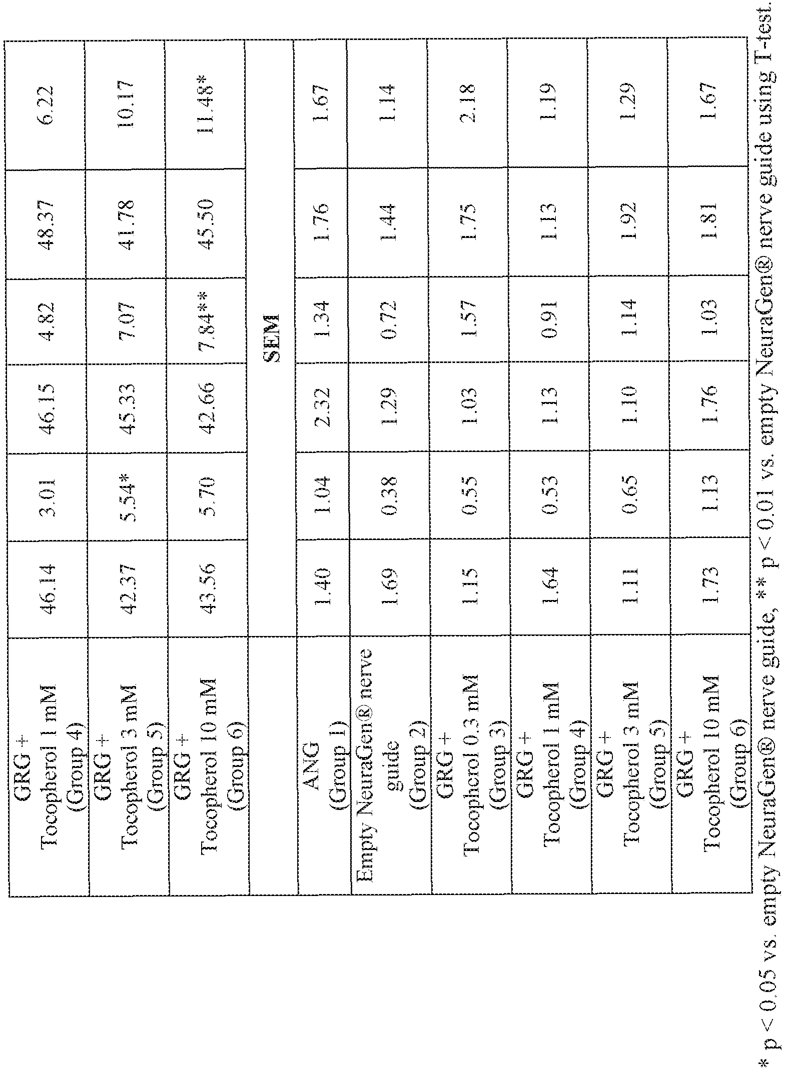

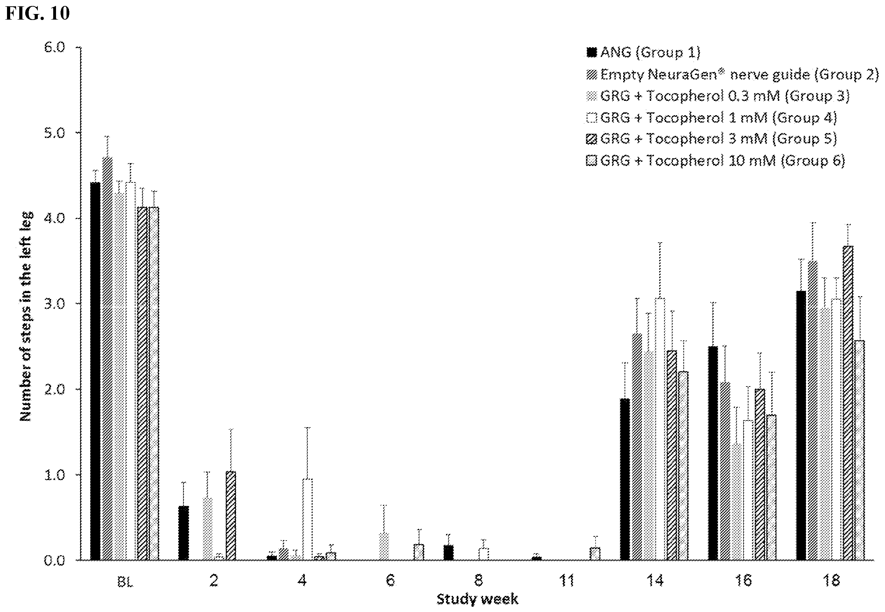

*Catwalk:* Gait analysis in rodent using the catwalk system is a well-established method in peripheral nerve injury, as well as spinal cord injury models (Bozkurt A, et al. J of Neuroscience Methods, 2008, 173:91-98; and Kappos EA et al. Brain and Behavior. 2017, 3:1-12). Analysis of the gait showed that in parallel to the improvement in the CMAPs the animals started using their operated leg more (Table 15 hereinbelow and Figure 10). The number of steps represented the actual use that the animals did with their leg. Number of steps was considered each time the system identified a step, it did not have to be a perfect shape step. Until day 14 the animals hardly stepped on the operated leg. However, starting on week 14, the animals have used their injured legs for walking. As the study progresses, the animals used more the operated leg. As an example, on week 11 the mean group number of steps that the empty NeuraGen^{®} nerve guide treated animals completed was 0.00 ± 0.00 steps vs. 4.71 ± 0.25 steps at baseline before the operation (p < 0.001). On study week 18, these animals walked 3.50 ± 0.45 steps, still statistically lower than baseline values (p < 0.05) but significantly better than week 11 or values recorded at earlier weeks. Treatment with GRG comprising Tocopherol resulted in an improvement in the number of steps at the operated leg (Table 15 hereinbelow and Figure 10).

No toe spread analysis (the length between the lateral toes) was possible as all the animals walked using ankle placement without spreading their paws and without touching the floor surface of the catwalk apparatus with their toes.

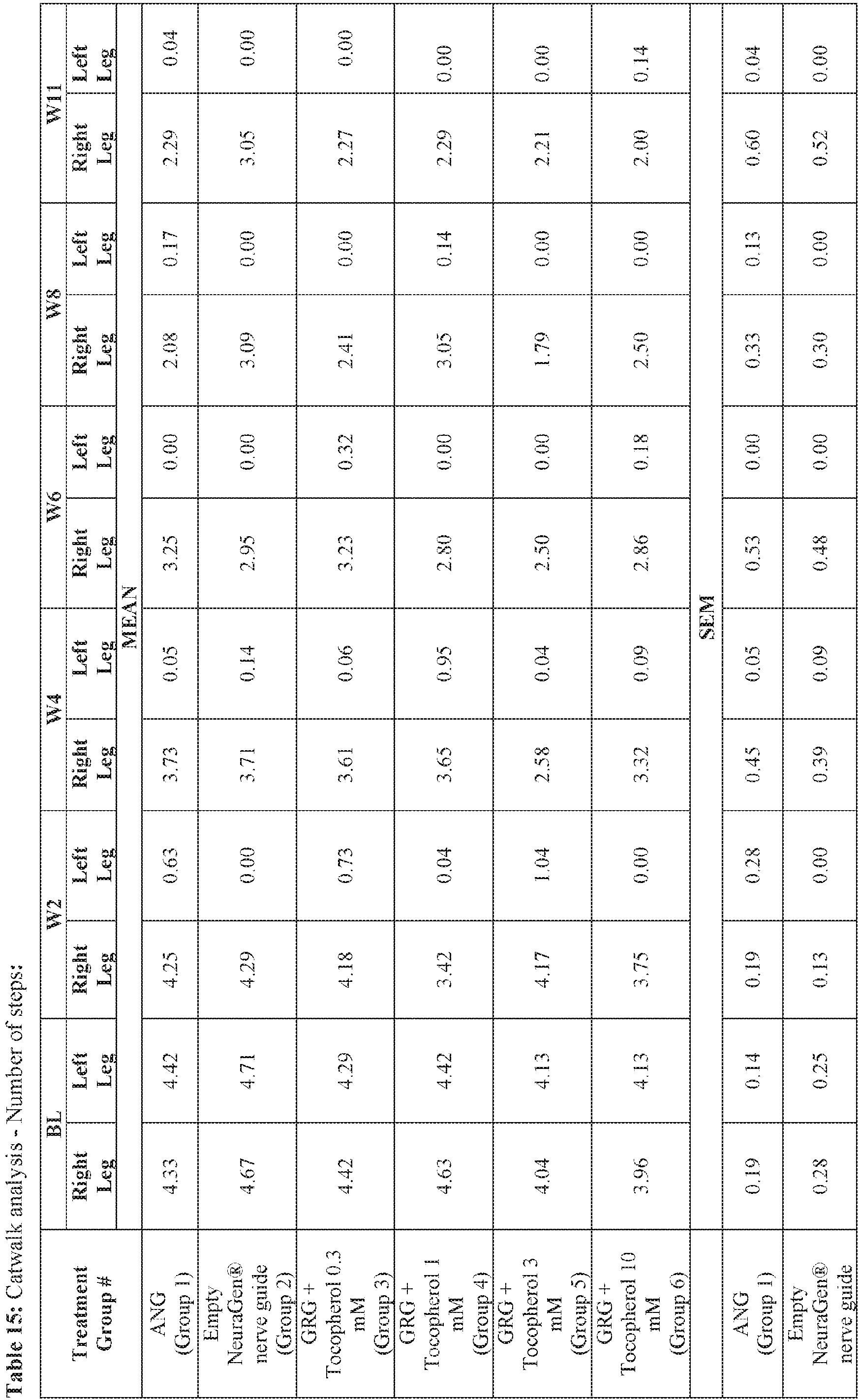

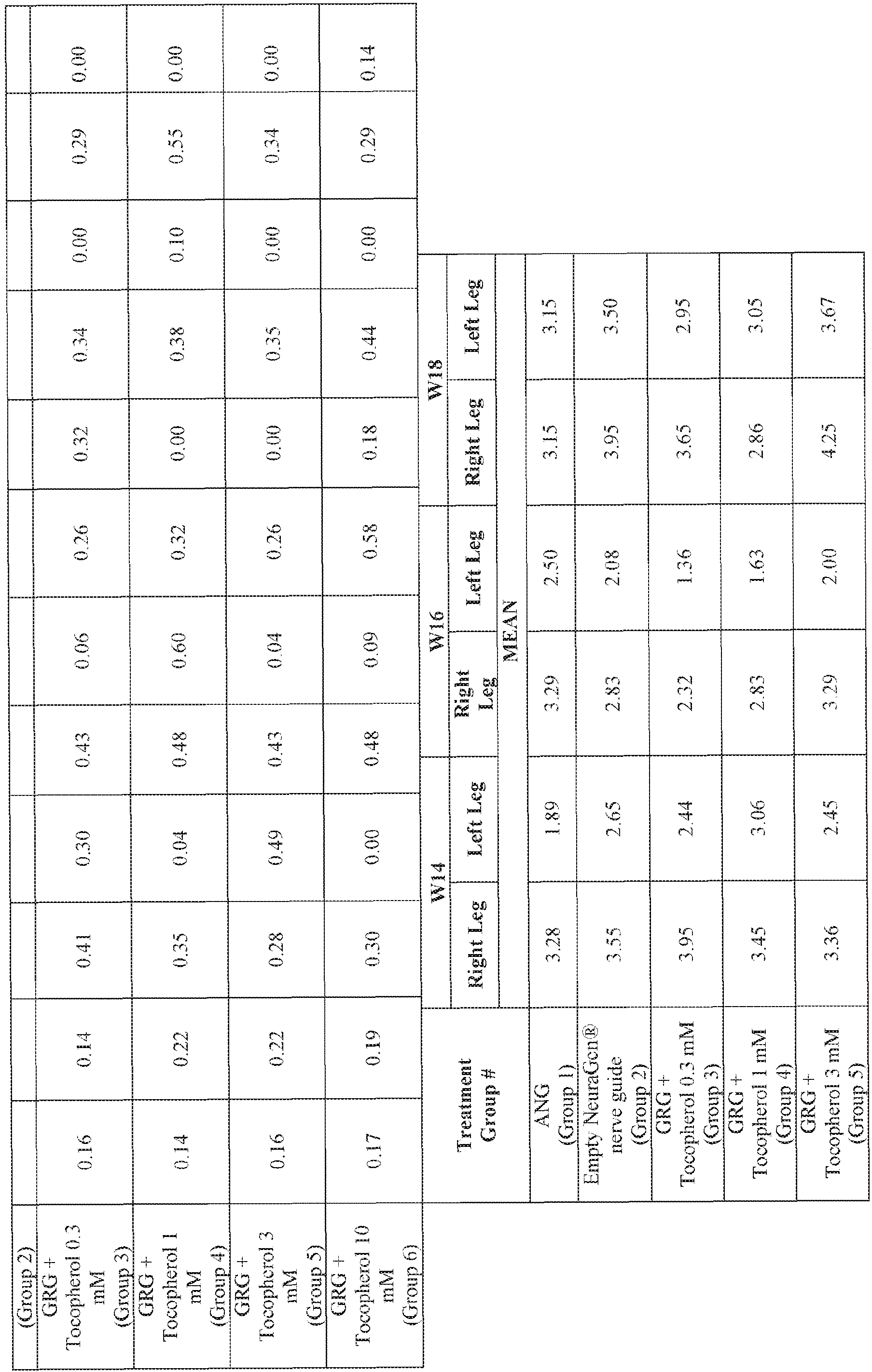

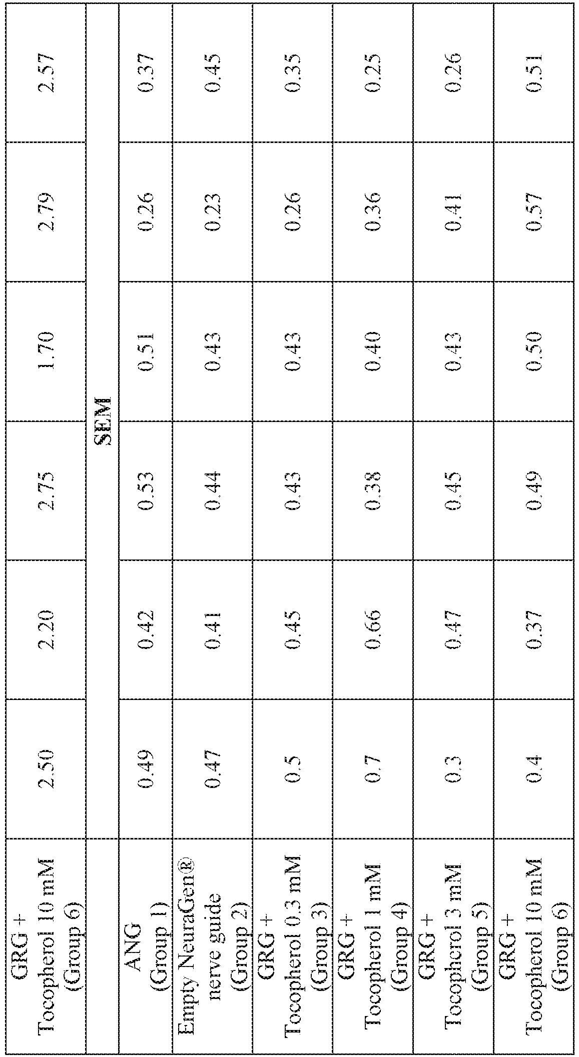

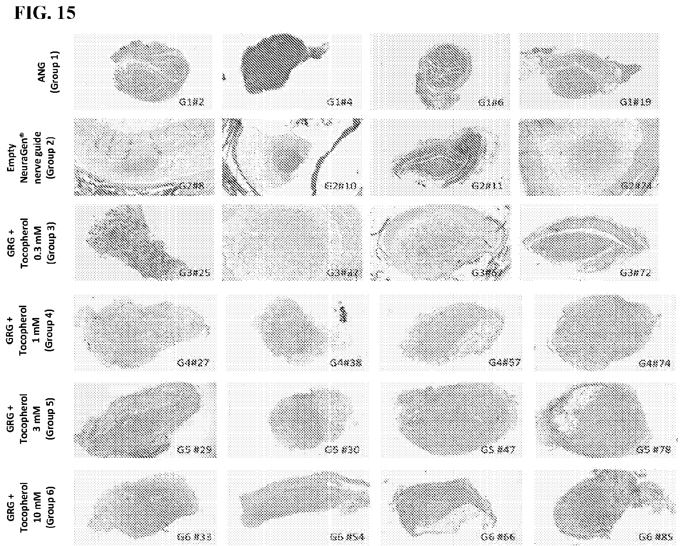

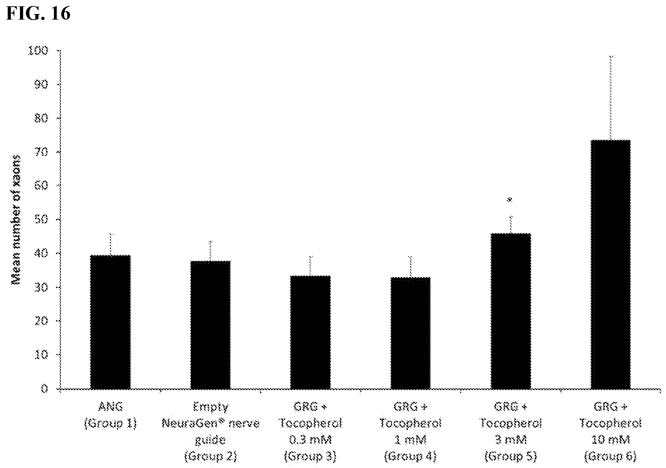

*Histological evaluation:* A general observation of the middle section taken from the injury area (Figure 15), demontrated that in the GRG comprising 3 mM Tocopherol and GRG comprising 10 mM Tocopherol treatment groups (groups 5 and 6, respectively), the newly formed nerve bundle is dense and consolidated / unified. In comparison, the nerve harvested from the GRG comprising 0.3 mM Tocopherol and GRG comprising 1 mM Tocopherol treamtment groupds (groups 3 and 4, respectively) showed a more loose formed nerve bundle with distinguished fibers. In the empty NeuraGen^{®} nerve guide treatment group (group 2), in the cases a new bundle was formed it is usually thin, and in several cases inflammation is depicted. Autologus tansplantation (group 1) resulted in usually thin bundles. Evaluating the number of axons, the highest number of axons in distal sections was detected following treatment with GRG comprising 10 mM Tocopherol (group 6). Excluding samples where the number of axons was lower than 10 (probably due to technical issues), the mean axon number at the distal sections (Table 25 hereinbelow and Figure 16) following treatment with GRG comprising 3 mM Tocopherol (group 5) was significantly higher in comparison with the empty NeuraGen^{®} nerve guide control (group 2): 45.82 ± 4.91 vs. 27.75 ± 5.60, respectevly (p < 0.05).

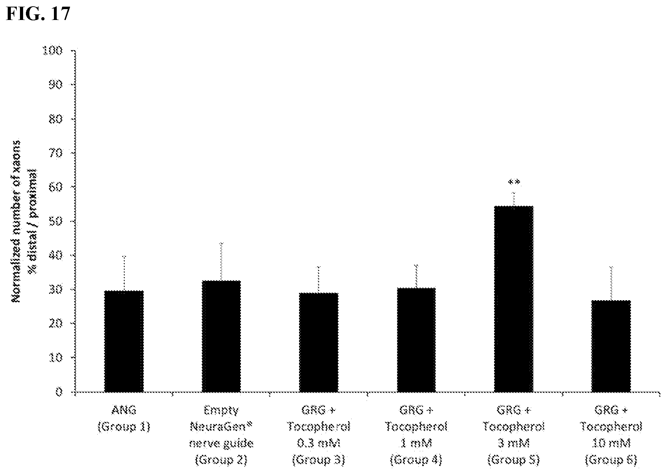

Normalization of axons' number from distal section to the corresponding proximal section (Table 26 hereinbelow and Figure 17) also demonstrated a statistically significant higher mean percentage of axons following treatment with GRG comprising 3 mM Tocopherol (group 5) as compared to empty NeuraGen^{®} nerve guide (group 2): 54.42 ± 3.89 vs. 32.46 ± 11.00, respectevly (p < 0.01).

**Table 25: Mean number of axons at distal section (only sections with number of axons > 10)**

| **Group #** | **# Group size** | **Treatment** | **# of axons in distal section** | |
|---|---|---|---|---|
| | | | **MEAN** | **SEM** |
| 1 | 6 | ANG (Group 1) | 39.33 | 6.33 |
| 2 | 8 | Empty NeuraGen^{®} nerve guide (Group 2) | 37.75 | 5.60 |
| 3 | 7 | GRG + Tocopherol 0.3 mM (Group 3) | 33.43 | 5.43 |
| 4 | 9 | GRG + Tocopherol 1 mM (Group 4) | 33.00 | 5.84 |
| 5 | 11 | GRG + Tocopherol 3 mM (Group 5) | 45.82* | 4.91 |
| 6 | 6 | GRG + Tocopherol 10 mM (Group 6) | 73.67 | 24.60 |

| | | | | |
|---|---|---|---|---|
| * p < 0.05 using T-test vs. empty NeuraGen^{®} Nerve Guide. | | | | |

**Table 26: Mean normalized number of axons (distal / proximal)**

| **Group #** | **# Group size** | **Treatm ent** | **% axons distal / proximal** | |
|---|---|---|---|---|
| | | | **MEAN** | **SEM** |
| 1 | 9 | ANG (Group 1) | 29.58 | 10.05 |
| 2 | 10 | Empty NeuraGen^{®} nerve guide (Group 2) | 32.46 | 11.00 |
| 3 | 11 | GRG + Tocopherol 0.3 mM (Group 3) | 28.99 | 7.54 |
| 4 | 11 | GRG + Tocopherol 1 mM (Group 4) | 30.40 | 6.69 |
| 5 | 10 | GRG + Tocopherol 3 mM (Group 5) | 54.42 | 3.89 |
| 6 | 8 | GRG + Tocopherol 10 mM (Group 6) | 26.75 | 9.89 |

| | | | | |
|---|---|---|---|---|
| * p < 0.01 using F-test vs. empty NeuraGen^{®} Nerve Guide. | | | | |

Taken together, the results suggest that treatment with GRG comprising Tocopherol at a concentration of 3 mM and 10 mM were active in improving the clinical score autotomy phenomenon,CMAPs and histology in the rat peripheral nerve injury model.

### EXAMPLE 3

### IN-VITRO EFFECT OF GRG AND A COMBINATION OF GRG AND ETANERCEPT OR COPAXONE ON NEURITE OUTGROWTH

### MATERIALS AND METHODS

***Materials*** - All vendors and storage conditions are shown in Table 16 hereinbelow.

Vehicle - DMSO 0.6 % or 1 %: 100 % DMSO was diluted in culture media to obtain a final concentration of 0.6 % or 1 %.

Copaxone at a concentration of 10 µg / ml or 50 µg / ml or 150 µg / ml: A stock solution of 20 mg / ml was used. For a final concentration of 150 µg / ml, 7.5 ul of this solution was added to each 1 ml medium. For a final concentration of 50 µg / ml, 2.5 µl of this solution was added to each 1 ml medium. For a final concentration of 10 µg / ml, 0.5 µl of this solution was added to each 1 ml medium.

Enbrel (Entanercept) at a concentration of 1 µg / ml, 10 µg / ml or 50 µg / ml: A stock solution of 50 mg / ml was used. For a final concentration of 50 µg / ml, 1 µl of this solution was added to each 1 ml medium. For a final concentration of 10 µg / ml, the stock solution was diluted 1:10 in PBS, and then 2 µl were added to each 1 ml medium. For a final concentration of 1 µg / ml, the stock solution was diluted 1:100 in PBS, and then 2 µl were added to each 1 ml medium.

D-L Tocopherol at a concentration of 0.3 mM or 1 mM or 3 mM: To achieve a final concentration of 3 mM in 0.4 % DMSO, 193.8 mg were weighed and diluted with 1 ml DMSO 100 %. This generated a solution at a concentration of 450 mM with 60 % DMSO, as the density of Tocopherol is 0.950 g / ml. For a final concentration of 1 mM, the stock solution (450 mM) were diluted 1:3 with 60 DMSO, to achieve a solution at a concentration of 150 mM. For a final concentration of 0.3 mM, the stock solution (450 mM) were diluted 1:10 with 60 % DMSO, to achieve a solution at a concentration of 45 mM. 6.67 µl of each solution were added to each 1 ml medium.

Complete Culture Media: To formulate the complete culture media, DMEM medium was supplemented with 2 % B27, 5 % horse serum, 1 % Glutamax and 3 µl gentamycin.

Incomplete Culture Media: Incomplete culture media contained DMEM medium with 2 % B27 and 1 % Glutamax.

0.2 % Hyaluronic Acid (HA): 0.2 % of HA in incomplete culture media was prepared aseptically and stored at 4 °C.

Laminin: A stock solution of Laminin at a concentration of 25 mg / ml was prepared aseptically in 100 % DMSO, filtered with 0.22 µm filter, and divided into aliquots and stored at -20 °C. For a final concentration of 10 µg / ml, the stock solution was diluted in DMSO to obtain a solution at a concentration of 5 mg / ml. Following, 4 µl from each solution was added to 2 ml incomplete culture media.

Guiding regenrative gel (GRG) formulation: GRG formulation was prepared as 0.2 % HA solution with the addition of Laminin at a final concentration of 10 µg / ml and Tocopherol at a final concentration of 3 mM.

**Table 16: list of materials**

| **Materials** | **Name** | **Cat. No.** | **Manufacturer** | **Storage Conditions** |
|---|---|---|---|---|
| Test Items | Vitamin E (Tocopherol) | 613420 | Millipore | 4 °C |
| | Enbrel (Etanercept) | N/A | Pfizer | 4 °C |
| | Glatiramer acetate (Copaxone) | N/A | Teva | 4 °C |
| Vehicle | 0.6 % DMSO | D5879 | SIGMA | RT |
| Culture and Maintenance Medium | Neurobasal Medium | 21103-049 | GIBCO | 4 °C |
| | 1 % glutamax | 35050-038 | GIBCO | RT |
| | 2 % B27 | 17504-044 | GIBCO | -20 °C |
| | 1 ug / ml gentamycin | J62834.1 | Holland Moran | RT |
| | 5 % horse Serum | 04-124-1A | Biological Industry | -20 °C |
| | DMEM | 01-052-1A | Biological Industry | 4 °C |
| | HBSS | 02-015-1B | Biological Industry | RT |
| Coating of Cover slips | ECL (Cell Attachment Matrix) | 08-110 | Millipore | -20 °C |
| | PDL (poly-D-lysine solution) | P0899 | Sigma | -20 °C |
| | Sterile dH2O | NA | NA | NA |
| Euthanasia Items | Ketamine | 036058774 23548 | Vetoquinol | RT |
| | Xylazine | 084697 | Phibro | RT |

***Animal used for purification of adult spine primary cells*** - Male Sprague-Dawley Rats (Harlan Laboratories Israel, Ltd.) 3 - 4 weeks old. Only animals in good health were acclimatized to laboratory conditions and used in the study. Weight variation of animals at the time of treatment initiation was not exceeded ± 20 % of the mean weight. During acclimation and throughout the entire study duration, animals were housed within a limited access rodent facility and kept in groups with a maximum of 2 rats per cage. The cages are made of polypropylene, fitted with solid bottoms and filled with sterile wood shavings as bedding material. Animals were provided ad libitum with a commercial rodent diet and had free access to drinking water that was supplied to each cage via polyethylene bottles with stainless steel sipper tubes. A feed lot analysis of the diet batch used in the study was included in the archives with the study data. Water was monitored periodically. Automatically controlled environmental conditions were set to maintain temperature at 17 - 23 °C with a relative humidity (RH) of 30 - 70 %, a 12:12 hour light: dark cycle and 15 - 30 air changes / h in the study room temperature and RH were monitored daily. The light cycle was monitored by a control clock.

***Coating of culture cover slips*** - Coverslips (CSs) were cleaned by shaking overnight in 1 M HCl, rinsed in DW and sterilized overnight in autoclave. The Coverslips were coated with either Cell Attachment Matrix (ECL) or Poly-D-lysine (PDL) as follows: ECL solution (Millipore 08-110) at a concentration of 40 µg / ml was prepared using sterile PBS. 300 µl of this solution were added on top of the coverslip for overnight incubation at 4 °C. PDL solution (Sigma P6407) at a concentration of 0.1 mg / ml was prepared using sterile dH2O. 300 µl of this solution were added on top of the coverslip for overnight incubation at 4 °C.

***In-vitro assay for evaluation of Neurite outgrowth** -* A schematic representation of the in-vitro assay is shown in Figure 11. 3 - 4 weeks old Rat was euthanized with Ketaminexylazine solution. Following the spinal cord was removed, placed in a sterile 10 mm petri dish with HBSS medium (without calcium and magnesium), and kept on ice. The meninges were stripped away; and the spine was dissected into small pieces and collected into a 15 ml centrifuge tube with 40 % TrypLE in HBSS. The tube was agitated horizontally at RT for 20 minutes. Following, the tube was centrifuged for 5 minutes at 200 g. The supernatant was discarded and 2 ml of fresh Complete Culture Media were added. The cells were dissociated by pipetting up and down 10 times, first in a normal Pasteur pipette, followed by 10 times in a pipette with a tip fire polished to nearly half the normal diameter. Clumps were left to stand for 5 minutes and the supernatant was collected into a new 15 ml centrifuge tube. The cells were then seeded on a coverslip in Complete Culture Media for 24 hours. Following 1 day of incubation, the Complete Culture Media was replaced to Incomplete Culture Media, and the tested compounds were added. Pictures from different areas were taken at various time-points from at least 3 wells per treatment group; and neurite outgrowth was evaluated by determining total number of neurites, total number of cells, mean neurite length / cell, mean number of neurites / cell and mean number of bifurcations / cell using ImageJ software with the NeuronJ plugin.

***Statistical Evaluation*** - Data is presented as mean ± SEM. Each treatment group is compared to a relevant group using one way ANOVA followed by Dunnet's post hoc test or Student's T-test. A p < 0.05 is considered to represent a significant difference.

### RESULTS

*In-vitro effect of GRG on neurite outgrowth:* Guiding Regenerative Gel (GRG) formulation comprises 3 components: Hyaluronic acid (HA), Laminin peptide and an antioxidant (e.g. SOD1, D-L Tocopherol). To test the effect of GRG on neurite outgrowth primary adult rat spine cells were cultured with DMEM media and DMEM with GRG (Figure 11). Treatment with GRG formulation comprising 0.2 HA, 3 mM Tocopherol and 10 µg / ml Laminin peptide significantly increased neurite outgrowth, manifested by increased number of neurites, increased neurite length and increased number of bifurcations (Table 17 hereinbelow).

In the next step, the effect of different concentrations of Laminin or Tocopherol in the GRG formulation on Neurite outgrowth was evaluated. As shown in Table 18 hereinbelow, GRG formulation comprising 3 mM Tocopherol was more effective in increasing neurite outgrowth at all parameters tested. For further studies, a GRG formulation comprising 0.2 % HA + 3 mM Tocopherol + 10 µg / ml Laminin was selected and the duration of culture was determined to be between 7-10 days. Importantly, no significant change on neurite outgrowth was seen following the addition of 1 % DMSO (the highest concentration used in this study) (Table 19 hereinbelow).

**Table 17: The effect of GRG on in-vitro neurite growth of rat primary spine cells.**

| **Treatment** | **Total number of neurites** | **Total number of cells** | **Mean neurite length / cell (µm)** | **Mean number of neurites / cell** | **Mean number of bifurcations / cell** |
|---|---|---|---|---|---|
| DMEM only | 16 | 6 | 275.43 ± 50.83 | 2.00 ± 1.00 | 0.20 ± 0.20 |
| GRG (HA 0.2 %+TOCO 3 mM + Laminin 10 µg/ml) | 159 | 65 | 285.08 ± 23.18 | 2.56 ± 0.14 | 0.36 ± 0.07 |

**Table 18: The effect of different concentrations of Tocopherol and/or Laminin peptide on in-vitro neurite growth of rat primary spine cells.**

| **Treatment** | **Total number of neurites** | **Total number of cells** | **Mean neurite length / cell (µm)** | **Mean number of neurites / cell** | **Mean number of bifurcations / cell** |
|---|---|---|---|---|---|
| HA 0.4 % + TOCO 0.3 mM + LAM 10 ug / | 5 | 5 | 16.62 ± 8.74 | 1.00 ± 0.00 | 0.00 ± 0.00 |
| HA 0.4 % + TOCO 1 mM + LAM 10 ug/ | 1 | 1 | 6.45 ± 0.00 | 1.00 ± 0.00 | 0.00 ± 0.00 |
| HA 0.4 % + TOCO 3 mM + LAM 2ug / ml | 35 | 18 | 150.51 ± 38.84 | 2.12 ± 0.21 | 0.44 ± 0.26 |
| HA +0.4 % + TOCO 3 mM LAM 10 ug / ml | 22 | 11 | 155.13 ± 37.60 | 2.00 ± 0.22 | 0.36 ± 0.28 |
| HA 0.4 % + TOCO 3 mM + LAM 50 ug / ml | 36 | 14 | 240.26 ± 40.44 | 2.63 ± 0.42 | 0.27 ± 0.13 |

**Table 19: No effect of DMSO on in-vitro neurite growth of rat primary spine cells.**

| **Treatment** | **Total number of neurites** | **Total number of cells** | **Mean neurite length / cell (µm)** | **Mean number of neurites / cell** | **Mean number of bifurcations / cell** |
|---|---|---|---|---|---|
| DMEM | 12 | 9 | 93.61 ± 15.05 | 1.36 ± 0.28 | 0.00 ± 0.00 |
| % HA 0.2 | 39 | 16 | 132.10 ± 13.41 | 2.19 ± 0.29 | 0.60 ± 0.20 |
| % HA 0.2 %+ DMSO 1 | 47 | 21 | 162.09 ± 24.31 | 2.39 ± 0.22 | 0.71 ± 0.26 |

*In-vitro effect of a combination of and Etanercept on neurite outgrowth:* The effect of a combination of GRG and Etanecept (Enbrel^{®}) on neurite outgrowth was evaluated in-vitro on primary adult rat spine cells and compared to the effect of treatment only with GRG (Figure 11). Treatment with GRG resulted in an increase of more than 40 % in neurite outgrowth vs. DMEM (p < 0.05) treated cells.

In order to improve this effect Etanecept (Enbrel^{®}) was added to the GRG.

Treatment with Enbrel at a concentration of 50 µg / ml in combination with GRG resulted in a significant increase of 58.86 % in the mean neurite length per cell, compared to the GRG formulation only (Tables 20A-B). The mean neurite length / cell following treatment Enbrel at 50 µg / ml was 404.04 ± 58.51 µm / cell (p < 0.05 vs. GRG).

**Table 20A: The effect of a combination of GRG and Etanecept (Enbrel^{®}) on in-vitro neurite growth of rat primary spine cells**.**

| **Treatment** | **Study #** | **Total number of neurites** | **Total number of cells** | **Mean neurite length / cell (µm)** | **Mean number of neurites / cell** | **Mean number of bifurcations / cell** |
|---|---|---|---|---|---|---|
| DMEM | 5 | 64.00 | 32.00 | 152.04 | 2.08 | 0.44 |
| | 6 | 16.00 | 10.00 | 75.74 | 1.92 | 0.17 |
| | 7 | 50.00 | 22.00 | 142.44 | 2.27 | 0.63 |
| | 8 | 15.00 | 7.00 | 107.38 | 2.08 | 0.33 |
| | 9 | 12.00 | 9.00 | 93.61 | 1.36 | 0.00 |
| | **Mean** | **31.40** | **16.00** | **114.24** | **1.94** | **0.32** |
| | **SEM** | **10.70** | **4.79** | **14.46** | **0.16** | **0.11** |
| GRG (HA 0.2 % + TOCO 3 mM + LAM 10 ug / ml) | 4 | 42.00 | 20.00 | 149.02 | 2.04 | 0.79 |
| | 5 | 97.00 | 44.00 | 196.80 | 2.28 | 0.43 |
| | 6 | 82.00 | 39.00 | 124.80 | 2.22 | 0.49 |
| | 7 | 60.00 | 18.00 | 246.20 | 3.24 | 2.32 |
| | 8 | 46.00 | 14.00 | 260.77 | 3.29 | 2.13 |
| | 9 | 81.00 | 39.00 | 211.50 | 2.08 | 0.21 |
| | **Mean** | **68.00*** | **29.00** | **198.18*** | **2.53** | **1.06** |
| | **SEM** | **9.00** | **5.33** | **21.76** | **0.24** | **0.38** |
| GRG + Enbrel 50 ug / ml | 7 | 54.00 | 19.00 | 345.54 | 2.79 | 1.44 |
| | 8 | 152.00 | 152.00 | 462.55 | 2.91 | 1.22 |
| | **Mean** | **103.00** | **85.50** | **404.04#** | **2.85** | **1.33** |
| | **SEM** | **49.00** | **66.50** | **58.51** | **0.06** | **0.11** |
| GRG + Enbrel 10 ug / ml | 4 | 117.00 | 64.00 | 174.61 | 2.04 | 0.53 |
| GRG + Enbrel 1 ug / ml | 4 | 170.00 | 72.00 | 167.70 | 2.46 | 0.82 |

| | | | | | | |
|---|---|---|---|---|---|---|
| **Neurite outgrowth was determined on day 7 in studies 4 and 6, on day 9 on studies 5 and 7 and day 10 on studies 8 and 9. * p < 0.05 vs. DMEM (Student T-test) # p < 0.05 vs. GRG only. | | | | | | |

**Table 20B: The effect of a combination of GRG and Etanecept (Enbrel^{®}) on in-vitro neurite growth of rat primary spine cells* compared to treatment with GRG only**.**

| **Treatment** | **Study #** | **Total number of neurites (%)** | **Total number of cells (%)** | **Mean neurite length / cell (%)** | **Mean number of neurites / cell (%)** | **Mean number of bifurcations / cell (%)** |
|---|---|---|---|---|---|---|
| DMEM | 5 | 65.98 | 72.73 | 77.26 | 90.92 | 101.92 |
| | 6 | 19.51 | 25.64 | 60.69 | 86.22 | 34.12 |
| | 7 | 83.33 | 122.22 | 57.85 | 70.12 | 27.36 |
| | 8 | 32.61 | 50.00 | 41.18 | 63.29 | 15.69 |
| | 9 | 14.81 | 23.08 | 44.26 | 65.14 | 0.00 |
| | **Mean** | **43.25** | **58.73** | **56.25** | **75.14** | **35.82** |
| | **SEM** | **13.43** | **18.26** | **6.46** | **5.65** | **17.51** |
| GRG (HA 0.2 % + TOCO 3 mM + LAM 10 ug / ml) | 4 | 100.00 | 100.00 | 100.00 | 100.00 | 100.00 |
| | 5 | 100.00 | 100.00 | 100.00 | 100.00 | 100.00 |
| | 6 | 100.00 | 100.00 | 100.00 | 100.00 | 100.00 |
| | 7 | 100.00 | 100.00 | 100.00 | 100.00 | 100.00 |
| | 8 | 100.00 | 100.00 | 100.00 | 100.00 | 100.00 |
| | 9 | 100.00 | 100.00 | 100.00 | 100.00 | 100.00 |
| | **Mean** | **100.00** | **100.00** | **100.00** | **100.00** | **100.00** |
| | **SEM** | **0.00** | **0.00** | **0.00** | **0.00** | **0.00** |
| GRG + Enbrel 50 ug / ml | 7 | 90.00 | 105.56 | 140.35 | 86.17 | 62.18 |
| | 8 | 330.43 | 1085.71 | 177.38 | 88.29 | 57.35 |
| | **Mean** | **210.22** | **595.63** | **158.86** | **87.23** | **59.77** |
| | **SEM** | **120.22** | **490.08** | **18.52** | **1.06** | **2.42** |
| GRG + Enbrel 10 ug / ml | 4 | 278.57 | 320.00 | 117.17 | 99.87 | 66.78 |
| GRG + Enbrel 1 ug / ml | 4 | 404.76 | 360.00 | 112.54 | 120.28 | 104.17 |

| | | | | | | |
|---|---|---|---|---|---|---|
| *Neurite outgrowth was determined on day 7 in studies 4 and 6, on day 9 on studies 5 and 7 and day 10 on studies 8 and 9. **Results were normalized to treatment with GRG per each study. | | | | | | |

*In-vitro effect of a combination of GRG and Copaxone on neurite outgrowth:* The effect of a combination of GRG and several concentration of Copaxone on neurite outgrowth was evaluated in-vitro on primary adult rat spine cells and compared to the effect of treatment only with GRG (Figure 11).

Treatment with GRG resulted in an increase of more than 40 % in neurite outgrowth vs. DMEM (p < 0.05) treated cells. In order to improve this effect Copaxone was added to the GRG.

Treatment with Copaxone at a concentration of 10 or 150 µg / ml in combination with GRG resulted in a significant increase of 87.35 % and 79.05 %, respectively, in the mean neurite length per cell compared to the GRG formulation only (Tables 21A-B). The mean neurite length / cell following treatment with GRG was 198.18 ± 21.76 µm / cell vs. 366.32 ± 37.64 µm / cell following treatment with 10 µg / ml Copaxone, 335.11 ± 35.10 following treatment with 50 µg / ml Copaxone; and 453.41 ± 2.51 following treatment with 150 µg / ml Copaxone (p < 0.05). Analyzing the total number of neurites (Tables 21A-B) demonstrated that Copaxone treatment at a concentration of 10 µg / ml resulted in an increase of nearly 2 folds vs. GRG treatment (GRG 68.00 ± 9.00 µm vs. 139.50 ± 24.45 µm Copaxone treatment p < 0.05). Treatment with the higher concentration of Copaxone did not result in further increase in total neurites growth. The number of neurons also showed a trend of increase following Copaxone treatment (GRG: 29.00 ± 5.33; Copaxone at a low dose 10 µg / ml: 70.33 ± 24.29; Copaxone at a high dose 150 µg / ml: 64.00 ± 20.00).

**Table 21A: The effect of a combination of GRG and Copaxone on in-vitro neurite growth of rat primary spine cells**.**

| **Treatment** | **Study #** | **Total number of neurites** | **Total number of cells** | **Mean neurite length / cell (µm)** | **Mean number of neurites / cell** | **Mean number of bifurcations / cell** |
|---|---|---|---|---|---|---|
| DMEM | 5 | 64.00 | 32.00 | 152.04 | 2.08 | 0.44 |
| | 6 | 16.00 | 10.00 | 75.74 | 1.92 | 0.17 |
| | 7 | 50.00 | 22.00 | 142.44 | 2.27 | 0.63 |
| | 8 | 15.00 | 7.00 | 107.38 | 2.08 | 0.33 |
| | 9 | 12.00 | 9.00 | 93.61 | 1.36 | 0.00 |
| | **Mean** | **31.40** | **16.00** | **114.24** | **1.94** | **0.32** |
| | **SEM** | **10.70** | **4.79** | **14.46** | **0.16** | **0.11** |
| GRG (HA 0.2 % + TOCO 3 mM + LAM 10 ug / ml) | 4 | 42.00 | 20.00 | 149.02 | 2.04 | 0.79 |
| | 5 | 97.00 | 44.00 | 196.80 | 2.28 | 0.43 |
| | 6 | 82.00 | 39.00 | 124.80 | 2.22 | 0.49 |
| | 7 | 60.00 | 18.00 | 246.20 | 3.24 | 2.32 |
| | 8 | 46.00 | 14.00 | 260.77 | 3.29 | 2.13 |
| | 9 | 81.00 | 39.00 | 211.50 | 2.08 | 0.21 |
| | **Mean** | **68.00*** | **29.00** | **198.18** * | **2.53** | **1.06** |
| | **SEM** | **9.00** | **5.33** | **21.76** | **0.24** | **0.38** |
| DMEM + Copaxone 10ug / ml | 5 | 126.00 | 42.00 | 326.73 | 3.06 | 1.23 |
| GRG + Copaxone 150 ug / ml | 7 | 160.00 | 44.00 | 455.92 | 3.72 | 2.86 |
| | 8 | 84.00 | 84.00 | 450.90 | 2.65 | 0.93 |
| | **Mean** | **122.00** | **64.00#** | **453.41#** | **3.18** | **1.89** |
| | **SEM** | **38.00** | **20.00** | **2.51** | **0.54** | **0.96** |
| | 7 | 49.00 | 17.00 | 329.21 | 2.87 | 1.15 |
| | 8 | 120.00 | 120.00 | 398.64 | 3.09 | 1.14 |
| | 9 | 80.00 | 31.00 | 277.49 | 2.53 | 0.44 |
| GRG + Copaxone 50 ug / ml | **Mean** | **83.00** | **56.00** | **335.11#** | **2.83** | **0.91** |
| | **SEM** | **20.55** | **32.25** | **35.10** | **0.16** | **0.23** |
| GRG + Copaxone 10 ug / ml | 4 | 136.00 | 47.00 | 278.60 | 2.85 | 1.45 |
| | 5 | 235.00 | 82.00 | 456.17 | 2.78 | 1.62 |
| | 6 | 86.00 | 40.00 | 252.20 | 2.17 | 0.66 |
| | 7 | 106.00 | 32.00 | 437.56 | 3.12 | 2.57 |
| | 8 | 186.00 | 186.00 | 449.13 | 2.96 | 1.92 |
| | 9 | 88.00 | 35.00 | 324.28 | 2.43 | 0.24 |
| | **Mean** | **139.50#** | **70.33** | **366.32#** | **2.72** | **1.41** |
| | **SEM** | **24.45** | **24.29** | **37.64** | **0.14** | **0.35** |

| | | | | | | |
|---|---|---|---|---|---|---|
| **Neurite outgrowth was determined on day 7 in studies 4 and 6, on day 9 on studies 5 and 7 and day 10 on studies 8 and 9. * p < 0.05 VS DMEM (Student T-test) # p < 0.05 vs. GRG only. | | | | | | |

**Table 21B: The effect of a combination of GRG and Copaxone on in-vitro neurite growth of rat primary spine cells* compared to treatment with GRG only**.**

| **Treatment** | **Study #** | **Total number of neurites (%)** | **Total number of cells (%)** | **Mean neurite length / cell (%)** | **Mean number of neurites / cell (%)** | **Mean number of bifurcations / cell (%)** |
|---|---|---|---|---|---|---|
| DMEM | 5 | 65.98 | 72.73 | 77.26 | 90.92 | 101.92 |
| | 6 | 19.51 | 25.64 | 60.69 | 86.22 | 34.12 |
| | 7 | 83.33 | 122.22 | 57.85 | 70.12 | 27.36 |
| | 8 | 32.61 | 50.00 | 41.18 | 63.29 | 15.69 |
| | 9 | 14.81 | 23.08 | 44.26 | 65.14 | 0.00 |
| | **Mean** | **43.25** | **58.73** | **56.25** | **75.14** | **35.82** |
| | **SEM** | **13.43** | **18.26** | **6.46** | **5.65** | **17.51** |
| GRG (HA 0.2 % + TOCO 3 mM + LAM 10 ug / ml) | 4 | 100.00 | 100.00 | 100.00 | 100.00 | 100.00 |
| | 5 | 100.00 | 100.00 | 100.00 | 100.00 | 100.00 |
| | 6 | 100.00 | 100.00 | 100.00 | 100.00 | 100.00 |
| | 7 | 100.00 | 100.00 | 100.00 | 100.00 | 100.00 |
| | 8 | 100.00 | 100.00 | 100.00 | 100.00 | 100.00 |
| | 9 | 100.00 | 100.00 | 100.00 | 100.00 | 100.00 |
| | **Mean** | **100.00** | **100.00** | **100.00** | **100.00** | **100.00** |
| | **SEM** | **0.00** | **0.00** | **0.00** | **0.00** | **0.00** |
| DMEM + Copaxone 10ug / ml | 5 | 129.90 | 95.45 | 166.02 | 134.08 | 284.62 |
| GRG + Copaxone 150 ug / ml | 7 | 266.67 | 244.44 | 185.18 | 115.02 | 123.19 |
| | 8 | 182.61 | 600.00 | 172.91 | 80.38 | 43.87 |
| | **Mean** | **224.64** | **422.22** | **179.05** | **97.70** | **83.53** |
| | **SEM** | **42.03** | **177.78** | **6.13** | **17.32** | **39.66** |
| | 7 | 81.67 | 94.44 | 133.71 | 88.58 | 49.48 |
| | 8 | 260.87 | 857.14 | 152.87 | 94.01 | 53.48 |
| GRG + Copaxone 50 ug / ml | 9 | 98.77 | 79.49 | 131.20 | 121.33 | 213.33 |
| | **Mean** | **147.10** | **343.69** | **139.26** | **101.31** | **105.43** |
| | **SEM** | **57.10** | **256.76** | **6.84** | **10.13** | **53.96** |
| **GRG** + Copaxone 10 ug / ml | 4 | 323.81 | 235.00 | 186.96 | 139.49 | 183.23 |
| | 5 | 242.27 | 186.36 | 231.79 | 121.65 | 373.31 |
| | 6 | 104.88 | 102.56 | 202.08 | 97.80 | 135.71 |
| | 7 | 176.67 | 177.78 | 177.72 | 96.34 | 110.74 |
| | 8 | 404.35 | 1328.57 | 172.23 | 89.86 | 90.56 |
| | 9 | 108.64 | 89.74 | 153.32 | 116.47 | 115.29 |
| | **Mean** | **226.77** | **353.34** | **187.35** | **110.27** | **168.14** |
| | **SEM** | **49.16** | **196.31** | **11.06** | **7.72** | **43.00** |

| | | | | | | |
|---|---|---|---|---|---|---|
| *Neurite outgrowth was determined on day 7 in studies 4 and 6, on day 9 on studies 5 and 7 and day 10 on studies 8 and 9. **Results were normalized to treatment with GRG per each study. | | | | | | |

### EXAMPLE 4

### THE IN-VIVO EFFECT OF GRG COMPRISING TOCOPHEROL IN A RAT ACUTE SPINAL CORD INJURY MODEL

### MATERIALS AND METHODS

***Materials*** - All vendors and storage conditions are shown in Table 22 hereinbelow.

**Table 22: list of materials**

| **Materials** | **Name** | ***Cat. No.*** | **Manufacturer** | **Supplier** | **Storage Conditions** |
|---|---|---|---|---|---|
| Spinal cord cover | NeuraWrap^{™} | NW740 NW540 | Integra | Sponsor | RT |
| GRG composition | Tocopherol | 613420 | Millipore | Millipore | 4 °C |
| | Laminin peptide | 4107326 | Bachem | Bachem | -20 °C |
| | HA 0.4 % | 025735 | Lifecore | Lifecore Biomedical | -20 °C |
| | DMSO | D5879 | Sigma | Sigma | RT |
| Antibiotic | Baytril | N/A | BAYER | Lidor chemicals | RT |
| | Synthomycine (Chloramphenicol 5 %) | N / A | Rekah, Israel | Vetmarket | RT |
| Antiseptic | Polydine solution | N / A | FLORIS ISRAEL | Vetmarket | RT |
| Adhesion material | TISSEEL Fibrin Sealant | NDC0338-4211-04 | Baxter | Baxter | 2 - 25 °C |
| Anesthesia Items | Ketamine | N / A | Vetqvinol | Vetmarket | RT |
| | Xylazine | N / A | SURUPET | Vetmarket | RT |
| Analgesia Items | Buprenorphine | N / A | Vetmarket | Vetmarket | RT |
| | Dipyron | N / A | Ratiopharm GmbH, Germany | Vetmarket | RT |
| | Amitriptyline | N / A | FAGRON | Tamar market | RT |
| | Marcaine 0.5 % | N / A | Astrazenca | Vetmarket | RT |
| | Voltaren cream | N / A | Novartis. Switzerland | Vetmarket | RT |
| Euthanasia Item | Pentobarbital Sodium | N / A | CST | Vetmarket | RT |
| Histology Item | Glutaraldehyde 2.5 % | N / A | Electron Microscopy Sciences | Bar Naor | 4 - (-18) °C |
| | Calci-clear rapid | N / A | National diagnostics | Bar Naor | RT |

***Experimental procedure** -* The animals (Male Sprague Dawley rats) were anesthetized using intraperitoneal (IP) injection of ketamine (60 mg / kg) and medetomidine (0.25 mg / Kg). Following, the animals (i.e. rats) were placed on the surgery table and the area of the surgery was shaved, washed with ethanol and povidone iodine solution and covered with a sterile sheet to ensure sterile conditions. All surgical procedures were performed on anesthetized rats, using a high magnification neurosurgical microscope. The spinal cord was exposed via a dorsal approach. The overlying muscles were retracted, T7-T8 laminae removed, the spinal cord with dura was completely transected using micro-scissor and a 1 mm segment of the spinal cord was removed. Following, a 0.3 ml GRG formulation was injected to the gap or left untreated (i.e. control). A NeuraWrap^{™} Nerve Protector was used to cover the gap and glued using TISSEEL fibrin sealant to prevent leakage of the CSF or GRG. The muscular and cutaneous planes were closed and sutured. The rats were followed-up for a period of 6 months. The experimental groups are shown in Table 23 hereinbelow.

**Table 23: Rat spinal cord injury experimental groups**

| **Group #** | **Group Size** | **Treatment** |
|---|---|---|
| 1 | n=36 | Untreated |
| 2 | n=32 | GRG (HA 0.4 % + Laminin10 µg / ml + Tocopherol 3 mM + DMSO 0.6 %) |

***Electrophysiology*** - Motor Evoked Potentials (MEPs; single- or repetitive-pulse stimulation of the brain causes the spinal cord and peripheral muscles to produce neuroelectrical signals) were performed from both lower legs of anesthetized rats. Animals were monitored for baseline level on week -5 prior to surgery. Following, all animals were monitored on weeks 5, 9 and prior termination of the experiment. The mean group of amplitude, peak-to-peak amplitude or latency were measured in both left and right legs.

***BBB locomotor rating score -*** The Basso, Beattie and Bresnahan Locomotor Rating Scale (BBB scale) was used to evaluate locomotion, graded from 0 (without performances) to 21 (with a complete-normal gait performance). The BBB scale is known as a valid and predictive measure of locomotor recovery able to distinguish behavioral outcomes due to different injuries and to predict anatomical alterations at the lesion center. Animals were monitored for baseline level on week 0. Following, all animals were monitored on weeks 3, 7, 11, 15, 17, 19, 21 and 24 of the experiment.

***Immunohistochemistry for anti-MBP** -* At termination, animals were perfused with saline + heparin, followed by fixation in glutaraldehyde (GA) 2.5 %. Following 2 weeks of fixation, the tissues underwent decalcification, and the spinal cord was gently removed out of the vertebrae, and grossly cut into 3 pieces- cranial, middle (tube site) and distal. Each piece was dehydrated and embedded in paraffin. Transverse sections were stained using HRP-conjugated anti-MBP antibody. Analysis of the middle sections was performed in a "blinded" manner according to the following scale:
Grade 0=the spinal cord is intact with no pathological lesions
Grade 1=very mild pathology
Grade 2=mild pathology
Grade 3=moderate pathology
Grade 4=severe pathology
Grade 5=very severe pathology

***Statistical evaluation*** - The GRG treatment group was compared to the sham control untreated group using Student's T-test (GraphPad). A p < 0.05 is considered to represent a significant difference.

***Animal care and use statement*** - The study was performed following approval of an application form submitted to the Committee for Ethical Conduct in the Care and Use of Laboratory Animals that stated that the study complied with the rules and regulations set forth.

### RESULTS

*Electrophysiology (Motor Evoked Potentials (MEP):* Treatment with GRG (red dots) resulted in a higher Latency in both legs, on study week 9, compared to the untreated group: 13.28 ± 2.29 ms for the right leg or 17.32 ± 3.39 ms for the left leg vs. 4.48 ± 2.05 ms or 4.44 ± 1.97 ms for the untreated group, respectively, p < 0.05 (Figure 12).

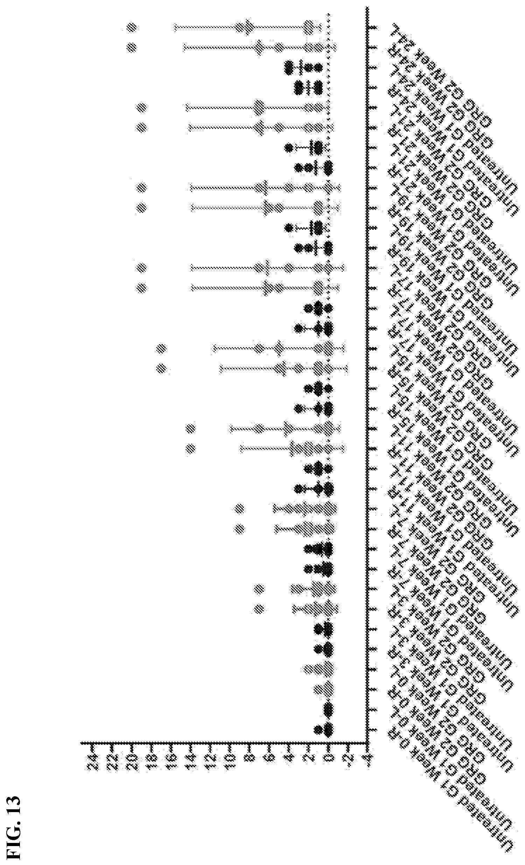

*BBB Locomotor Rating scale:* Animals treated with GRG (red dots) showed a higher mean BBB locomotor score throughout the study, compared to the untreated group. For example, on week 21, the mean locomotor score of animals treated with GRG (red dots) was 6.80 ± 3.23 points for the right leg or 7.20 ± 3.20 points for the left leg. For comparison, the mean BBB locomotor score of the untreated animals (black dots) on study week 21 was 1.25 ± 0.75 points or 1.75 ± 0.75 points for the right and left leg, respectively (Figure 13 and Table 24 hereinbelow).

**Table 24: BBB locomotor score**

| **Treatment Group #** | **Weeks** | | | | | | | | | |
|---|---|---|---|---|---|---|---|---|---|---|
| | **Week 0 (after surgery)** | | **3** | | **7** | | **11** | | **15** | |
| | **Right Leg** | **Left Leg** | **Right Leg** | **Left Leg** | **Right Leg** | **Left Leg** | **Right Leg** | **Left Leg** | **Right Leg** | **Left Leg** |
| | **MEAN** | | | | | | | | | |
| Untreated (Group 1) | 0.06 | 0.00 | 0.08 | 0.31 | 0.50 | 0.67 | 1.00 | 1.00 | 1.00 | 1.00 |
| GRG (Group 2) | 0.06 | 0.25 | 1.30 | 1.50 | 2.38 | 2.38 | 3.67 | 4.33 | 4.50 | 5.00 |

| | **SEM** | | | | | | | | | |
|---|---|---|---|---|---|---|---|---|---|---|
| Untreated (Group 1) | 0.04 | 0.00 | 0.08 | 0.13 | 0.34 | 0.33 | 0.71 | 0.41 | 0.71 | 0.41 |
| GRG (Group 2) | 0.04 | 0.09 | 0.68 | 0.67 | 1.02 | 1.08 | 2.11 | 2.23 | 2.60 | 2.67 |

| **Treatment Group #** | **Weeks** | | | | | | | |
|---|---|---|---|---|---|---|---|---|
| | **17** | | **19** | | **21** | | **22 / 24*** | |
| | **Right Leg** | **Left Leg** | **Right Leg** | **Left Leg** | **Right Leg** | **Left Leg** | **Right Leg** | **Left Leg** |
| | **MEAN** | | | | | | | |
| Untreated (Group 1) | 1.00 | 1.00 | 1.25 | 1.75 | 1.25 | 1.75 | 2.00 | 2.75 |
| GRG (Group 2) | 6.40 | 6.20 | 6.40 | 6.40 | 6.80 | 7.20 | 7.00 | 8.20 |

| | **SEM** | | | | | | | |
|---|---|---|---|---|---|---|---|---|
| Untreated (Group 1) | 0.71 | 0.41 | 0.75 | 0.75 | 0.75 | 0.75 | 0.58 | 0.75 |
| GRG (Group 2) | 3.31 | 3.43 | 3.31 | 3.36 | 3.23 | 3.20 | 3.42 | 3.29 |

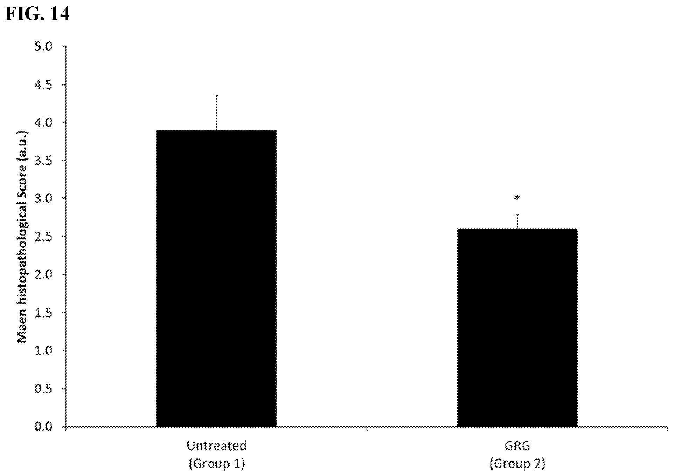

*Immunohistochemistry for Anti-MBP:* Myelin Basic Protein (MBP) was used as a regeneration marker. A statistically significant reduction in the mean histopathological score was found following treatment with GRG (Group 2) compared to the Untreated (Group 1) animals (2.60 ± 0.19 vs. 3.90 ± 0.46 for Group 2 vs. Group 1, p < 0.05) (Figure 14). These findings suggest a neuronal recovery in the injured site following treatment with GRG.

Taken together, the results suggest that treatment with GRG comprising Tocopherol at a concentration of 3 mM was active in improving electrophysiology, BBB locomotor score and histopathological scores, suggesting neuronal recovery in the rat acute spinal cord injury model.

### EXAMPLE 5

### THE IN-VIVO EFFECT OF A COMBNED TREATMENT WITH GRG COMPRISING COPAXONE IN A RAT ACUTE SPINAL CORD INJURY MODEL

### MATERIALS AND METHODS

***Materials*** - As in Example 4 hereinabove, with the addition of Copaxone (glatiramer acetate; Teva Pharmaceutical Industries Ltd., stored as 4 °C). GRG comprising Copaxone was prepared using the GRG formulation: 0.4 % HA solution with the addition of 10 µg / ml laminin, 3 mM tocopherol and 0.6 % DMSO. Additionally, a stock solution of 20 mg / ml was used and 0.5 µl were solved with 1 ml of PBS, to reach a final concentration of 10 µg / ml, which were added to the GRG.

***Experimental procedure*** - As in Example 4 hereinabove. The experimental groups are shown in Table 27 hereinbelow.

**Table 27: Rat spinal cord injury experimental groups**

| **Group #** | **Group Size** | **Treatment** |
|---|---|---|
| 1 | n=36 | Untreated |
| 2 | n=32 | GRG comprising Copaxone (HA 0.4 % + Laminin10 µg / ml + Tocopherol 3 mM + DMSO 0.6 % + Copaxone 10 µg / ml) |

***Electrophysiology -*** Somatosensory Evoked Potentials (SEPs were performed from both lower legs of anesthetized rats (ketamine (85 mg / kg) and xylazine (3 mg / Kg)), using a Dantec^{™} KEYPONT^{®} PORTABLE. The SEPs were recorded by placing subcutaneously a needle electrode between the eyes (anode) and a needle electrode (cranial level, over the approximate location of the somatosensory cortex) as cathode. For the induction of the SEPs one needle electrode was placed in the gastrocnemius muscle (cathode) and another one subcutaneously at the foot pad level (anode). For ground, an electrode was placed subcutaneously in the lumbar region. Throughout the experiments an average of 300 single pulses were delivered having a duration of 0.2 ms at a rate of 3 / sec. The stimulus intensity was increased gradually, until slight twitching of the hind leg will appear.

***Immunohistochemistry for anti-MBP** -* At termination, rats were perfused with saline + heparin, followed by fixation in glutaraldehyde (GA) 2.5 %. Following 2 weeks of fixation, the tissues underwent decalcification, and the spinal cord was gently removed out of the vertebrae, and grossly cut into 3 pieces - cranial, middle (injury site) and caudal. Each piece was dehydrated and embedded in paraffin. Embedded tissues in paraffin blocks were sectioned at approximately 5 µM thickness, 2 slides per block, put on a glass slide and immunohistochemistry staining with Myelin Basic Protein (IHC:MBP, Zotal, Israel). The stained slides were subjected to histological evaluation and pictures acquisition was performed only on pathological changes and of representative rats. An area of interest (AOI) and spatial calibration were applied to each image. Then RGB Histogram threshold was used to depict the brown stain and the Area and Area ratio (%) of each threshold were measured.

***Statistical evaluation** -* The GRG comprising Copaxone treatment group was compared to the control untreated group using Student's T-test (GraphPad). A p < 0.01 or p < 0.05 is considered to represent a significant difference.

***Animal care and use statement** -* As in Example 4 hereinabove.

### RESULTS

*Llectrophysiology (somatosensory Evoked Potentils (SEPs):* Although, the small sample at the endof the study (week 24) it is important to point out that 3 out of 4 rats, treated with 0.4 % GRG comprising Copaxone showed a signal on the right leg, while in the untreated group only one rat showed a signal (Table 28 hereinbelow and Figure 18).

**Table 28: Number of SEPs recorded at week 24**

| **Treatment** | **Week 22 / 24*** |
|---|---|
| Untreated (Group 1) | 1/4 |
| GRG Copaxone (Group 2) | 3 / 4 |

| | |
|---|---|
| * One animal from Group 4 was tested on week 22. | |

*Immunohistochemistry for Anti-MBP:* Myelin Basic Protein (MBP) was used as a regeneration marker. A significant regeneration of the injured area (i.e. middle sections) in the group treated with GRG comprising Copaxone compared to the untreated group (Figures 19A-B and Table 29 hereinbelow; * p < 0.01). Both cranial and caudal sections showed a high MBP signal, as expected. Cranial section was used as an individual control section for each rat. In the caudal section only a minor loss of myelin was noted.

**Table 29: MBP relative area in percentage**

| **Treatment Group #** | **Cranial** | **(Injury area)** | **Caudal** |
|---|---|---|---|
| | **Mean** | | |
| Untreated (Group 1) | 70.11 | 5.08 | 78.98 |
| GRG Copaxone (Group 2) | 82.62 | 29.41 ** | 64.73 |

| | **SEM** | | |
|---|---|---|---|
| Untreated (Group 1) | 2.66 | 2.69 | 10.69 |
| GRG comprising Copaxone (Group 2) | 5.66 | 10.95 | 11.01 |

| | | | |
|---|---|---|---|
| ** p < 0.1 using one-tailed Student's T-test vs. untreated p < vs.. | | | |

Taken together, the results suggest that treatment with GRG comprising Copaxone was active in improving electrophysiology and immunohistochemistry evaluation, suggesting neuronal recovery in the rat acute spinal cord injury model.

## Claims

1. A composition comprising a hyaluronic acid, a laminin polypeptide, vitamin E, and Copolymer 1 at a concentration range of 10 - 150 µg / ml.

2. The composition of claim 1, wherein said Copolymer 1 concentration range is 10 - 50 µg / ml.

3. The composition of claim 1, wherein said Copolymer 1 is at a concentration of 10 µg / ml.

4. The composition of any one of claims 1-3, wherein said vitamin E is at a concentration range of 0.3 - 30mM, or 1-30mM, or at a concentration of 3mM.

5. The composition of any one of claims 1-4, wherein said laminin polypeptide is set forth by SEQ ID NO: 1.

6. A matrix or a hydrogel comprising the composition of any of claims 1-5.

7. A method of inducing ex-vivo formation of a tissue, comprising:
(a) providing the composition, the matrix or the hydrogel of any one of claims 1-6, and;
(ii) seeding said composition, said matrix or said hydrogel with cells in a medium suitable for proliferation, differentiation and/or migration of said cells,
thereby inducing the formation of the tissue.

8. The composition, the matrix or the hydrogel of any one of claims 1-6 for use in treating a pathology **characterized by** diseased, damaged or loss of tissue in a subject in need thereof.

9. The composition, the matrix, or the hydrogel of any one of claims 1-6 for use in preventing or treating neurogenic shock following nerve injury in a subject in need thereof.

10. The composition of claim 1, comprising vitamin E at a concentration of 3mM, and Copolymer 1 at a concentration of 10 µg /ml.

## Patentansprüche

1. Zusammensetzung umfassend eine Hyaluronsäure, ein Laminin-Polypeptid, Vitamin E und Copolymer 1 in einem Konzentrationsbereich von 10 bis 150 µg/ml.

2. Zusammensetzung nach Anspruch 1, wobei der Konzentrationsbereich des Copolymers 1 10-50 µg/ml beträgt.

3. Zusammensetzung nach Anspruch 1, wobei das Copolymer 1 in einer Konzentration von 10 µg/ml vorliegt.

4. Zusammensetzung nach einem der Ansprüche 1 bis 3, wobei das Vitamin E in einem Konzentrationsbereich von 0,3 bis 30 mM oder 1 bis 30 mM oder in einer Konzentration von 3 mM vorliegt.

5. Zusammensetzung nach einem der Ansprüche 1 bis 4, wobei das Laminin-Polypeptid durch SEQ ID NO: 1 angegeben ist.

6. Matrix oder Hydrogel, umfassend die Zusammensetzung nach einem der Ansprüche 1 bis 5.

7. Verfahren zur Induktion der Ex-vivo-Bildung eines Gewebes, umfassend:
(a) Bereitstellen der Zusammensetzung, der Matrix oder des Hydrogels nach einem der Ansprüche 1 bis 6 und;
(ii) Besiedeln der Zusammensetzung, der Matrix oder des Hydrogels mit Zellen in einem Medium, das für die Proliferation, Differenzierung und/oder Migration der Zellen geeignet ist,
wodurch die Bildung des Gewebes induziert wird.

8. Zusammensetzung, Matrix oder Hydrogel nach einem der Ansprüche 1 bis 6 zur Verwendung bei der Behandlung einer Pathologie, die durch erkrankte, beschädigte oder verlorene Gewebe bei einem Patienten gekennzeichnet ist, der dies benötigt.

9. Zusammensetzung, Matrix oder Hydrogel nach einem der Ansprüche 1 bis 6 zur Verwendung bei der Vorbeugung oder Behandlung eines neurogenen Schocks nach einer Nervenverletzung bei einem Patienten, der dies benötigt.

10. Zusammensetzung nach Anspruch 1, umfassend Vitamin E in einer Konzentration von 3 mM und Copolymer 1 in einer Konzentration von 10 µg/ml.

## Revendications

1. Une composition comprenant un acide hyaluronique, un polypeptide de laminine, de la vitamine E et du copolymère 1 à une concentration comprise entre 10 et 150 µg/ml.

2. La composition selon la revendication 1, dans laquelle ladite concentration en copolymère 1 est comprise entre 10 et 50 µg/ml.

3. Composition selon la revendication 1, dans laquelle ledit copolymère 1 est à une concentration de 10 µg/ml.

4. La composition selon l'une quelconque des revendications 1 à 3, dans laquelle ladite vitamine E est à une plage de concentration de 0,3 à 30 mM, ou de 1 à 30 mM, ou à une concentration de 3 mM.

5. La composition selon l'une quelconque des revendications 1 à 4, dans laquelle ledit polypeptide de laminine est défini par SEQ ID NO: 1.

6. Matrice ou hydrogel comprenant la composition de l'une quelconque des revendications 1 à 5.

7. Procédé pour induire la formation ex vivo d'un tissu, comprenant :
(a) la fourniture de la composition, de la matrice ou de l'hydrogel de l'une quelconque des revendications 1 à 6, et ;
(ii) ensemencer ladite composition, ladite matrice ou ledit hydrogel avec des cellules dans un milieu approprié pour la prolifération, la différenciation et/ou la migration desdites cellules,
induisant ainsi la formation du tissu.

8. La composition, la matrice ou l'hydrogel de l'une quelconque des revendications 1 à 6, destinée à être utilisée dans le traitement d'une pathologie **caractérisée par** une maladie, une lésion ou une perte de tissu chez un sujet qui en a besoin.

9. La composition, la matrice ou l'hydrogel de l'une quelconque des revendications 1 à 6, destinée à être utilisée dans la prévention ou le traitement d'un choc neurogène consécutif à une lésion nerveuse chez un sujet qui en a besoin.

10. Composition selon la revendications 1, comprenant de la vitamine E à une concentration de 3 mM et un copolymère 1 à une concentration de 10 µg/ml.
